# EUROPEAN PATENT APPLICATION

(11) **EP 1 762 569 A1**
(43) Date of publication of application: **14.03.2007**
(21) Application number: 05019796.1
(22) Date of filing: 12.09.2005
(51) Int. Cl.: C07D 489/08, A61K 31/485, A61P 25/00

(54) **Novel 6-amino-morphinan derivatives, method of manufacturing them and their application as analgesics**

(71) Applicant: ALCASYNN PHARMACEUTICALS GMBH, 6020 Innsbruck (AT)
(72) Inventor: Kayatz, Peter, 6020 Innsbruck (AT); Schütz, Johannes, 6020 Innsbruck (AT)
(74) Representative: Grünecker, Kinkeldey, Stockmair & Schwanhäusser Anwaltssozietät

(57) **Abstract**

This invention relates to a class of 6-amino-morphinan compounds which can be used as highly active analgesics. This invention also relates to their pharmaceutically acceptable salts and easily accessible derivatives (e.g. esters or amides of the amino acid derivatives), to a process for their manufacture and their application in the manufacture of pharmaceutical specialities.

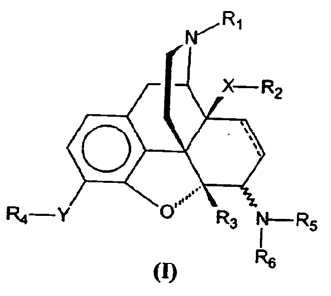

## Description

This invention relates to a class of 6-amino-morphinan compounds which can be used as highly active analgesics. This invention also relates to their pharmaceutically acceptable salts and easily accessible derivatives (e.g. esters or amides of the amino acid derivatives), to a process for their manufacture and their application in the manufacture of pharmaceutical specialities.

The existence of opioid receptors as receptors of the central nervous system (CNS), which transfer an analgesic effect, has been clearly proven. These receptors are subdivided into three subtypes, µ, κ and δ. Activation of these receptors by opioids results in an analgesic effect. The activation of the µ receptors causes the highest analgesic effect, whereby particularly morphinans with an oxygen function in position 6 (morphine, oxymorphone, hydromorphone, etc.) are used as effective analgesics. In the past a great deal of work has been invested in the structure-activity relationship studies of this class of substance.

In the Journal of Medicinal Chemistry 1984, 27, pp. 1575-1579 various 14-methoxymorphinan-6-ones with various substituents in position 3 are described. These derivatives exhibit higher analgesic activity than their 14-hydroxy counterparts.

A detailed study of 5-methyloxymorphone (= 14-hydroxy-5-methyldihydromorphinone) is described in Helvetica Chimica Acta (1988, 71, pp. 1801-1804) which arrives at the result that the introduction of a 5-methyl group reduces the opioid agonistic characteristics of oxymorphone.

A further study on 14-alkoxymorphinan-6-ones is described in Helvetica Chimica Acta 1989, 72, pp. 1233-1239 in which the influence of various substituents in position 3 and of the amino nitrogen was evaluated.

The German disclosure document DE 34 12 727 describes 14-alkoxy-N-methylmorphinan-6-ones (14-O-alkyloxymorphone) with higher activity than their 14-hydroxy counterparts.

Recently the existence of opioid receptors in the periphery has also been detected (e.g. in bones, joints, cartilage, muscles, etc.). It could be shown that analgesia is also imparted via these peripheral opioid receptors (C. Stein, New Engl. J. Med. 1995, 332, pp. 1685-1690). For this, only a slight dose of an opioid (e.g. morphine), which is applied directly into the injured tissue by injection, is necessary. This slight dose does not result in any side effects being imparted by the central nervous system. The analgesic effect has been observed especially during the treatment of inflammation and neuropathic pain (R. Likar et al., Brit. J. Anaesth. 1999, 83, pp. 241-244; V. Kayser et al., Neurosci. 1995, 64, 537-545). The type of application (injection) represents a significant disadvantage of the treatment. Repeated injections into the affected tissue or joint are associated with risks such as bleeding, infections or cartilage damage. Analgesically effective substances, which have only a limited access to the central nervous system (due to the fact that they cannot pass, or pass only to a very small extent, the blood-brain barrier) and which can be administered systemically or orally, are of great interest.

The object of this invention was to produce highly active analgesics which preferably possess restricted access to the CNS and which preferably act peripherally and not centrally and which also can be preferably systemically or orally administered. Substances showing promise of success in this connection would be ones which indicate an exclusively peripheral analgesic effect, without the side effects which occur with a centrally acting effect.

This invention solves the object presented above through the object of the independent claims. Preferred embodiments are given in the subclaims.

Accordingly the present invention provides three embodiments, i.e. compounds according to formulae (I), (Ia), and (II). These embodiments will be discussed in greater detail below.

### EMBODIMENT 1

Compounds of formula (I),

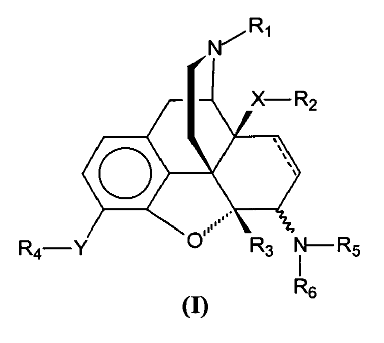

in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyi; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl; iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cydoalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C,₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl-and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably C₉-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1 H-imidazol-2-yl, 1,4,5,6-tetrahydropyrimidin-2-yl, 4,5,6,7-tetrahydro-1 H-[1,3]diazepin-2-yl;
and a group selected from an acid group or a derivative thereof bearing residue and moieties forming, together with the nitrogen atom to which they are bound, a residue corresponding to an amino acid, an amino acid derivative and/or a dimer or oligomer thereof and/or a peptide comprising up to 30 amino acid units, wherein at least one of R₅ and R₆ is selected from such a group;
X is oxygen, sulphur or methylene or the group (X-R₂) is H and
Y is oxygen or the group (Y-R₄) is H;
and pharmaceutically acceptable acid addition salts as well as base addition salts and easily accessible derivatives (e.g. esters or amides of amino acid derivatives). Some compounds of EMBODIMENT 1 may exist in different stereochemical configuration and/or may show more than one crystalline structure, in particular the compounds possessing one or more chiral carbon atom. The present invention comprises all those specific embodiments, such as diastereomer, enantiomers, polymorphs etc, in any given or desired mixture or in isolated form.

The various terms as employed above do have the following meaning and preferred embodiments are as follows:

The dotted line between the carbon atoms 7 and 8 of the morphinan skeleton designates that these carbon atoms may be unsaturated (double bond between C7 and C8) or saturated (single bond between C7 and C8).

In this invention the terms alkyl, alkenyl and alkynyl include both branched and also unbranched alkyl, alkenyl and alkynyl groups as well as mono-, di- and trihydroxy-substituted b ranched a nd unbranched alkyl, alkenyl a nd alkynyl groups. These groups furthermore may be substituted once twice or three times with substituents selected independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents are cyclic groups, including carbocycles and heterocycles which may be saturated unsaturated or aromatic. Preferred examples comprise from 3 to 8 ring atoms, selected from C, N, O, and S. The term aryl defines aromatic rings comprising preferably from 5 to 14 ring atoms and the term aryl comprises furthermore carbocyclic aryl groups as well as heterocyclic aryl groups, comprising preferably from 1 to 3 heteroatoms selected from N, O and S. Aryl can be unsubstituted or mono-, di- or tri-substituted, whereby the substituents can be chosen independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents are cyclic groups, including carbocycles and heterocycles which may be saturated unsaturated or aromatic. Preferred examples comprise from 3 to 8 ring atoms, selected from C, N, O, and S. The term aryl defines aromatic rings comprising preferably from 5 to 14 ring atoms and the term aryl comprises furthermore carbocyclic aryl groups as well as heterocyclic aryl groups, comprising preferably from 1 to 3 heteroatoms selected from N, O and S. The aryl goups as defined above may furthermore be fused ring systems such as naphthyl or anthracenyl or the corresponding heterocyclic groups comprising from 1 to 3 heteroatoms selected from N, O, and S. The definitions listed above for alkyl, alkenyl, alkynyl and aryl are valid for all substituents of this application.

As defined in claim 1, the compounds of EMBODIMENT 1 comprise at least one substituent R₅ or R₆ which forms with the nitrogen atom to which they are bound a group resembling an amino acid, of natural or synthetic origin, including cyclic structures corresponding to the amino acids proline, Tic and tryptophan. This designation comprises not only groups comprising a group -COOH or a derivative thereof as acid group but also groups wherein the acid functionality or derivative thereof is provided by means of other acidic groups, in particular groups involving a sulfur atom or a phosphorus atom. The present invention furthermore contemplates substituents which correspond to (amino) acid derivatives, such as esters, acid halides, amides etc. Furthermore comprised are substituents corresponding to amino acid dimmers, trimers or higher oligomers. Also comprised are peptide structures comprising up to 30 amino acid groups.

Suitable examples of amino acids which may form the basis for any one of the substituents R₅ or R₆ are Ala, GABA, Asp, Asn, Glu, Gln, Met, Cys, Val, Trp, Pro, Leu, Ile, Ser, Thr, Orn, Cit, Arg, Lys, Phe, Tyr, Dopa, His, Tic, including derivatives, such as hydroxy derivatives, phenyl derivatives etc. As outlined above the present invention also contemplates structures for the substituents R₅ or R₆ corresponding to dimers, trimers or higher oligomers of these acids.

Further comprised are acid groups derived from sulphonic and phosphonic acids, including derivatives such as esters and amides.

Preferred compounds of the present invention are compounds, wherein the at least one group selected from an acid group or a derivative thereof bearing residue and moieties forming, together with the nitrogen atom to which they are bound, a residue corresponding to an amino acid, an amino acid derivative and/or a dimer or oligomer thereof and/or a peptide comprising up to 30 amino acid units for R₅ and R₆, which can be the same or different, is selected from (C₁-C₃₀-alkyl)CO₂B, preferably C1-C12, more preferably C1-C6 alkyl; (C₂-C₃₀-alkenyl)CO₂B, preferably C2-C12, more preferably C2-C6 alkenyl; (C₂-C₃₀-alkynyl)CO₂B, preferably C2-C12, more preferably C2-C6 alkynyl; (C₄-C₃₀-cycloalkylalkyl)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CO₂B, where aryl is C₆-C₁₀-aryl and alkyl is C₁-C₂₄-alkyl, preferably C1-C12, more preferably C1-C6 alkyl; (C₈-C₃₀-arylalkenyl)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl)CO₂B, where aryl is C₆-C₁₀-aryl and alkynyl is C₂-C₂₄-alkynyl, preferably C2-C12, more preferably C2-C6 alkynyl;
(cyclic C₃-C₁₀-alkyl)CO₂B; (cyclic C₃-C₁₀-alkenyl)CO₂B; (cyclic C₃-C₁₀-alkynyl)CO₂B; (bicyclic C₆-C₂₀-alkyl)CO₂B; (bicyclic C₆-C₂₀-alkenyl)CO₂B; (bicyclic C₆-C₂₀-alkynyl)CO₂B; (cyclic C₃-C₁₀-alkyl fused with C₆-C₁₄ aromatic ring system)CO₂B; (cyclic C₃-C₁₀-alkenyl fused with C₆-C₁₄ aromatic ring system)CO₂B; (cyclic C₃-C₁₄-alkynyl fused with C₆-C₁₀ aromatic ring system)CO₂B;
[(C₁-C₃₀-alkyl)CO₂B]CO₂B, preferably C1-C12, more preferably C1-C6 alkyl; [(C₂-C₃₀-alkenyl)CO₂B]CO₂B, preferably C2-C12, more preferably C2-C6 alkenyl; [(C₂-C₃₀-alkynyl)CO₂B]CO₂B, preferably C2-C12, more preferably C2-C6 alkynyl; [(C₄-C₃₀-cycloalkylalkyl)CO₂B]CO₂B, where cycloalkyl is C₃-C₁₀-cycloalkyl and alkyl is C₁-C₂₇-alkyl, preferably C1-C12, more preferably C1-C6 alkyl; [(C₅-C₃₀-cycloalkylalkenyl)CO₂B]CO₂B, where cycloalkyl is C₃-C₁₀-cycloalkyl and alkenyl is C₂-C₂₇-alkenyl, preferably C2-C12, more preferably C2-C6 alkenyl; [(C₅-C₃₀-cycloalkylalkynyl)CO₂B]CO₂B, where cycloalkyl is C₃-C₁₀-cycloalkyl and alkynyl is C₂-C₂₇-alkynyl, preferably C2-C12, more preferably C2-C6 alkynyl; [(C₇-C₃₀-arylalkyl)CO₂B]CO₂B, where aryl is C₆-C₁₀-aryl and alkyl is C₁-C₂₄-alkyl, preferably C1-C12, more preferably C1-C6 alkyl; [(C₈-C₃₀-arylalkenyl)CO₂B]CO₂B, where aryl is C₆-C₁₀-aryl and alkenyl is C₂-C₂₄-alkenyl, preferably C2-C12, more preferably C2-C6 alkenyl; [(C₈-C₃₀-arylalkynyl)CO₂B]CO₂B, where aryl is C₆-C₁₀-aryl and alkynyl is C₂-C₂₄-alkynyl, preferably C2-C12, more preferably C2-C6 alkynyl;
[(C₁-C₃₀-alkyl)CONH₂]CO₂B, preferably C1-C12, more preferably C1-C6 alkyl; [(C₂-C₃₀-alkenyl)CONH₂]CO₂B, preferably C2-C12, more preferably C2-C6 alkenyl; [(C₂-C₃₀-alkynyl)CONH₂]CO₂B, preferably C2-C12, more preferably C2-C6 alkynyl; [(C₄-C₃₀-cycloalkylalkyl)CONH₂]CO₂B, where cycloalkyl is C₃-C₁₀-cycloalkyl and alkyl is C₁-C₂₇-alkyl, preferably C1-C12, more preferably C1-C6 alkyl; [(C₅-C₃₀-cycloalkylalkenyl)CONH₂]CO₂B, where cycloalkyl is C₃-C₁₀-cycloalkyl and alkenyl is C₂-C₂₇-alkenyl, preferably C2-C12, more preferably C2-C6 alkenyl; [(C₅-C₃₀-cycloalkylalkynyl)CONH₂]CO₂B, where cycloalkyl is C₃-C₁₀-cycloalkyl and alkynyl is C₂-C₂₇-alkynyl, preferably C2-C12, more preferably C2-C6 alkynyl; [(C₇-C₃₀-arylalkyl)CONH₂]CO₂B, where aryl is C₆-C₁₀-aryl and alkyl is C₁-C₂₄-alkyl, preferably C1-C12, more preferably C1-C6 alkyl; [(C₈-C₃₀-arylalkenyl)CONH₂]CO₂B, where aryl is C₆-C₁₀-aryl and alkenyl is C₂-C₂₄-alkenyl, preferably C2-C12, more preferably C2-C6 alkenyl; [(C₈-C₃₀-arylalkynyl)CONH₂]CO₂B, where aryl is C₆-C₁₀-aryl and alkynyl is C₂-C₂₄-alkynyl, preferably C2-C12, more preferably C2-C6 alkynyl;
(C₁-C₃₀-alkyl-S-A)CO₂B, preferably C1-C12, more preferably C1-C6 alkyl; (C₂-C₃₀-alkenyl-S-A)CO₂B, preferably C2-C12, more preferably C2-C6 alkenyl; (C₂-C₃₀-alkynyl-S-A)CO₂B, preferably C2-C12, more preferably C2-C6 alkynyl; (C₄-C₃₀-cycloalkylalkyl-S-A)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl-S-A)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl-S-A)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl-S-A)CO₂B, where aryl is C₆-C₁₀-aryl and alkyl is C₁-C₂₄-alkyl, preferably C1-C12, more preferably C1-C6 alkyl; (C₈-C₃₀-arylalkenyl-S-A)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl-S-A)CO₂B, where aryl is C₆-C₁₀-aryl and alkynyl is C₂-C₂₄-alkynyl, preferably C2-C12, more preferably C2-C6 alkynyl; A is H; C₁-C₃₀-alkyl, preferably C1-C12, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cycloalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C1-C12, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl; or A is selected from (C₁-C₃₀-alkyl-S-a)CO₂B, preferably C1-C12, more preferably C1-C6 alkyl; (C₂-C₃₀-alkenyl-S-a)CO₂B, preferably C2-C12, more preferably C2-C6 alkenyl; (C₂-C₃₀-alkynyl-S-a)CO₂B, preferably C2-C12, more preferably C2-C6 alkynyl; (C₄-C₃₀-cycloalkylalkyl-S-a)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl-S-a)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl-S-a)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl-S-a)CO₂B, where aryl is C₆-C₁₀-aryl and alkyl is C₁-C₂₄-alkyl, preferably C1-C12, more preferably C1-C6 alkyl; (C₈-C₃₀-arylalkenyl-S-a)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl-S-a)CO₂B, where aryl is C₆-C₁₀-aryl and alkynyl is C₂-C₂₄-alkynyl, preferably C2-C12, more preferably C2-C6 alkynyl; wherein a designates the connecting bond;
(CHDCONH)ₙCHDCO₂B, where n is from 1 to 30, where D is H; C₁-C₃₀-alkyl, preferably C1-C12, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cycloalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C1-C12, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl;
(C₁-C₃₀-alkyl)CO₂B, preferably C1-C12, more preferably C1-C6 alkyl; (C₂-C₃₀-alkenyl)CO₂B, preferably C2-C12, more preferably C2-C6 alkenyl; (C₁-C₃₀-alkynyl)CO₂B, preferably C2-C12, more preferably C2-C6 alkynyl; (C₄-C₃₀-cycloalkylalkyl)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CO₂B, where aryl is C₆-C₁₀-aryl and alkyl is C₁-C₂₄-alkyl, preferably C1-C12, more preferably C1-C6 alkyl; (C₈-C₃₀-arylalkenyl)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl)CO₂B, where aryl is C₆-C₁₀-aryl and alkynyl is C₂-C₂₄-alkynyl, preferably C2-C12, more preferably C2-C6 alkynyl;
(C₁-C₃₀-alkyl)CONH₂ , preferably C1-C12, more preferably C1-C6 alkyl; (C₂-C₃₀-alkenyl)CONH₂ , preferably C2-C12, more preferably C2-C6 alkenyl; (C₂-C₃₀-alkynyl)CONH₂ , preferably C2-C12, more preferably C2-C6 alkynyl; (C₄-C₃₀-cycloalkylalkyl)CONH₂, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CONH₂, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CONH₂, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CONH₂, where aryl is C₆-C₁₀-aryl and alkyl is C₁-C₂₄-alkyl, preferably C1-C12, more preferably C1-C6 alkyl; (C₈-C₃₀-arylalkenyl)CONH₂, where aryl is C₆-C₁₀-aryl and alkenyl is C₂-C₂₄-alkenyl, preferably C2-C12, more preferably C2-C6 alkenyl; (C₈-C₃₀-arylalkynyl)CONH₂, where aryl is C₆-C₁₀-aryl and alkynyl is C₂-C₂₄-alkynyl, preferably C2-C12, more preferably C2-C6 alkynyl;
wherein B is as defined in claim 1;
(C₁-C₃₀-alkyl)SO₃A#, preferably C1-C12, more preferably C1-C6 alkyl; (C₂-C₃₀-alkenyl)SO₃A#, preferably C2-C12, more preferably C2-C6 alkenyl; (C₂-C₃₀-alkynyl)SO₃A#, preferably C2-C12, more preferably C2-C6 alkynyl; (C₄-C₃₀-cycloalkylalkyl)SO₃A#, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)SO₃A#, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)SO₃A#, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)SO₃A#, where aryl is C₆-C₁₀-aryl and alkyl is C₁-C₂₄-alkyl, preferably C1-C12, more preferably C1-C6 alkyl; (C₈-C₃₀-arylalkenyl)SO₃A#, where aryl is C₆-C₁₀-aryl and alkenyl is C₂-C₂₄-alkenyl, preferably C2-C12, more preferably C2-C6 alkenyl; (C₈C₃₀-arylalkynyl)SO₃A#, where aryl is C₆-C₁₀-aryl and alkynyl is C₂-C₂₄-alkynyl, preferably C2-C12, more preferably C2-C6 alkynyl; (C₁-C₃₀-alkyl)PO(OA#)₂, preferably C1-C12, more preferably C1-C6 alkyl; (C₁-C₃₀-alkenyl)PO(OA#)₂, preferably C2-C12, more preferably C2-C6 alkenyl; (C₁-C₃₀-alkynyl)PO(OA#)₂, preferably C2-C12, more preferably C2-C6 alkynyl; (C₄-C₃₀-cycloalkylalkyl)PO(OA#)₂, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)PO(OA#)₂, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)PO(OA#)₂, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)PO(OA#)₂, where aryl is C₆-C₁₀-aryl and alkyl is C₁-C₂₄-alkyl, preferably C₁-C₁₂, more preferably C1-C6 alkyl; (C₈-C₃₀-arylalkenyl)PO(OA#)₂, where aryl is C₆-C₁₀-aryl and alkenyl is C₂-C₂₄-alkenyl, preferably C2-C12, more preferably C2-C6 alkenyl; (C₈-C₃₀-arylalkynyl)PO(OA#)₂, where aryl is C₆-C₁₀-aryl and alkynyl is C₂-C₂₄-alkynyl, preferably C2-C12, more preferably C2-C6 alkynyl; A# is H; C₁-C₃₀-alkyl, preferably C1-C12, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cydoalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C1-C12, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl.

The compounds of this invention contain pharmaceutically and pharmacologically acceptable salts of the compounds of formula (I). According to this invention both inorganic and also organic salts are suitable. Examples of suitable inorganic salts for this invention are hydrochlorides, hydrobromides, hydroiodides, sulphates, phosphates and tetrafluoroborates. Possible organic salts are, for example, acetates, tartrates, lactates, benzoates, stearates, pamoates, methane sulphonates, salicylates, fumarates, maleinates, succinates, aspartates, citrates, oxalates, trifluoroacetates and orotates.

Acid addition salts are preferred as conventional pharmaceutically acceptable addition salts, particularly preferred are the hydrochlorides, hydrobromides, hydroiodides, tetrafluoroborates and trifluoroacetates. X and Y are preferably oxygen. Preferably R₁ is alkyl as defined above, in particular methyl or ethyl, whereby methyl is preferred, or cycloalkylalkyl, preferably cyclopropylmethyl. R₂ is preferably not H and also not a group which forms an ester unit with X. The other definitions for R₂ as defined in Claim 1 are, in contrast, preferred, whereby especially alkyl as defined above is preferred, particularly preferred are methyl, ethyl and propyl, where necessary substituted, e.g. with a phenyl group, for example to produce a 3-phenylpropyl group (i.e., put differently, an arylalkyl group is also preferred for R₂, in particular 3-phenylpropyl). R₁ and R₂ are especially preferably both simultaneously alkyl, in particular either both simultaneously methyl or methyl (R₁) and ethyl (R₂). A further preferred combination of R₁ and R₂ is cycloalkylalkyl, in particular cyclopropylmethyl for R₁ and arylalkyl, preferably phenylpropyl for R₂. R₃ and R₄ are in each case preferably hydrogen or alkyl, whereby methyl is especially preferred as an alkyl group. R₄ is in addition preferred as C(N-Boc)(NH-Boc). R₅ and R₆ are preferably chosen such that one is H and the other is a radical different to H, wherein this radical preferably is not halogenated.

In a specially preferred representation X and Y are oxygen. Then preferably, R₁ is methyl and cyclopropylmethyl and R₂ is alkyl and arylalkyl, in particular methyl and 3-phenylpropyl, and R₃, R₄ and R₆ are hydrogen.

Preferred compounds of the present invention are further the base addition salts, comprising metal salts, such as lithium salts, sodium salts, potassium salts, beryllium salts, magnesium salts, calcium salts, strontium salts, aluminum salts and zinc salts; ammonium salts, such as C₁-C₃₀ monoalkylammonium salts, C₁-C₃₀ dialkylammonium salts, C₁-C₃₀ trialkylammonium salts, C₁-C₃₀ tetraalkylammonium salts; C₂-C₃₀ monoalkenylammonium salts, C₂-C₃₀ dialkenylammonium salts, C₂-C₃₀ trialkenylammonium salts, C₂-C₃₀ tetraalkenylammonium salts; C₂-C₃₀ monoalkynylammonium salts, C₂-C₃₀ dialkynylammonium salts, C₂-C₃₀ trialkynylammonium salts, C₂-C₃₀ tetraalkynylammonium salts; C₄-C₃₀ mono(cycloalkylalkylammonium) salts, C₄-C₃₀ di(cycloalkylalkylammonium) salts, C₄-C₃₀ tri(cycloalkylalkylammonium) salts, C₄-C₃₀ tetra(cycloalkylalkylammonium) salts, where cycloalkyl is C₃-C₁₀-cycloalkyl and alkyl is C₁-C₂₇-alkyl; C₅-C₃₀ mono(cycloalkylalkenylammonium) salts, C₅-C₃₀ di(cycloalkylalkenylammonium) salts, C₅-C₃₀ tri(cycloalkylalkenylammonium) salts, C₅-C₃₀ tetra(cycloalkylalkenylammonium) salts, where cycloalkyl is C₃-C₁₀-cycloalkyl and alkenyl is C₂-C₂₇-alkenyl; C₅-C₃₀ mono(cycloalkylalkynylammonium) salts, C₅-C₃₀ di(cycloalkylalkynylammonium) salts, C₅-C₃₀ tri(cycloalkylalkynylammonium) salts, C₅-C₃₀ tetra(cycloalkylalkynylammonium) salts, where cycloalkyl is C₃-C₁₀-cycloalkyl and alkynyl is C₂-C₂₇-alkynyl; C₇-C₃₀ mono(arylalkylammonium) salts, C₇-C₃₀ di(arylalkylammonium) salts, C₇-C₃₀ tri(arylalkylammonium) salts, C₇-C₃₀ tetra(arylalkylammonium) salts, where aryl is C₆-C₁₀-aryl and alkyl is C₁-C₂₄-alkyl; C₈-C₃₀ mono(arylalkenylammonium) salts, C₈-C₃₀ di(arylalkenylammonium) salts, C₈-C₃₀ tri(arylalkenylammonium) salts, C₈-C₃₀ tetra(arylalkenylammonium) salts, where aryl is C₆-C₁₀-aryl and alkenyl is C₂-C₂₄-alkenyl; C₈-C₃₀ mono(arylalkynylammonium) salts, C₈-C₃₀ di(arylalkynylammonium) salts, C₈-C₃₀ tri(arylalkynylammonium) salts, C₈-C₃₀ tetra(arylalkynylammonium) salts, where aryl is C₆-C₁₀-aryl and alkynyl is C₂-C₂₄-alkynyl, combinations of the ammonium salts listed above, and salts derived from heterocyclic bases, in particular heterocyclic nitrogen bases. These include salts derived from heterocyclic compounds comprising the following cycles: pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, pyrrolidine, imidazolidine, pyrazolidine, oxazolidine, isoxazolidine, thiazolidine, isothiazolidine, thiadiazolidine, sulpholane, imidazolidine, pyridine, pyridazine, pyrazine, pyrimidine, piperazine, piperidine, morpholine, tetrazole, triazole, triazolidine, tetrazolidine, azepine, homopiperazine and azetidine.

Preferred compounds of EMBODIMENT 1 are selected among the following:
(2S)-2-[(4,5α-Epoxy-3,14β-dihydroxy-17-methylmorphinan-6α-yl)amino]propionic acid tert-butyl ester
(2S)-2-[(4,5α-Epoxy-3,14β-dihydroxy-17-methylmorphinan-6β-yl)amino]propionic acid tert-butyl ester
(2S)-2-[(4,5α-Epoxy-3,14β-dihydroxy-17-methylmorphinan-6α-yl)amino]propionic acid
(2S)-2-[(4,5α-Epoxy-3,14β-dihydroxy-17-methylmorphinan-6β-yl)amino]propionic acid
(2S)-2-[(4,5α-Epoxy-3,14β-dihydroxy-17-methylmorphinan-6α-yl)amino]-3-phenylpropionic acid tert-butyl ester
(2S)-2-[(4,5α-Epoxy-3,14β-dihydroxy-17-methylmorphinan-6β-yl)amino]-3-phenylpropionic acid tert-butyl ester
(2S)-2-[(4,5α-Epoxy-3,14β-dihydroxy-17-methylmorphinan-6α-yl)amino]-3-phenylpropionic acid
(2S)-2-[(4,5α-Epoxy-3,14β-dihydroxy-17-methylmorphinan-6β-yl)amino]-3-phenylpropionic acid
3-[(4,5α-Epoxy-3-hydroxy-14β-methoxy-17-methylmorphinan-6α-yl)amino]propionic acid tert-butyl ester
3-[(4,5α-Epoxy-3-hydroxy-14β-methoxy-17-methylmorphinan-6β-yl)amino]propionic acid tert-butyl ester
3-[(4,5α-Epoxy-3-hydroxy-14β-methoxy-17-methylmorphinan-6α-yl)amino]propionic acid
3-[(4,5α-Epoxy-3-hydroxy-14β-methoxy-17-methylmorphinan-6β-yl)amino]propionic acid
4-[(4,5α-Epoxy-3-hydroxy-14β-methoxy-17-methylmorphinan-6α-yl)amino]butyric acid tert-butyl ester
4-[(4,5α-Epoxy-3-hydroxy-14β-methoxy-17-methylmorphinan-6β-yl)amino]butyric acid tert-butyl ester
4-[(4,5α-Epoxy-3-hydroxy-14β-methoxy-17-methylmorphinan-6α-yl)amino]butyric acid
4-[(4,5α-Epoxy-3-hydroxy-14β-methoxy-17-methylmorphinan-6β-yl)amino]butyric acid
(2S)-2-[(4,5α-Epoxy-3-hydroxy-14β-methoxy-17-methylmorphinan-6α-yl)amino]-3-methylbutyric acid tert-butyl ester
(2S)-2-[(4,5α-Epoxy-3-hydroxy-14β-methoxy-17-methylmorphinan-6β-yl)amino]-3-methylbutyric acid tert-butyl ester
(2S)-2-[(4,5α-Epoxy-3-hydroxy-14β-methoxy-17-methylmorphinan-6α-yl)amino]-3-methylbutyric acid
(2S)-2-[(4,5α-Epoxy-3-hydroxy-14β-methoxy-17-methylmorphinan-6β-yl)amino]-3-methylbutyric acid
(2R)-2-[(4,5α-Epoxy-3-hydroxy-14β-methoxy-17-methylmorphinan-6α-yl)amino]-3-methylbutyric acid tert-butyl ester
(2R)-2-[(4,5α-Epoxy-3-hydroxy-14β-methoxy-17-methylmorphinan-6β-yl)amino]-3-methylbutyric acid tert-butyl ester
(2R)-2-[(4,5α-Epoxy-3-hydroxy-14β-methoxy-17-methylmorphinan-6α-yl)amino]-3-methylbutyric acid
(2R)-2-[(4,5α-Epoxy-3-hydroxy-14β-methoxy-17-methylmorphinan-6β-yl)amino]-3-methylbutyric acid
(2S)-2-[(4,5α-Epoxy-3-hydroxy-14β-methoxy-17-methylmorphinan-6α-yl)amino]butanedioic acid di-tert-butyl ester
(2S)-2-[(4,5α-Epoxy-3-hydroxy-14β-methoxy-17-methylmorphinan-6β-yl)amino]butanedioic acid di-tert-butyl ester
(2S)-2-[(4,5α-Epoxy-3-hydroxy-14β-methoxy-17-methylmorphinan-6α-yl)amino]butanedioic acid
(2S)-2-[(4,5α-Epoxy-3-hydroxy-14β-methoxy-17-methylmorphinan-6β-yl)amino]butanedioic acid
(2S)-2-[(4,5α-Epoxy-3-hydroxy-14β-methoxy-17-methylmorphinan-6α-yl)amino]pentanedioic acid di-tert-butyl ester
(2S)-2-[(4,5α-Epoxy-3-hydroxy-14β-methoxy-17-methylmorphinan-6β-yl)amino]pentanedioic acid di-tert-butyl ester
(2S)-2-[(4,5α-Epoxy-3-hydroxy-14β-methoxy-17-methylmorphinan-6α-yl)amino]pentanedioic acid
(2S)-2-[(4,5α-Epoxy-3-hydroxy-14β-methoxy-17-methylmorphinan-6β-yl)amino]pentanedioic acid
(2S)-3-Carbamoyl-2-[(4,5α-epoxy-3-hydroxy-14β-methoxy-17-methylmorphinan-6α-yl)amino]propionic acid tert-butyl ester
(2S)-3-Carbamoyl-2-[(4,5α-epoxy-3-hydroxy-14β-methoxy-17-methylmorphinan-6β-yl)amino]propionic acid tert-butyl ester
(2S)-3-Carbamoyl-2-[(4,5α-epoxy-3-hydroxy-14β-methoxy-17-methylmorphinan-6α-yl)amino]propionic acid
(2S)-3-Carbamoyl-2-[(4,5α-epoxy-3-hydroxy-14β-methoxy-17-methylmorphinan-6β-yl)amino]propionic acid
(2S)-4-Carbamoyl-2-[(4,5α-epoxy-3-hydroxy-14β-methoxy-17-methylmorphinan-6α-yl)amino]butyric acid tert-butyl ester
(2S)-4-Carbamoyl-2-[(4,5α-epoxy-3-hydroxy-14β-methoxy-17-methylmorphinan-6β-yl)amino]butyric acid tert-butyl ester
(2S)-4-Carbamoyl-2-[(4,5α-epoxy-3-hydroxy-14β-methoxy-17-methylmorphinan-6α-yl)amino]butyric acid
(2S)-4-Carbamoyl-2-[(4,5α-epoxy-3-hydroxy-14β-methoxy-17-methylmorphinan-6β-yl)amino]butyric acid
(2S)-2-[(4,5α-Epoxy-3-hydroxy-14β-methoxy-17-methylmorphinan-6α-yl)amino]-4-methylthiobutyric acid tert-butyl ester
(2S)-2-[(4,5α-Epoxy-3-hydroxy-14β-methoxy-17-methylmorphinan-6β-yl)amino]-4-methylthiobutyric acid tert-butyl ester
(2S)-2-[(4,5α-Epoxy-3-hydroxy-14β-methoxy-17-methylmorphinan-6α-yl)amino]-4-methylthiobutyric acid
(2S)-2-[(4,5α-Epoxy-3-hydroxy-14β-methoxy-17-methylmorphinan-6β-yl)amino]-4-methylthiobutyric acid
(2S)-2-[(4,5α-Epoxy-3-hydroxy-14β-methoxy-17-methylmorphinan-6α-yl)amino]-3-(1H-indol-3-yl)propionic acid tert-butyl ester
(2S)-2-[(4,5α-Epoxy-3-hydroxy-14β-methoxy-17-methylmorphinan-6β-yl)amino]-3-(1H-indol-3-yl)propionic acid tert-butyl ester
(2S)-2-[(4,5α-Epoxy-3-hydroxy-14β-methoxy-17-methylmorphinan-6α-yl)amino]-3-(1H-indol-3-yl)propionic acid
(2S)-2-[(4,5α-Epoxy-3-hydroxy-14β-methoxy-17-methy)morphinan-6β-yl)amino]-3-(1H-indol-3-yl)propionic acid
(2S)-1-[(4,5α-Epoxy-3-hydroxy-14β-methoxy-17-methylmorphinan-6α-yl)]pyrrolidine-2-carboxylic acid tert-butyl ester
(2S)-1-[(4,5α-Epoxy-3-hydroxy-14β-methoxy-17-methylmorphinan-6β-yl)]pyrrolidine-2-carboxylic acid tert-butyl ester
2-[(4,5α-Epoxy-3-hydroxy-14β-methoxy-17-methylmorphinan-6α-yl)amino]ethanesulfonic acid
2-[(4,5α-Epoxy-3-hydroxy-14β-methoxy-17-methylmorphinan-6α-yl)amino]ethanesulfonic acid
2-{2-[(4,5α-epoxy-3-hydroxy-14β-methoxy-17-methylmorphinan-6α-yl)amino]acetylamino}acetic acid benzyl ester
2-{2-[(4,5α-epoxy-3-hydroxy-14β-methoxy-17-methylmorphinan-6β-yl)amino]acetylamino}acetic acid benzyl ester
2-{2-[(4,5α-epoxy-3-hydroxy-14β-methoxy-17-methylmorphinan-6α-yl)amino]acetylamino}acetic acid
2-{2-[(4,5α-epoxy-3-hydroxy-14β-methoxy-17-methylmorphinan-6β-yl)amino]acetylamino}acetic acid
(2S)-2-{[(4,5α-Epoxy-3-hydroxy-14β-methoxy-17-methylmorphinan-6α-yl)amino]-(2S)-3-methylbutyrylamino}-3-(4-hydroxyphenyl)propionic acid benzyl ester
(2S)-2-{[(4,5α-Epoxy-3-hydroxy-14β-methoxy-17-methylmorphinan-6β-yl)amino]-(2S)-3-methylbutyrylamino}-3-(4-hydroxyphenyl)propionic acid benzyl ester
(2S)-2-{[(4,5α-Epoxy-3-hydroxy-14β-methoxy-17-methylmorphinan-6α-yl)amino]-(2S)-3-methylbutyrylamino}-3-(4-hydroxyphenyl)propionic acid
(2S)-2-{[(4,5α-Epoxy-3-hydroxy-14β-methoxy-17-methylmorphinan-6β-yl)amino]-(2S)-3-methylbutyrylamino}-3-(4-hydroxyphenyl)propionic acid
3-[(17-Cyclopropylmethyl-4,5α-epoxy-3-hydroxy-14β-methoxymorphinan-6α-yl)amino]propionic acid tert-butyl ester
3-[(17-Cyclopropylmethyl-4,5α-epoxy-3-hydroxy-14β-methoxymorphinan-6β-yl)amino]propionic acid tert-butyl ester
3-[(17-Cyclopropylmethyl-4,5α-epoxy-3-hydroxy-14β-methoxymorphinan-6α-yl)amino]propionic acid
3-[(17-Cyclopropylmethyl-4,5α-epoxy-3-hydroxy-14β-methoxymorphinan-6β-yl)amino]propionic acid
4-[(17-Cyclopropylmethyl-4,5α-epoxy-3-hydroxy-14β-methoxymorphinan-6α-yl)amino]butyric acid tert-butyl ester
3-[(17-Cyclopropylmethyl-4,5α-epoxy-3-hydroxy-14β-methoxymorphinan-6β-yl)amino]butyric acid tert-butyl ester
3-[(17-Cyclopropylmethyl-4,5α-epoxy-3-hydroxy-14β-methoxymorphinan-6α-yl)amino]butyric acid
3-[(17-Cyclopropylmethyl-4,5α-epoxy-3-hydroxy-14β-methoxymorphinan-6β-yl)amino]butyric acid
(25)-2-[(17-Cyclopropylmethyl-4,5α-epoxy-3-hydroxy-14β-methoxymorphinan-6α-yl)amino]-3-methylbutyric acid tert-butyl ester
(2S)-2-[(17-Cyclopropylmethyl-4,5α-epoxy-3-hydroxy-14β-methoxymorphinan-6β-yl)amino]-3-methylbutyric acid tert-butyl ester
(2S)-2-[(17-Cyclopropylmethyl-4,5α-epoxy-3-hydroxy-14β-methoxymorphinan-6α-yl)amino]-3-methylbutyric acid
(2S)-2-[(17-Cyclopropylmethyl-4,5α-eEpoxy-3-hydroxy-14β-methoxymorphinan-6β-yl)amino]-3-methylbutyric acid
(2R)-2-[(17-Cyclopropylmethyl-4,5α-epoxy-3-hydroxy-14β-methoxymorphinan-6α-yl)amino]-3-methylbutyric acid tert-butyl ester
(2R)-2-[(17-Cyclopropylmethyl-4,5α-epoxy-3-hydroxy-14β-methoxymorphinan-6β-yl)amino]-3-methylbutyric acid tert-butyl ester
(2R)-2-[(17-Cyclopropylmethyl-4,5α-epoxy-3-hydroxy-14β-methoxymorphinan-6α-yl)amino]-3-methylbutyric acid
(2R)-2-[(17-Cyclopropylmethyl-4,5α-eEpoxy-3-hydroxy-14β-methoxymorphinan-6β-yl)amino]-3-methylbutyric acid
(2S)-2-[(17-Cyclopropylmethyl-4,5α-epoxy-3-hydroxy-14β-methoxymorphinan-6α-yl)amino]butanedioic acid di-tert-butyl ester
(2S)-2-[(17-Cyclopropylmethyl-4,5α-epoxy-3-hydroxy-14β-methoxymorphinan-6β-yl)amino]butanedioic acid di-tert-butyl ester
(2S)-2-[(17-Cyclopropylmethyl-4,5α-epoxy-3-hydroxy-14β-methoxymorphinan-6α-yl)amino]butanedioic acid
(2S)-2-[(17-Cyclopropylmethy)-4,5α-epoxy-3-hydroxy-14β-methoxymorphinan-6β-yl)amino]butanedioic acid

It has now been found that the compounds of the pertinent invention represent effective opioid receptor ligands of the type 6-aminomorphinan and exhibit a high therapeutic application potential as analgesics, as immunomodulators with immunostimulating or immunosuppressive effect, as cancer therapeutics, inflammation inhibitors, as anti-rheumatics, diuretics, anorectics, as an agent against diarrhoea, anaesthetics or as neuroprotective active substances.

The compounds quoted in the claims are therefore potentially applicable to the treatment of pain, functional intestinal diseases, such as abdominal pain, intestinal obstruction (ileus) or obstipation, for the treatment of mammals, in particular humans, for the treatment of Raynaud's disease, for the treatment of complaints caused by vasoconstriction, for the treatment of dysmenorrhoea, angina pectoris, myocardial infarct, emphysema, bronchial spasms, chronic obstructive bronchitis, rheumatic complaints, nephrosis, nephritis in conjunction with rheumatic diseases, for the treatment of tumours, phaeochromocytoma, Addison's disease, hepatic cirrhosis, chronic inflammation of the small and large intestines (e.g. irritable colon syndrome - colon irritabile, colitis ulcerosa, morbus Crohn), addiction withdrawal of, for example, opiates, cocaine or alcohol, or for the treatment of psychic diseases such as dysphoria or schizophrenia.

The compounds of this invention are suitable for application in the production of a medicament for the treatment of pain, including acute and chronic pain, on the locomotor system such as pain in the neck, back, hip, knee, shoulder or myofacial pain, treatment of complex regional pain syndromes, phantom pain, facial neuralgia, rheumatalgia, cancer pain, pain from burns, pain after accidents, pain due to chronic inflammation, visceralgia, headaches such as for example tension headaches, cervically related headache or migraine, pain after central lesions such as for example with paraplegia or thalamic lesions, neuralgic pain such as zoster neuralgia, postzoster neuralgia, ischaemic pain such as angina pectoris or peripheral occlusive arterial disease, postoperative pain, neuropathic pain such as pain with diabetic neuropathy, pain after virus infections or pain after nerve lesions.

The pharmaceutical compositions according to the invention, which contain a compound of this invention and / or a pharmaceutically acceptable salt of it as active ingredient together with a pharmaceutically acceptable carrier substance, are suitable for the treatment of the conditions quoted in the description.

The application according to the invention includes application as analgesic, immunomodulating, antitumour, antiproliferative, anti-inflammatory, antirheumatic, diuretic, anorectic, antidiarrhoeal, anaesthetic, neuroprotective active substance and as active substance for the prevention and treatment of intestinal obstruction (ileus).

Preferred applications take place for the production of a medicament for the treatment of pain, functional intestinal diseases, of the Raynaud's disease, for the treatment of complaints caused by vasoconstriction, angina pectoris, myocardial infarct, emphysema, bronchial spasms, chronic obstructive bronchitis, rheumatic complaints (including rheumatoid arthritis, arthrosis, osteoarthritis, spondylosis, lumbago, lupus erythematosus, spondyarthropathy), nephrosis, nephritis in conjunction with rheumatic diseases, for the treatment of tumours, cancer, phaeochromocytoma, Addison's disease, hepatic cirrhosis, chronic inflammation of the small and large intestines (e.g. irritable colon syndrome - colon irritabile, colitis ulcerosa, morbus Crohn), for the treatment of drug abuse, psychic diseases, erectile dysfunction and / or for the suppression of rejection of transplants after transplantation on mammals, particularly on humans.

Surprisingly it was also found that the compounds of this invention were not capable of overcoming the blood-brain barrier or only to a slight extent, and therefore a special significance could be attributed to them with regard to their application as peripherally effective therapeutics, for example as medicaments for the treatment of pain, rheumatic therapy, suppression of organ rejection after transplantations on mammals, particularly humans and also for the treatment of erectile disturbances. The limited access to the central nervous system is accompanied by a much reduced rate of side effects relating to central side effects such for example nausea, vomiting, sedation, dizziness, confusion, respiratory depression and mania.

In addition, it was surprisingly found that the compounds of this invention have a very long analgesically effective period. This enables a lower dosage and less frequent administration of the medicament, which results in a lower rate of side effects and toxicity as well as a higher readiness of patients to take the medicament.

The compounds according to EMBODIMENT 1 may be prepared as follows:

### Typical procedure for the synthesis of the esters: Examples 1-20; Compounds 1-40

A mixture of 1 mmol of the 6-keto precursor¹, 2 mmol of the corresponding amino acid ester¹ triethylamine² and 10 mL anhydrous methanol³ is stirred under inert conditions at room temperature for 3 hours⁴. After addition of 1 mmol sodium cyanoborohydride⁵, the mixture is stirred for 2 days⁵ at room temperature. The end of the reaction is monitored by TLC. After addition of water, the mixture is evaporated and the residue is partitioned between water and dichloromethane⁶. The crude product is separated and purified by column chromatography (silica gel; dichloromethane/methanol)⁷. Different modifications of this typical procedure are possible, e.g.:
1) In this invention, Oxymorphone, 14-O-Methyloxymorphone and 14-O-Methylnaltrexone were used. The 6-keto precursors and the amino acid esters can be applied as salts or as free base. The amount of amino acid ester can vary from 1 to 5 equivalents.
2) Triethylamine can be replaced by any tertiary non-nucleophilic amino or by any other base, preferably by N,N-diisopropylethylamine. The amount of triethylamine can vary from 0 to 5 equivalents. Water scavengers such as molecular sieves or trimethyl orthoformate also can be applied.
3) Methanol can be replaced by other protic solvents, preferrably by ethanol or isopropanol. The amount of methanol can vary from 1 to 50 mL per mmol 6-keto precursor.
4) The reaction time can vary from 0 to 20 hours.
5) Sodium cyanoborohydride can be replaced by other reduction agents such as complex metal hydrides in THF or other aprotic solvents. The amount of sodium cyanoborohydride can vary from 0.5 to 3 equivalents. The reaction time can vary from 0.5 to 10 days.
6) Instead of water, also brine can be used, instead of dichloromethane, each suitable organic solvent can be used.
7) The liquid phase for column chromatography can consist of various mixtures of dichloromethane/methanol; or dichloromethane/methanol/ammonia solution; or dichloromethane/methanol/triethylamine; or other appropriate solvents and mixtures thereof.

The following examples show the analytical data of the esters:

### Example 1:

### (2S)-2-[(4,5α-Epoxy-3,14β-dihydroxy-17-methylmorphinan-6α-yl)amino]propionic acid tert-butyl ester (Compound 1) and (25)-2-[(4,5α-Epoxy-3,14β-dihydroxy-17-methylmorphinan-6β-yl)amino]propionic acid tert-butyl ester (Compound 2)

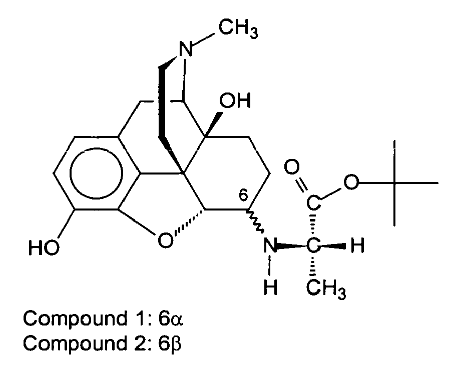

Compound 1: 20% yield; ¹H-NMR (CDCl₃): 6.69 (d, J=8.0, 1 ar. H), 6.49 (d, J=8.0, 1 ar. H), 4.65 (d, J=2.8, H-C(5)), 2.34 (s, MeN), 1.45 (s, t-Bu), 1.30 (d, J=7.0, NHCHMe). Compound 2: 18% yield; ¹H-NMR (CDCl₃): 6.70 (d, J=8.0, 1 ar. H), 6.55 (d, J=8.0, 1 ar. H), 4.37 (d, J=7.8, H-C(5)), 2.40 (s, MeN), 1.42 (s, t-Bu), 1.24 (d, J=7.0, NHCHMe).

### Example 2:

### (2S)-2-[(4,5α-Epoxy-3,14β-dihydroxy-17-methylmorphinan-6α-yl)amino]-3-phenylpropionic acid tert-butyl ester (Compound 3) and (2S)-2-[(4,5α-Epoxy-3,14β-dihydroxy-17-methylmorphinan-6β-yl)amino]-3-phenylpropionic acid tert-butyl ester (Compound 4)

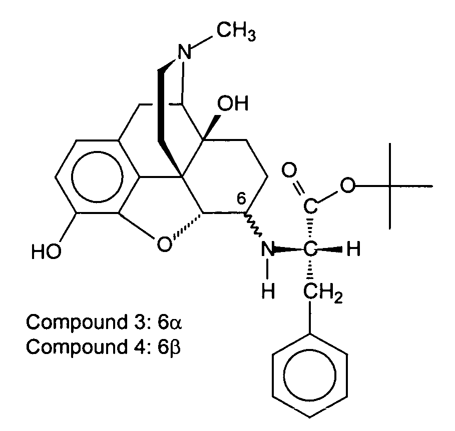

Compound 3: 16% yield; ¹H-NMR (CDCl₃): 7.35-7.23 (m, 5 ar. H), 6.69 (d, J=8.1, 1 ar. H), 6.49 (d, J=8.1, 1 ar. H), 4.65 (d, J=3.0, H-C(5)), 3.65 (t, J=7.5, NHC*H*), 2.35 (s, MeN), 1.39 (s, t-Bu).

Compound 4: 40% yield; ¹H-NMR (CDCl₃): 7.30-7.21 (m, 5 ar. H), 6.69 (d, J=8.2, 1 ar. H), 6.56 (d, J=8.2, 1 ar. H), 4.37 (d, J=7.8, H-C(5)), 3.46 (t, J=7.5, NHCH), 2.37 (s, MeN), 1.32 (s, t-Bu).

### Example 3:

### 3-[(4,5α-Epoxy-3-hydroxy-14β-methoxy-17-methylmorphinan-6α-yl)amino]propionic acid tert-butyl ester (Compound 5) and 3-[(4,5α-Epoxy-3-hydroxy-14β-methoxy-17-methylmorphinan-6β-yl)amino]propionic acid tert-butyl ester (Compound 6)

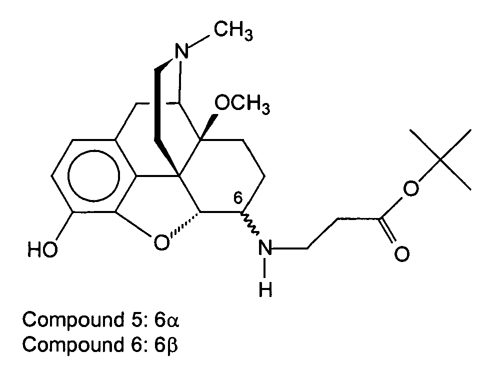

Compound 5: 12% yield; ¹H-NMR (CDCl₃):6.64 (d, J=8.3, 1 ar. H), 6.48 (d, J=8.3, 1 ar. H), 4.68 (d, J=3.8, H-C(5)), 3.22 (s, MeO), 2.42 (s, MeN), 1.45 (s, t-Bu).

Compound 6: 23% yield; ¹H-NMR (CDCl₃): 6.64 (d, J=8.0, 1 ar. H), 6.54 (d, J=8.0, 1 ar. H), 4.51 (d, J=7.4, H-C(5)), 3.20 (s, MeO), 2.39 (s, MeN), 1.46 (s, t-Bu).

### Example 4:

### 3-[(17-Cyclopropylmethyl-4,5α-epoxy-3-hydroxy-14β-methoxymorphinan-6α-yl)amino]propionic acid tert-butyl ester (Compound 7) and 3-[(17-Cyclopropylmethyl-4,5α-epoxy-3-hydroxy-14β-methoxymorphinan-6β-yl)amino]propionic acid tert-butyl ester (Compound 8)

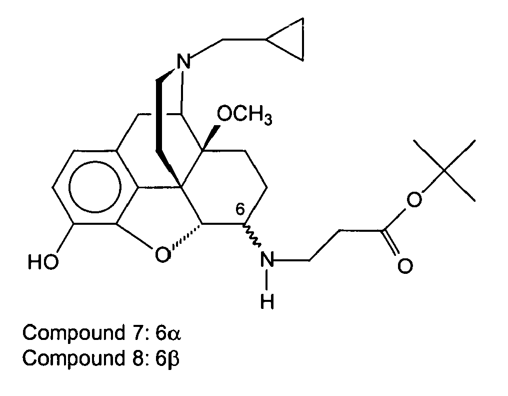

Compound 7: 11 % yield; ¹H-NMR (CDCl₃): 6.64 (d, J=8.1, 1 ar. H), 6.45 (d, J=8.1, 1 ar. H), 4.71 (d, J=3.6, H-C(5)), 3.28 (s, MeO), 1.44 (s, t-Bu), 0.88 (m, CH-cp), 0.51 (m, CH₂-cp), 0.13 (m, CH₂-cp).

Compound 8 : 28% yield; ¹H-NMR (CDCl₃): 6.62 (d, J=8.2, 1 ar. H), 6.51 (d, J=8.2, 1 ar. H), 4.53 (d, J=7.4, H-C(5)), 3.25 (s, MeO), 1.46 (s, t-Bu), 0.88 (m, CH-cp), 0.51 (m, CH₂-cp), 0.16 (m, CH₂-cp). cp: cyclopropyl

### Example 5:

### 4-[(4,5α-Epoxy-3-hydroxy-14β-methoxy-17-methylmorphinan-6α-yl)amino]butyric acid tert-butyl ester (Compound 9) and 4-[(4,5α-Epoxy-3-hydroxy-14β-methoxy-17-methylmorphinan-6β-yl)amino]butyric acid tert-butyl ester (Compound 10)

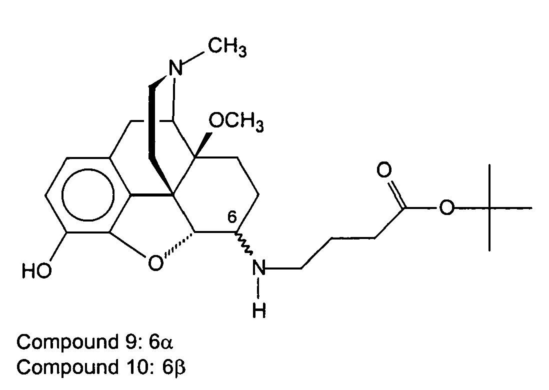

Compound 9: 15% yield; ¹H-NMR (CDCl₃): 6.67 (d, J=8.2, 1 ar. H), 6.48 (d, J=8.2, 1 ar. H), 4.70 (d, J=3.6, H-C(5)), 3.22 (s, MeO), 2.34 (s, MeN), 1.44 (s, t-Bu).

Compound 10: 53% yield; ¹H-NMR (CDCl₃): 6.68 (d, J=8.0, 1 ar. H), 6.55 (d, J=8.0, 1 ar. H), 4.43 (d, J=6.8, H-C(5)), 3.20 (s, MeO), 2.37 (s, MeN), 1.45 (s, t-Bu).

### Example 6:

### 4-[(17-Cyclopropylmethyl-4,5α-epoxy-3-hydroxy-14β-methoxymorphinan-6α-yl)amino]butyric acid tert-butyl ester (Compound 11) and 3-[(17-Cyclopropylmethyl-4,5α-epoxy-3-hydroxy-14β-methoxymorphinan-6β-yl)amino]butyric acid tert-butyl ester (Compound 12)

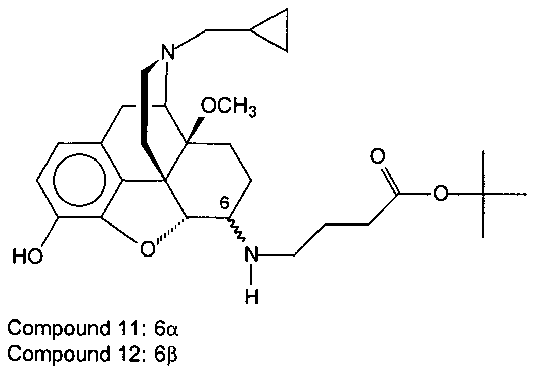

Compound 11: 24% yield; ¹H-NMR (CDCl₃): 6.65 (d, J=8.1, 1 ar. H), 6.46 (d, J=8.1, 1 ar. H), 4.70 (d, J=3.6, H-C(5)), 3.28 (s, MeO), 1.44 (s, t-Bu), 0.88 (m, CH-cp), 0.51 (m, CH₂-cp), 0.13 (m, CH₂-cp).

Compound 12: 21% yield; ¹H-NMR (CDCl₃): 6.65 (d, J=8.2, 1 ar. H), 6.52 (d, J=8.2, 1 ar. H), 4.46 (d, J=6.8, H-C(5)), 3.25 (s, MeO), 1.44 (s, t-Bu), 0.84 (m, CH-cp), 0.51 (m, CH₂-cp), 0.15 (m, CH₂-cp).

### Example 7:

### (2S)-2-[(4,5α-Epoxy-3,14β-dihydroxy-17-methylmorphinan-6α-yl)amino]-3-methylbutyric acid tert-butyl ester (Compound 13) and (2S)-2-[(4,5α-Epoxy-3,14β-dihydroxy-17-methylmorphinan-6β-yl)amino]-3-methylbutyric acid tert-butyl ester (Compound 14)

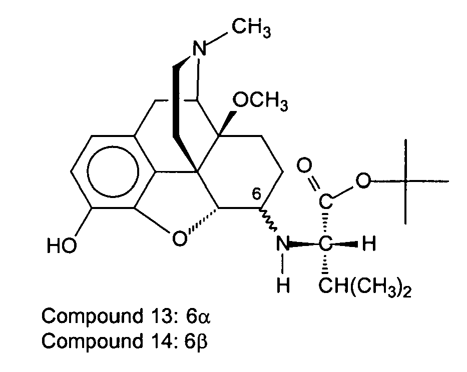

Compound 13: 8% yield; ¹H-NMR (CDCl₃): 6.71 (d, J=8.0, 1 ar. H), 6.51 (d, J=8.0, 1 ar. H), 4.65 (d, J=2.2, H-C(5)), 3.21 (s, MeO), 2.36 (s, MeN), 1.48 (s, t-Bu), 0.95 (d, J=7.0, CHMe), 0.93 (d, J=7.0, CHMe).

Compound 14: 26% yield; ¹H-NMR (CDCl₃): 6.70 (d, J=8.4, 1 ar. H), 6.55 (d, J=8.4, 1 ar. H), 4.37 (d, J=7.6, H-C(5)), 3.23 (s, MeO), 2.43 (s, MeN), 1.43 (s, t-Bu), 0.92 (d, J=6.8, 2 × CH*Me*).

### Example 8:

### (2S)-2-[(17-Cyclopropylmethyl-4,5α-epoxy-3-hydroxy-14β-methoxymorphinan-6α-yl)amino]-3-methylbutyric acid tert-butyl ester (Compound 15) and (2S)-2-[(17-Cyclopropylmethyl-4,5α-epoxy-3-hydroxy-14β-methoxymorphinan-6α-yl)amino]-3-methylbutyric acid tert-butyl ester (Compound 16)

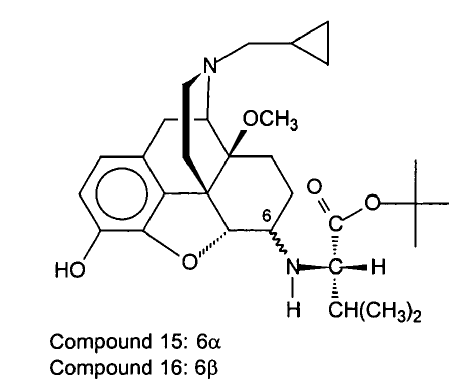

Compound 15:

Compound 16: 16% yield; H-NMR (CDCl₃): 6.67 (d, J=8.2, 1 ar. H), 6.51 (d, J=8.2, 1 ar. H), 4.37 (d, J=7.2, H-C(5)), 3.24 (s, MeO), 1.43 (s, t-Bu), 0.92 (d, J=6.6, 2 × CHMe), 0.50 (m, CH₂-cp), 0.14 (m, CH₂-cp).

### Example 9:

### (2R)-2-[(4,5α-Epoxy-3,14β-dihydroxy-17-methylmorphinan-6α-yl)amino]-3-methylbutyric acid tert-butyl ester (Compound 17) and (2R)-2-[(4,5α-Epoxy-3,14β-dihydroxy-17-methylmorphinan-6β-yl)amino]-3-methylbutyric acid tert-butyl ester (Compound 18)

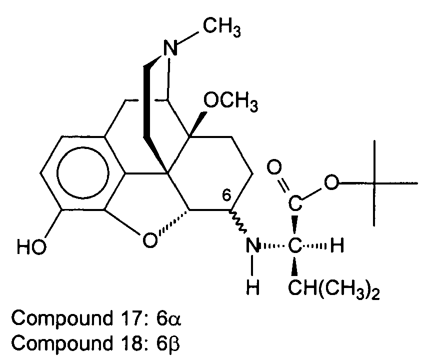

Compound 17: 46% yield; ¹H-NMR (CDCl₃): 6.67 (d, J=8.0, 1 ar. H), 6.50 (d, J=8.0, 1 ar. H), 4.61 (d, J=4.0, H-C(5)), 3.22 (s, MeO), 2.36 (s, MeN), 1.51 (s, t-Bu), 0.92 (d, J=7.0, CHMe), 0.90 (d, J=7.0, CHMe).

Compound 18: 12% yield; ¹H-NMR (CDCl₃): 6.70 (d, J=8.2, 1 ar. H), 6.54 (d, J=8.0, 1 ar. H), 4.46 (d, J=6.6, H-C(5)), 3.24 (s, MeO), 2.45 (s, MeN), 1.36 (s, t-Bu), 1.02 (d, J=6.8, CH*Me*), 0.97 (d, J=6.8, CHMe).

### Example 10:

### (2R)-2-[(17-Cyclopropylmethyl-4,5α-epoxy-3-hydroxy-14β-methoxymorphinan-6α-yl)amino]-3-methylbutyric acid tert-butyl ester (Compound 19) and (2R)-2-[(17-Cyclopropylmethyl-4,5α-epoxy-3-hydroxy-14β-methoxymorphinan-6α-yl)amino]-3-methylbutyric acid tert-butyl ester (Compound 20)

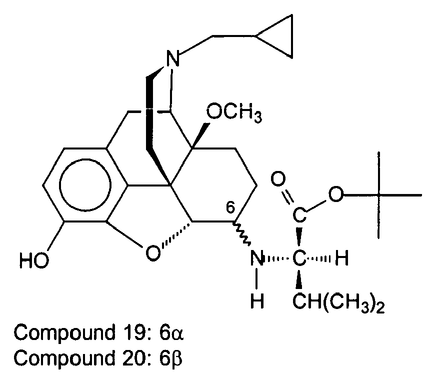

Compound 19: 15% yield; ¹H-NMR (CDCl₃): 6.64 (d, J=8.0, 1 ar. H), 6.46 (d, J=8.0, 1 ar. H), 4.63 (d, J=3.6, H-C(5)), 3.27 (s, MeO), 1.51 (s, t-Bu), 0.92 (d, J=6.8, CHMe), 0.90 (d, J=6.8, CHMe), 0.51 (m, CH₂-cp), 0.14 (m, CH₂-cp).

Compound 20:

### Example 11:

### (2S)-2-[(4,5α-Epoxy-3-hydroxy-14β-methoxy-17-methylmorphinan-6α-yl)amino]butanedioic acid di-tert-butyl ester (Compound 21) and (2S)-2-[(4,5α-Epoxy-3-hydroxy-14β-methoxy-17-methylmorphinan-6β-yl)amino]butanedioic acid di-tert-butyl ester (Compound 22)

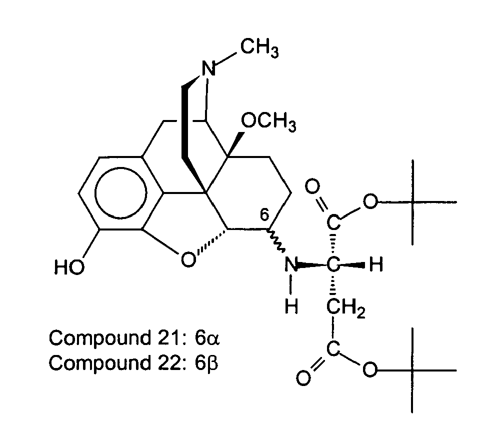

Compound 21: 8% yield; ¹H-NMR (CDCl₃): 6.70 (d, J=8.0, 1 ar. H), 6.50 (d, J=8.0, 1 ar. H), 4.71 (d, J=3.8, H-C(5)), 3.74 (t, J=6.5, NHCH), 3.21 (s, MeO), 2.35 (s, MeN), 1.48 (s, t-Bu), 1.45 (s, t-Bu).

Compound 22: 21% yield; ¹H-NMR (CDCl₃): 6.70 (d, J=8.3, 1 ar. H), 6.56 (d, J=8.3, 1 ar. H), 4.41 (d, J=7.4, H-C(5)), 3.82 (t, J=6.6, NHCH), 3.21 (s, MeO), 2.40 (s, MeN),1.45 (s, t-Bu), 1.44 (s, t-Bu).

### Example 12:

### (2S)-2-[(17-Cyclopropylmethyl-4,5α-epoxy-3-hydroxy-14β-methoxymorphinan-6α-yl)amino]butanedioic acid di-tert-butyl ester (Compound 23) and (2S)-2-[(17-Cyclopropylmethyl-4,5α-epoxy-3-hydroxy-14β-methoxymorphinan-6β-yl)amino]butanedioic acid di-tert-butyl ester (Compound 24)

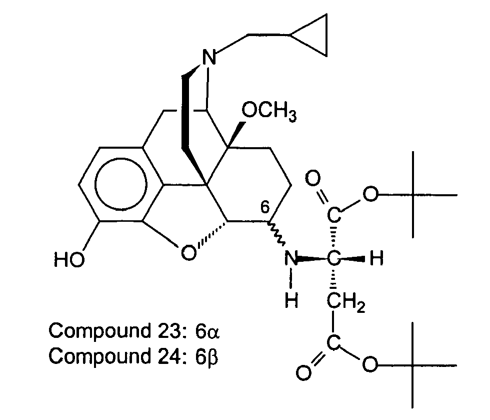

Compound 23:

Compound 24: 14% yield; ¹H-NMR (CDCl₃): 6.69 (d, J=8.0, 1 ar. H), 6.54 (d, J=8.0, 1 ar. H), 4.43 (d, J=7.4, H-C(5)), 3.85 (t, J=6.6, NHC*H*), 3.24 (s, MeO), 1.46 (s, t-Bu), 1.45 (s, t-Bu), 0.88 (m, CH-cp), 0.51 (m, CH₂-cp), 0.15 (m, CH₂-cp).

### Example 13:

### (2S)-3-Carbamoyl-2-[(4,5α-epoxy-3-hydroxy-14β-methoxy-17-methylmorphinan-6α-yl)amino]propionic acid tert-butyl ester (Compound 25) and (2S)-3-Carbamoyl-2-[(4,5α-epoxy-3-hydroxy-14β-methoxy-17-methylmorphinan-6β-yl)amino]propionic acid tert-butyl ester (Compound 26)

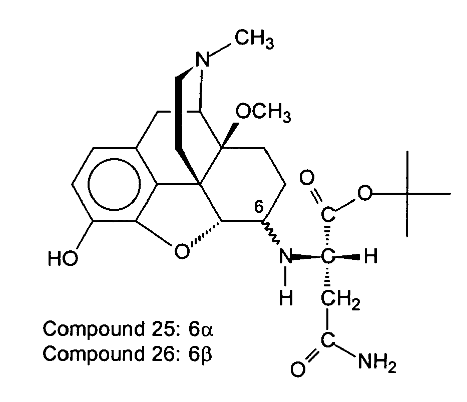

Compound 25: 9% yield; ¹H-NMR (CDCl₃): 6.72 (d, J=8.0, 1 ar. H), 6.49 (d, J=8.0, 1 ar. H), 4.68 (d, J=3.6, H-C(5)), 3.19 (s, MeO), 2.34 (s, MeN), 1.46 (s, t-Bu).

Compound 26: 23% yield; ¹H-NMR (CDCl₃): 6.71 (d, J=8.0, 1 ar. H), 6.57 (d, J=8.0, 1 ar. H), 4.45 (d, J=7.2, H-C(5)), 3.87 (t, J=7.0, NHC*H*), 3.23 (s, MeO), 2.43 (s, MeN), 1.46 (s, t-Bu).

### Example 14:

### (2S)-2-[(4,5α-Epoxy-3-hydroxy-14β-methoxy-17-methylmorphinan-6α-yl)amino]pentanedioic acid di-tert-butyl ester (Compound 27) and (2S)-2-[(4,5α-Epoxy-3-hydroxy-14β-methoxy-17-methy)morphinan-6β-yl)amino]pentanedioic acid di-tert-butyl ester (Compound 28)

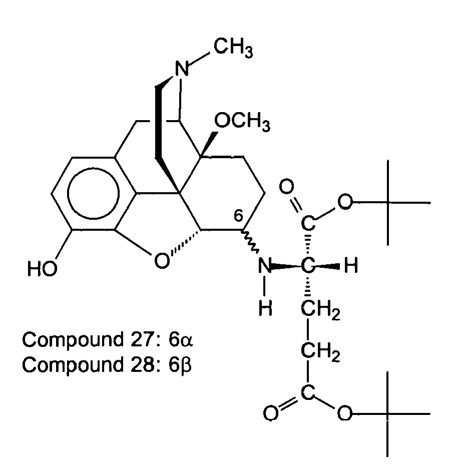

Compound 27: 11 % yield; ¹H-NMR (CDCl₃): 6.71 (d, J=8.0, 1 ar. H), 6.50 (d, J=8.0, 1 ar. H), 4.62 (d, ³J=4.0, ⁴J=1.2, H-C(5)), 3.20 (s, MeO), 2.35 (s, MeN), 1.48 (s, t-Bu), 1.45 (s, t-Bu).

Compound 28: 27% yield; ¹H-NMR (CDCl₃): 6.69 (d, J=8.2, 1 ar. H), 6.55 (d, J=8.0, 1 ar. H), 4.38 (d, J=7.4, H-C(5)), 3.20 (s, MeO), 2.38 (s, MeN), 1.46 (s, 2 × t-Bu).

### Example 15:

### (2S)-4-Carbamoyl-2-[(4,5α-epoxy-3-hydroxy-14β-methoxy-17-methylmorphinan-6α-yl)amino]butyric acid tert-butyl ester (Compound 29) and (2S)-4-Carbamoyl-2-[(4,5α-epoxy-3-hydroxy-14β-methoxy-17-methylmorphinan-6β-yl)amino]butyric acid tert-butyl ester (Compound 30)

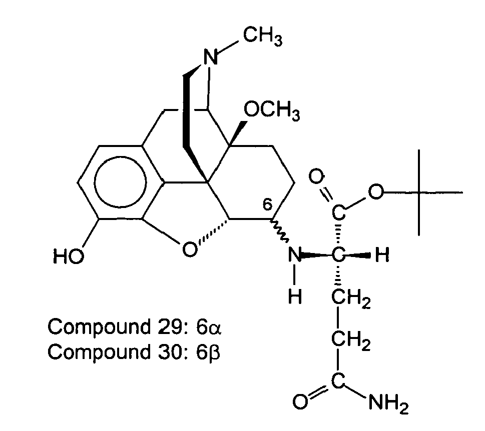

Compound 29: 7% yield; ¹H-NMR (CDCl₃): 6.71 (d, J=8.0, 1 ar. H), 6.49 (d, J=8.0, 1 ar. H), 4.65 (d, J=3.6, H-C(5)), 3.19 (s, MeO), 2.34 (s, MeN), 1.47 (s, t-Bu).

Compound 30: 13% yield; ¹H-NMR (CDCl₃): 6.72 (d, J=8.0, 1 ar. H), 6.56 (d, J=8.0, 1 ar. H), 4.40 (d, J=7.0, H-C(5)), 3.23 (s, MeO), 2.46 (s, MeN), 1.46 (s, t-Bu).

### Example 16:

### (2S)-2-[(4,5α-Epoxy-3-hydroxy-14β-methoxy-17-methylmorphinan-6α-yl)amino]-4-methylthiobutyric acid tert-butyl ester (Compound 31) and (2S)-2-[(4,5α-Epoxy-3-hydroxy-14β-methoxy-17-methylmorphinan-6β-yl)amino]-4-methylthiobutyric acid tert-butyl ester (Compound 32)

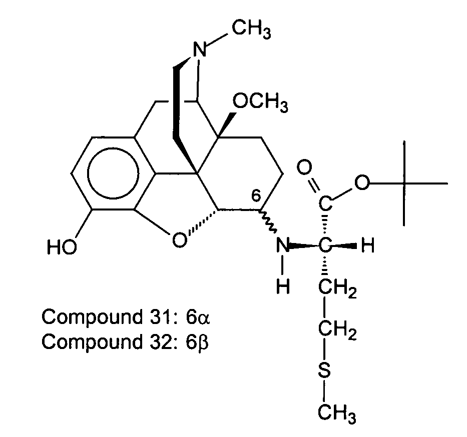

Compound 31: 11% yield; ¹H-NMR (CDCl₃): 6.70 (d, J=8.0, 1 ar. H), 6.50 (d, J=8.0, 1 ar. H), 4.68 (d, J=3.6, H-C(5)), 3.21 (s, MeO), 2.36 (s, MeN), 2.09 (s, MeS), 1.48 (s, t-Bu). Compound 32: 28% yield; ¹H-NMR (CDCl₃): 6.70 (d, J=8.0, 1 ar. H), 6.57 (d, J=8.0, 1 ar. H), 4.38 (d, J=7.2, H-C(5)), 3.24 (s, MeO), 2.45 (s, MeN), 2.09 (s, MeS), 1.44 (s, t-Bu).

### Example 17:

### (2S)-2-[(4,5α-Epoxy-3-hydroxy-14β-methoxy-17-methylmorphinan-6α-yl)amino]-3-(1H-indol-3-yl)propionic acid tert-butyl ester (Compound 33) and (2S)-2-[(4,5a-Epoxy-3-hydroxy-14β-methoxy-17-methylmorphinan-6β-yl)amino]-3-(1H-indol-3-yl)propionic acid tert-butyl ester (Compound 34)

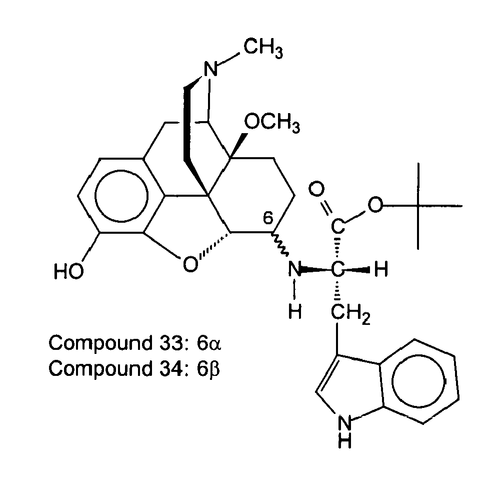

Compound 33: 7% yield; ¹H-NMR (CDCl₃): 7.74-7.12 (m, 4 ar. H, 1 olef. H), 6.63 (d, J=8.0, 1 ar. H), 6.45 (d, J=8.0, 1 ar. H), 4.69 (d, J=3.0, H-C(5)), 3.14 (s, MeO), 2.35 (s, MeN), 1.39 (s, t-Bu).

Compound 34: 40% yield; ¹H-NMR (CDCl₃): 7.59-7.06 (m, 4 ar. H, 1 olef. H), 6.67 (d, J=8.0, 1 ar. H), 6.53 (d, J=8.0, 1 ar. H), 4.39 (d, J=7.8, H-C(5)), 3.18 (s, MeO), 2.40 (s, MeN), 1.28 (s, t-Bu).

### Example 18:

### (2S)-1-[(4,5α-Epoxy-3-hydroxy-14β-methoxy-17-methylmorphinan-6α-yl)]pyrrolidine-2-carboxylic acid tert-butyl ester (Compound 35) and (2S)-1-[(4,5α-Epoxy-3-hydroxy-14β-methoxy-17-methylmorphinan-6β-yl)]pyrrolidine-2-carboxylic acid tert-butyl ester (Compound 36)

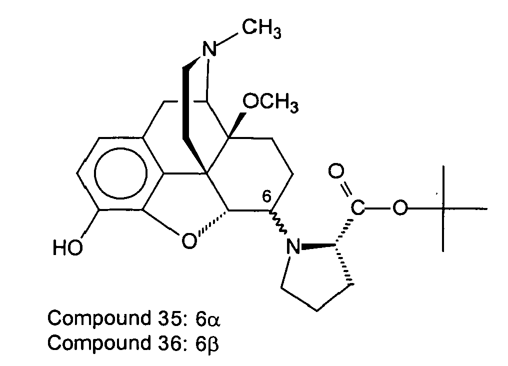

Compound 35:

Compound 36: 8% yield; ¹H-NMR (CDCl₃): 6.69 (d, J=8.2, 1 ar. H), 6.54 (d, J=8.2, 1 ar. H), 4.61 (d, J=7.8, H-C(5)), 3.21 (s, MeO), 2.38 (s, MeN), 1.42 (s, t-Bu).

### Example 19:

### 2-{2-[(4,5α-epoxy-3-hydroxy-14β-methoxy-17-methylmorphinan-6α-yl)amino]acetylamino}acetic acid benzyl ester (Compound 37) and 2-{2-[(4,5α-epoxy-3-hydroxy-14β-methoxy-17-methylmorphinan-6β-yl)amino]acetyamino}acetic acid benzyl ester (Compound 38)

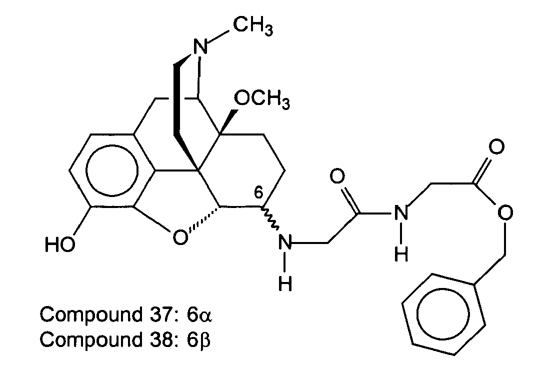

Compound 37: 1 % yield; ¹H-NMR (CDCl₃): 7.36 (s, 5 ar. H), 6.72 (d, J=8.2, 1 ar. H), 6.53 (d, J=8.2, 1 ar. H), 5.22 (s, C*H*₂-Ph), 4.62 (d, J=3.6, H-C(5)), 3.22 (s, MeO), 2.37 (s, MeN).

Compound 38: 13% yield; ¹H-NMR (CDCl₃): 7.33 (s, 5 ar. H), 6.63 (d, J=8.2, 1 ar. H), 6.53 (d, J=8.2, 1 ar. H), 5.27 (d, J=12.4, C*H*₂-Ph), 5.10 (d, J=12.4, C*H*₂-Ph), 4.34 (d, J=7.4, H-C(5)), 3.19 (s, MeO), 2.36 (s, MeN).

### Example 20:

### (2S)-2-{[(4,5α-Epoxy-3-hydroxy-14β-methoxy-17-methylmorphinan-6α-yl)amino]-(2S)-3-methylbutyrylamino}-3-(4-hydroxyphenyl)propionic acid benzyl ester (Compound 39) and (2S)-2-{[(4,5α-Epoxy-3-hydroxy-14β-methoxy-17-methylmorphinan-6β-yl)amino]-(2S)-3-methylbutyrylamino}-3-(4-hydroxyphenyl)propionic acid benzyl ester (Compound 40)

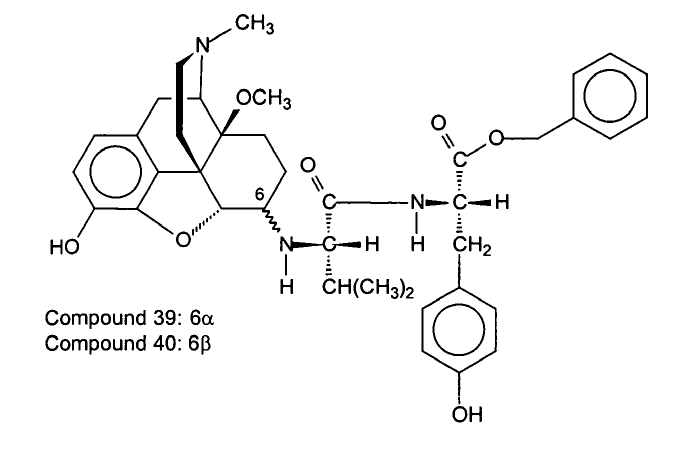

Compound 39: 18% yield; ¹H-NMR (CDCl₃): 7.34-6.49 (m, 11 ar. H), 5.13 (s, CH₂-Ph), 4.60 (d, J=3.6, H-C(5)), 3.17 (s, MeO), 2.42 (s, MeN), 0.90 (d, J=6.7, CHMe), 0.80 (d, J=6.7, CHMe).

Compound 40: 18% yield; ¹H-NMR (CDCl₃):7.32-6.55 (m, 11 ar. H), 5.21 (d, J=12.3, CH₂-Ph), 5.08 (d, J=12.3, C*H*₂-Ph), 4.30 (d, J=7.0, H-C(5)), 3.27 (s, MeO), 2.50 (s, MeN), 0.87 (d, J=6.8, CHMe), 0.67 (d, J=6.8, CHMe).

### Example 21:

### Synthesis of 2-[(4,5α-Epoxy-3-hydroxy-14β-methoxy-17-methylmorphinan-6α-yl)amino]ethanesulfonic acid (Compound 41) and 2-[(4,5α-Epoxy-3-hydroxy-14β-methoxy-17-methylmorphinan-6a-yl)amino]ethanesulfonic acid (Compound 42)

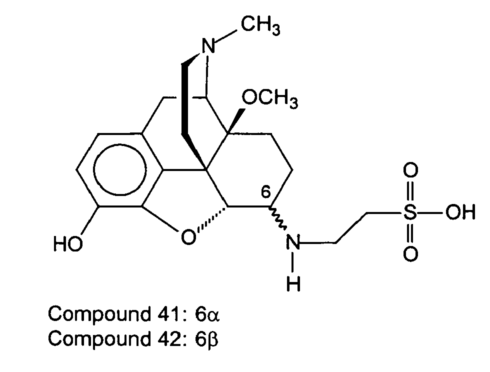

A mixture of 1.00 g (2.52 mmol) 14-O-methyloxymorphone hydrobromide 0.63 g (5.05 mmol) taurine, 0.35 mL (5.05 mmol) triethylamine and 25 mL anhydrous methanol was stirred under inert conditions at room temperature for 3 hours. After addition of 0.16 g (2.52 mmol) sodium cyanoborohydride, the mixture was stirred for 22 days at room temperature. The end of the reaction was monitored by TLC. After addition of 50 mL water, the mixture was evaporated and the residue partitioned between water and dichloromethane. The aqueous phase was evaporated.

Compound 41:

Compound 42:

### Typical procedure for the synthesis of the amino acid derivatives from the corresponing esters: Examples 22-49a, 51-56: Compounds 43-76

A mixture of 1 mmol of the corresponding ester and 5 mL 4 M hydrogen chloride solution in 1,4-dioxane¹ is refluxed until the reaction is completed². The end of the reaction is monitored by TLC. The product is filtered off³, dried and recrystallized from ethanol⁴.
1) The amount of 4 M hydrogen chloride solution in dioxane can vary from 2 to 15 mL.
2) The reaction time can range from 20 min to 12 hours.
3) The filtered off product can be washed with 1,4-dioxane or diethyl ether.
4) The product can be recrystallized from other alcohols, such as methanol or isopropanol. It can also be isolated by evaporating the reaction mixture. Or the residue can be dissolved in water and freeze dried to obtain a lyophilisate.

The following examples show the analytical data of the amino acids:

### Example 22:

### (2S)-2-[(4,5α-Epoxy-3,14β-dihydroxy-17-methylmorphinan-6α-yl)amino]propionic acid Dihydrochloride (Compound 43)

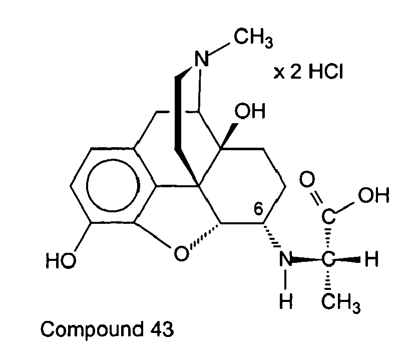

42% yield; ¹H-NMR (D₂O): 6.83 (d, J=8.3, 1 ar. H), 6.74 (d, J=8.3, 1 ar. H), 4.98 (d, J=3.8, H-C(5)), 2.85 (s, MeN), 1.54 (d, J=7.0, NHCHMe).

### Example 23:

### (2S)-2-[(4,5α-Epoxy-3,14β-dihydroxy-17-methylmorphinan-6β-yl)amino]propionic acid Dihydrochloride (Compound 44)

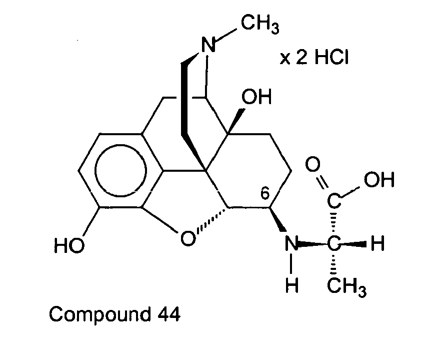

53% yield; ¹H-NMR (D₂O): 6.90 (d, J=8.3, 1 ar. H), 6.85 (d, J=8.3, 1 ar. H), 4.89 (d, J=8,0, H-C(5)), 2.91 (s, MeN), 1.55 (d, J=6,8, NHCHMe).

### Example 24:

### (2S)-2-[(4,5α-Epoxy-3,14β-dihydroxy-17-methylmorphinan-6α-yl)amino]-3-phenylpropionic acid Dihydrochloride (Compound 45)

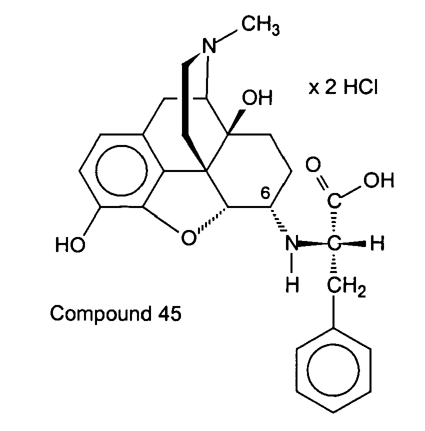

62% yield; ¹H-NMR (D₂O): 7.33 (s, 5 ar. H), 6.82 (d, J=8.1, 1 ar. H), 6.74 (d, J=8.1, 1 ar. H), 4.92 (ps-s, H-C(5)), 4.33 (t, J=6.6, NHCH), 2.86 (s, MeN).

### Example 25:

### (2S)-2-[(4,5α-Epoxy-3,14β-dihydroxy-17-methylmorphinan-6β-yl)amino]-3-phenylpropionic acid Dihydrochloride (Compound 46)

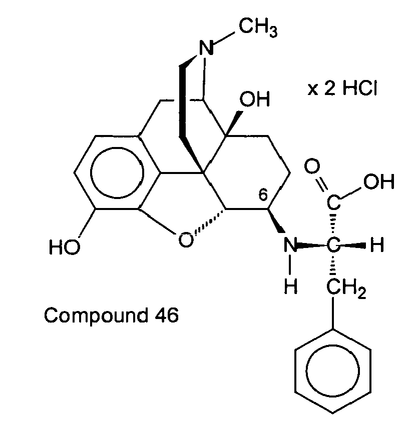

75% yield; ¹H-NMR (D₂O): 7.24 (s, 5 ar. H), 6.83 (d, J=8.1, 1 ar. H), 6.77 (d, J=8.1, 1 ar. H), 4.81 (d, J=7.5, H-C(5)), 4.37 (t, J=7.1, NHC*H*), 2.81 (s, MeN).

### Example 26:

### 3-[(4,5α-Epoxy-3-hydroxy-14β-methoxy-17-methylmorphinan-6α-yl)amino]propionic acid Dihydrochloride (Compound 47)

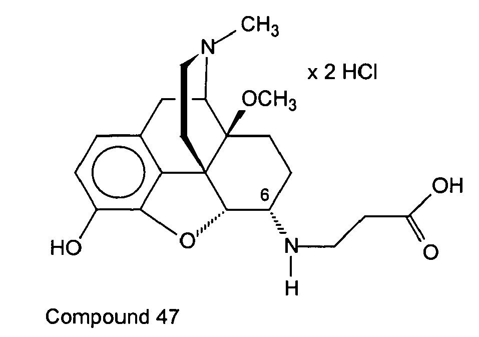

88% yield; ¹H-NMR (D₂O): 6.82 (d, J=8.3, 1 ar. H), 6.74 (d, J=8.3, 1 ar. H), 5.01 (d, J=3.8, H-C(5)), 3.28 (s, MeO), 2.87 (s, MeN).

### Example 27:

### 3-[(4,5α-Epoxy-3-hydroxy-14β-methoxy-17-methylmorphinan-6β-yl)amino]propionic acid Dihydrochloride (Compound 48)

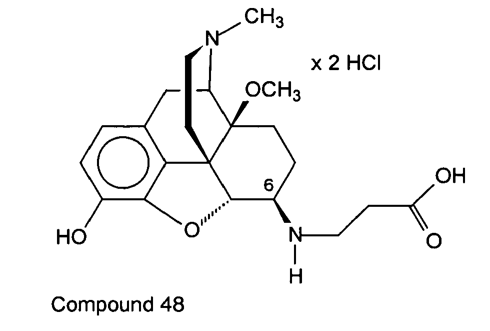

56% yield; ¹H-NMR (D₂O): 6.83 (d, J=8.4, 1 ar. H), 6.78 (d, J=8.4, 1 ar. H), 4.81 (d, J=7.8, H-C(5)), 3.25 (s, MeO), 2.85 (s, MeN).

### Example 28:

### 3-[(17-Cyclopropylmethyl-4,5α-epoxy-3-hydroxy-14β-methoxymorphinan-6α-yl)amino]propionic acid Dihydrochlroide (Compound 49)

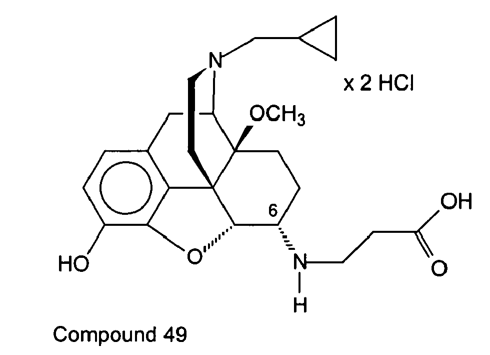

48% yield; ¹H-NMR (D₂O): 6.83 (d, J=8.3, 1 ar. H), 6.73 (d, J=8.3, 1 ar. H), 5.02 (d, J=3.4, H-C(5)), 3.33 (s, MeO), 1.44 (s, t-Bu), 0.71 (m, CH₂-cp), 0.40 (m, CH₂-cp).

### Example 29:

### 3-[(17-Cyclopropylmethyl-4,5α-epoxy-3-hydroxy-14β-methoxymorphinan-6β-yl)amino]propionic acid Dihydrochlroide (Compound 50)

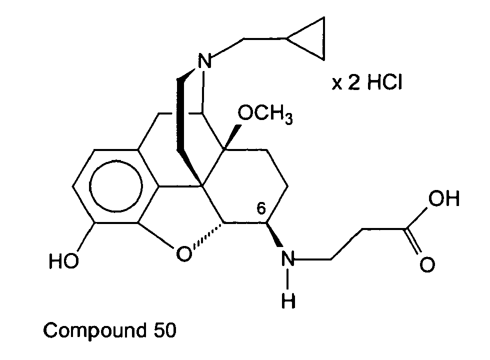

92% yield; ¹H-NMR (D₂O): 6.85 (d, J=8.4, 1 ar. H), 6.80 (d, J=8.4, 1 ar. H), 4.85 (d, J=7.6, H-C(5)), 3.32 (s, MeO), 0.74 (m, CH₂-cp), 0.43 (m, CH₂-cp).

### Example 30:

### 4-[(4,5α-Epoxy-3-hydroxy-14β-methoxy-17-methylmorphinan-6α-yl)amino]butyric acid Dihydrochloride (Compound 51)

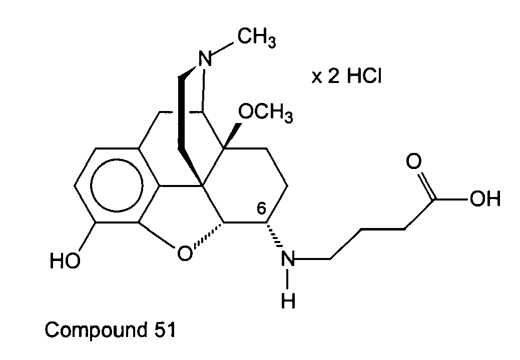

46% yield; ¹H-NMR (D₂O): 6.83 (d, J=8.2, 1 ar. H), 6.74 (d, J=8.2, 1 ar. H), 4.98 (d, J=3.6, H-C(5)), 3.29 (s, MeO), 2.88 (s, MeN).

### Example 31:

### 4-[(4,5α-Epoxy-3-hydroxy-14β-methoxy-17-methy)morphinan-6β-yl)amino]butyric acid Dihydrochloride (Compound 52)

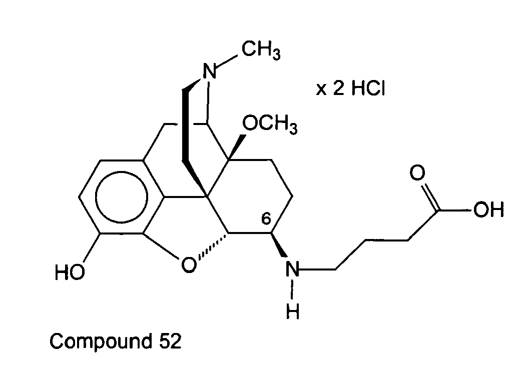

73% yield; ¹H-NMR (D₂O): 6.83 (d, J=8.1, 1ar. H), 6.78 (d, j=8.1, 1 ar. H), 4.78 (d, J=7.6, H-C(5)), 3.25 (s, MeO), 2.85 (s, MeN).

### Example 32:

### 4-[(17-Cyclopropylmethyl-4,5α-epoxy-3-hydroxy-14β-methoxymorphinan-6α-yl)amino]butyric acid Dihydrochloride (Compound 53)

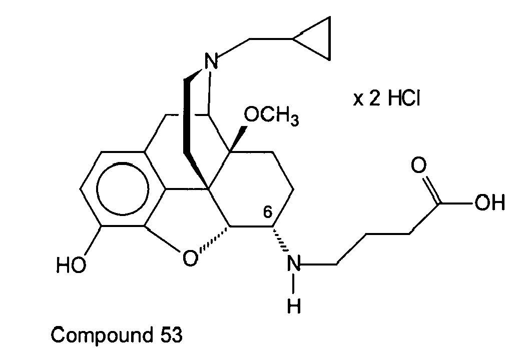

48% yield; ¹H-NMR (D₂O): 6.83 (d, J=8.0, 1 ar. H), 6.74 (d, J=8.0, 1 ar. H), 4.98 (d, J=2.8, H-C(5)), 3.33 (s, MeO), 0.71 (m, CH₂-cp), 0.40 (m, CH₂-cp).

### Example 33:

### 4-[(17-Cyclopropylmethyl-4,5α-epoxy-3-hydroxy-14β-methoxymorphinan-6β-yl)amino]butyric acid Dihydrochloride (Compound 54)

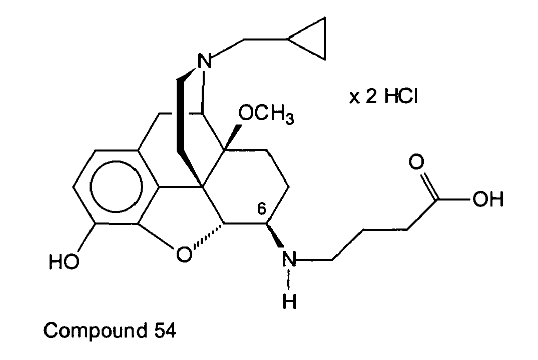

75% yield; ¹H-NMR (D₂O): 6.85 (d, J=8.5, 1 ar. H), 6.79 (d, J=8.5, 1 ar. H), 4.81 (d, J=7.4, H-C(5)), 3.32 (s, MeO), 0.73 (m, CH₂-cp), 0.43 (m, CH₂-cp).

### Example 34:

### (2S)-2-[(4,5α-Epoxy-3,14β-dihydroxy-17-methylmorphinan-6α-yl)amino]-3-methylbutyric acid Dihydrochloride (Compound 55)

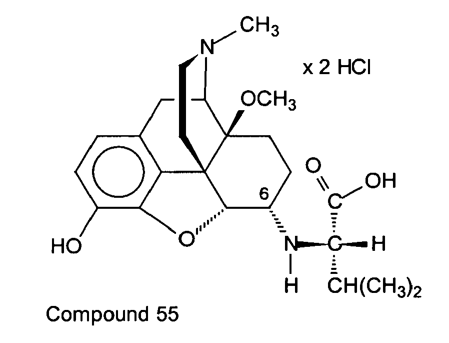

84% yield; ¹H-NMR (D₂O): 6.83 (d, J=8.2, 1 ar. H), 6.74 (d, J=8.0, 1 ar. H), 4.99 (d, J=2.8, H-C(5)), 3.26 (s, MeO), 2.86 (s, MeN), 1.01 (d, J=6.8, CHMe), 1.00 (d, J=6.8, CHMe).

### Example 35:

### (2S)-2-[(4,5α-Epoxy-3,14β-dihydroxy-17-methylmorphinan-6β-yl)amino]-3-methylbutyric acid Dihydrochloride (Compound 56)

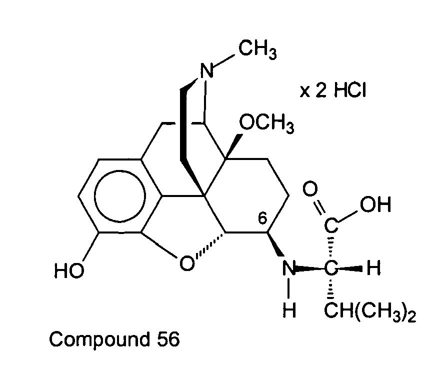

97% yield; ¹H-NMR (D₂O): 6.80 (d, J=8.1, 1 ar. H), 6.76 (d, J=8.1, 1 ar. H), 4.82 (d, J=7.8, H-C(5)), 3.24 (s, MeO), 2.84 (s, MeN), 0.99 (d, J=7.0, CHMe), 0.93 (d, J=7.0, CHMe).

### Example 36:

### (2R)-2-[(4,5α-Epoxy-3,14β-dihydroxy-17-methylmorphinan-6α-yl)amino]-3-methylbutyric acid Dihydrochloride (Compound 57)

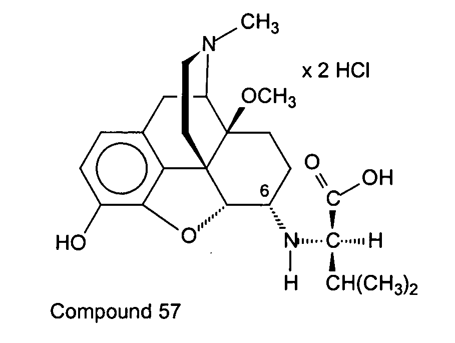

95% yield; ¹H-NMR (D₂O): 6.82 (d, J=8.4, 1 ar. H), 6.73 (d, J=8.4, 1 ar. H), 4.95 (d, J=2.8, H-C(5)), 3.25 (s, MeO), 2.86 (s, MeN), 1.02 (d, J=7.0, CHMe), 0.95 (d, J=7.0, CHMe).

### Example 37:

### (2R)-2-[(4,5α-Epoxy-3,14β-dihydroxy-17-methylmorphinan-6β-yl)amino]-3-methylbutyric acid Dihydrochloride (Compound 58)

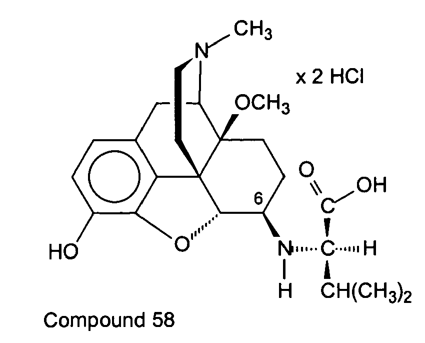

99% yield; ¹H-NMR (D₂O): 6.81 (d, J=8.6, 1 ar. H), 6.76 (d, J=8.6, 1 ar. H), 4.83 (d, J=8,0, H-C(5)), 3.24 (s, MeO), 2.84 (s, MeN), 1.00 (d, J=7.5, CHMe), 0.96 (d, J=7.5, CHMe).

### Example 38:

### (2R)-2-[(17-Cyclopropylmethyl-4,5α-epoxy-3-hydroxy-14β-methoxymorphinan-6α-yl)amino]-3-methylbutyric acid Dihydrochloride (Compound 59)

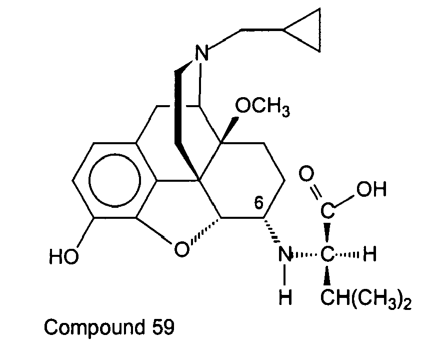

74% yield; ¹H-NMR (D₂O): 6.82 (d, J=8.5, 1 ar. H), 6.72 (d, J=8.5, 1 ar. H), 4.95 (d, J=2.8, H-C(5)), 3.29 (s, MeO), 1.02 (d, J=7.0, CHMe), 0.94 (d, J=7.0, CHMe), 0.69 (m, CH₂-cp), 0.37 (m, CH₂-cp).

### Example 39:

### (2S)-2-[(4,5α-Epoxy-3-hydroxy-14β-methoxy-17-methylmorphinan-6β-yl)amino]butanedioic acid Dihydrochloride (Compound 60)

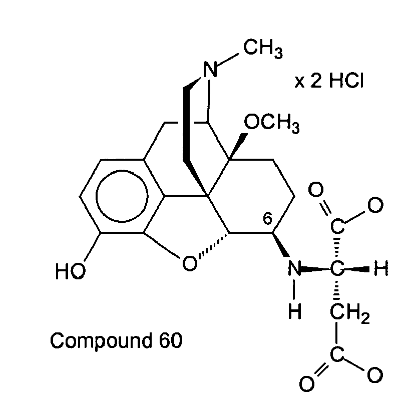

77% yield; ¹H-NMR (DMSO-d₆): 6.82 (d, J=8.1, 1 ar. H), 6.70 (d, J=8.1, 1 ar. H), 4.84 (d, J=7.2, H-C(5)), 4.34 (t, J=4.8, NHCH), 3.26 (s, MeO), 2.85 (d, J=3.4, MeN).

### Example 40:

### (2S)-3-Carbamoyl-2-[(4,5α-epoxy-3-hydroxy-14β-methoxy-17-methylmorphinan-6α-yl)amino]propionic acid Dihydrochloride (Compound 61)

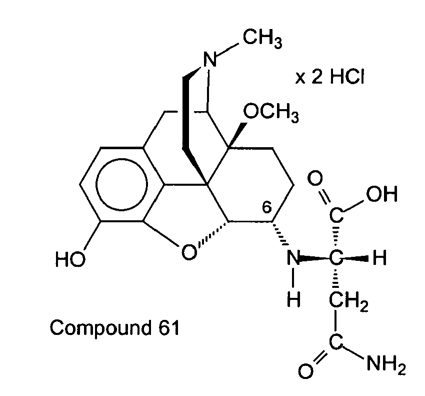

97% yield; ¹H-NMR (D₂O): 6.83 (d, J=8.0, 1 ar. H), 6.74 (d, J=8.0, 1 ar. H), 5.00 (d, J=3.4, H-C(5)), 3.28 (s, MeO), 2.87 (s, MeN).

### Example 41:

### (2S)-3-Carbamoyl-2-[(4,5α-epoxy-3-hydroxy-14β-methoxy-17-methylmorphinan-6β-yl)amino]propionic acid Dihydrochloride (Compound 62)

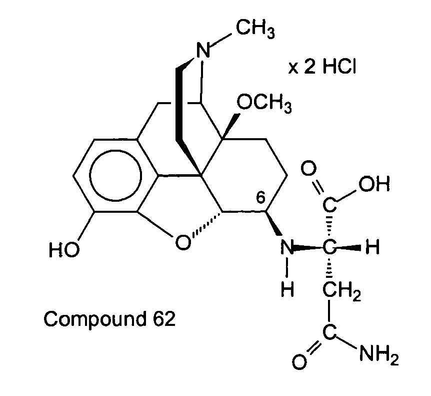

99% yield; ¹H-NMR (D₂O): 6.79 (s, 2 ar. H), 4.89 (d, J=7.4, H-C(5)), 3.25 (s, MeO), 2.84 (s, MeN).

### Example 42:

### (2S)-2-[(4,5α-Epoxy-3-hydroxy-14β-methoxy-17-methylmorphinan-6α-yl)amino]pentanedioic acid Dihydrochloride (Compound 63)

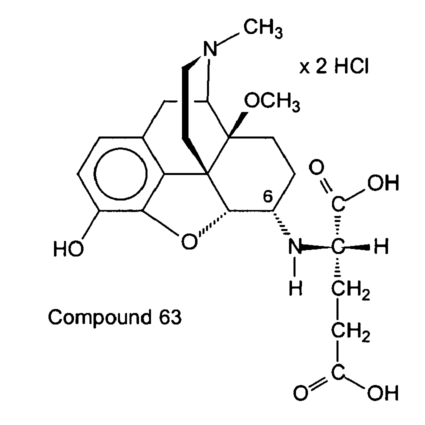

38% yield; ¹H-NMR (D₂O): 6.82 (d, J=8.4, 1 ar. H), 6.73 (d, J=8.4, 1 ar. H), 4.94 (d, J=3.0, H-C(5)), 3.27 (s, MeO), 2.86 (s, MeN).

### Example 43:

### (2S)-2-[(4,5α-Epoxy-3-hydroxy-14β-methoxy-17-methylmorphinan-6β-yl)amino]pentanedioic acid Dihydrochloride (Compound 64)

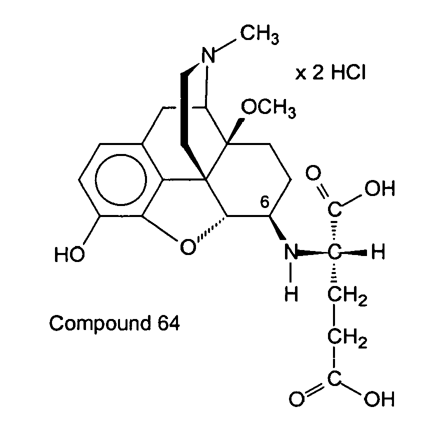

99% yield; ¹H-NMR (D₂O): 6.78 (s, 2 ar. H), 4.81 (d, J=7.8, H-C(5)), 3.24 (s, MeO), 2.84 (s, MeN).

### Example 44:

### (2S)-4-Carbamoyl-2-[(4,5α-epoxy-3-hydroxy-14β-methoxy-17-methylmorphinan-6α-yl)amino]butyric acid Dihydrochloride (Compound 65)

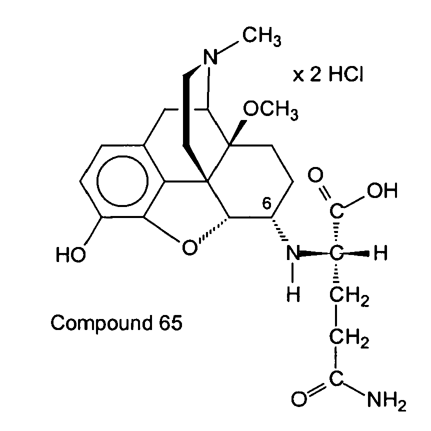

67% yield; ¹H-NMR (D₂O): 6.82 (d, J=8.4, 1 ar. H), 6.73 (d, J=8.4, 1 ar. H), 4.95 (d, J=3.6, H-C(5)), 3.27 (s, MeO), 2.86 (s, MeN).

### Example 45:

### (2S)-4-Carbamoyl-2-[(4,5cc-epoxy-3-hydroxy-1 40-methoxy-1 7-methylmorphinan-6p-yl)amino]butyric acid Dihydrochloride (Compound 66)

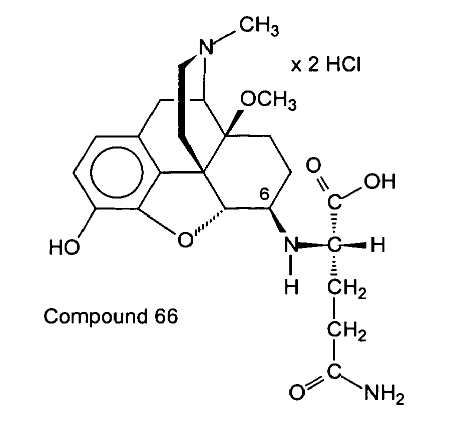

63% yield; ¹H-NMR (D₂O): 6.78 (s, 2 ar. H), 4.82 (d, J=6,8, H-C(5)), 3.25 (s, MeO), 2.84 (s, MeN).

### Example 46:

### (2S)-2-[(4,5α-Epoxy-3-hydroxy-14β-methoxy-17-methylmorphinan-6α-yl)amino]-4-methylthiobutyric acid Dihydrochloride (Compound 67)

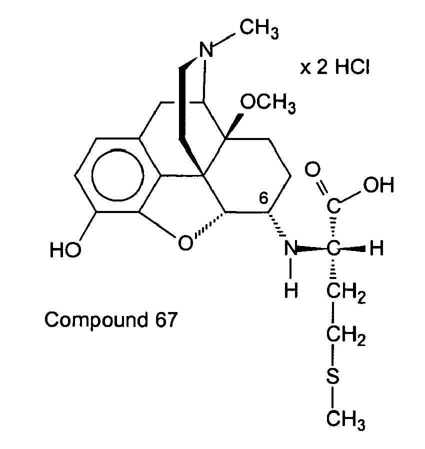

92% yield; ¹H-NMR (D₂O): 6.83 (d, J=8.2, 1 ar. H), 6.73 (d, J=8.2, 1 ar. H), 4.97 (d, J=3.4, H-C(5)), 3.27 (s, MeO), 2.86 (s, MeN), 2.06 (s, MeS).

### Example 47:

### (2S)-2-[(4,5α-Epoxy-3-hydroxy-14β-methoxy-17-methylmorphinan-6β-yl)amino]-4-methylthiobutyric acid Dihydrochloride (Compound 64)

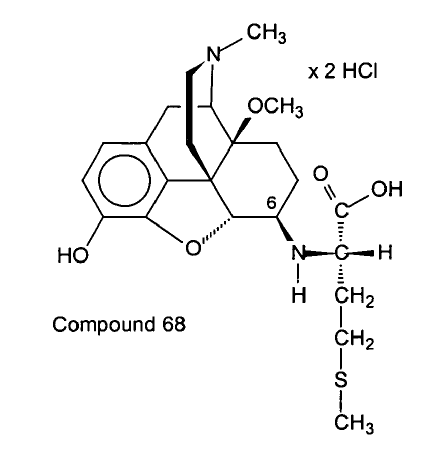

94% yield; ¹H-NMR (D₂O): 6.78 (s, 2 ar. H), 4.81 (d, J=7,2, H-C(5)), 3.24 (s, MeO), 2.84 (s, MeN), 2.00 (s, MeS).

### EMBODIMENT 2

Compounds of formula (Ia), 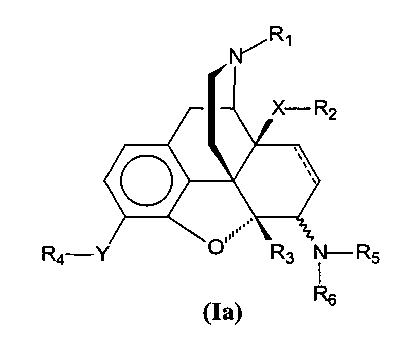 in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl; iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₀-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl-and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cydoalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably C₉-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1H-imidazol-2-yl, 1,4,5,6-tetrahydropyrimidin-2-yl, 4,5,6,7-tetrahydro-1H-[1,3]diazepin-2-yl;
X is oxygen, sulphur or methylene or the group (X-R₂) is H and
Y is oxygen or the group (Y-R₄) is H;
and pharmaceutically acceptable acid addition salts as well as base addition salts and easily accessible derivatives (e.g. esters or amides of amino acid derivatives). Some compounds of EMBODIMENT 2 may exist in different stereochemical configuration and/or may show more than one crystalline structure, in particular the compounds possessing one or more chiral carbon atom. The present invention comprises all those specific embodiments, such as diastereomer, enantiomers, polymorphs etc, in any given or desired mixture or in isolated form.

The various terms as employed above do have the following meaning and preferred embodiments are as follows:

The dotted line between the carbon atoms 7 and 8 of the morphinan skeleton designates that these carbon atoms may be unsaturated (double bond between C7 and C8) or saturated (single bond between C7 and C8).

In this invention the terms alkyl, alkenyl and alkynyl include both branched and also unbranched alkyl, alkenyl and alkynyl groups as well as mono-, di- and trihydroxy- substituted branched and unbranched alkyl, alkenyl and alkynyl groups. These groups furthermore may be substituted once twice or three times with substituents selected independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents are cyclic groups, including carbocycles and heterocycles which may be saturated unsaturated or aromatic. Preferred examples comprise from 3 to 8 ring atoms, selected from C, N, O, and S. Aryl can be unsubstituted or mono-, di- or tri-substituted, whereby the substituents can be chosen independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents are cyclic groups, including carbocycles and heterocycles which may be saturated unsaturated or aromatic. Preferred examples comprise from 3 to 8 ring atoms, selected from C, N, O, and S. The term aryl defines aromatic rings comprising preferably from 5 to 14 ring atoms and the term aryl comprises furthermore carbocyclic aryl groups as well as heterocyclic aryl groups, comprising preferably from 1 to 3 heteroatoms selected from N, O and S. The aryl goups as defined above may furthermore be fused ring systems such as naphthyl or anthracenyl or the corresponding heterocyclic groups comprising from 1 to 3 heteroatoms selected from N, O, and S. The definitions listed above for alkyl, alkenyl, alkynyl and aryl are valid for all substituents of this application.

The compounds of this invention contain pharmaceutically and pharmacologically acceptable salts of the compounds of formula (1a). According to this invention both inorganic and also organic salts are suitable. Examples of suitable inorganic salts for this invention are hydrochlorides, hydrobromides, hydroiodides, sulphates, phosphates and tetrafluoroborates. Possible organic salts are, for example, acetates, tartrates, lactates, benzoates, stearates, pamoates, methane sulphonates, salicylates, fumarates, maleinates, succinates, aspartates, citrates, oxalates, trifluoroacetates and orotates.

Acid addition salts are preferred as conventional pharmaceutically acceptable addition salts, particularly preferred are the hydrochlorides, hydrobromides, hydroiodides, tetrafluoroborates and trifluoroacetates. X and Y are preferably oxygen. Preferably R₁ is alkyl as defined above, in particular methyl or ethyl, whereby methyl is preferred, or cycloalkylalkyl, preferably cyclopropylmethyl. R₂ is preferably not H and also not a group which forms an ester unit with X. The other definitions for R₂ as defined in claim 4 are, in contrast, preferred, whereby especially alkyl as defined above is preferred, particularly preferred are methyl, ethyl and propyl, where necessary substituted, e.g. with a phenyl group, for example to produce a 3-phenylpropyl group (i.e., put differently, an arylalkyl group is also preferred for R₂, in particular 3-phenylpropyl). R₁ and R₂ are especially preferably both simultaneously alkyl, in particular either both simultaneously methyl or methyl (R₁) and ethyl (R₂). A further preferred combination of R₁ and R₂ is cycloalkylalkyl, in particular cyclopropylmethyl for R₁ and arylalkyl, preferably phenylpropyl for R₂. R₃ and R₄ are in each case preferably hydrogen or alkyl, whereby methyl is especially preferred as an alkyl group. R₄ is in addition preferred as C(N-Boc)(NH-Boc). R₅ and R₆ are preferably chosen such that one is H and the other is different to H, whereby this radical, different to H, is preferably not halogenated. R₅ and R₆ are preferably selected, independent of one another, from hydrogen, CH₂COOC(CH₃)₃, CH₂COOH, CH(CH₃)COOC(CH₃), CH(CH₃)COOH, CH(CH₂Ph)COOC(CH₃)₃, CH(CH₂Ph)COOH, C(N-Boc)NH-BOC and C(NH)NH₂, whereby R₆ is preferably H and R₅ is preferably one of the groups mentioned above or is H. Also preferred, R₅ and R₆ are both H.

In a specially preferred representation X and Y are oxygen. Then preferably, R₁ is methyl and cyclopropylmethyl and R₂ is alkyl and arylalkyl, in particular methyl and 3-phenylpropyl, and R₃, R₄ and R₆ are hydrogen.

Preferred compounds of the present invention are further the base addition salts, comprising metal salts, such as lithium salts, sodium salts, potassium salts, beryllium salts, magnesium salts, calcium salts, strontium salts, aluminum salts and zinc salts; ammonium salts, such as C₁-C₃₀ monoalkylammonium salts, C₁-C₃₀ dialkylammonium salts, C₁-C₃₀ trialkylammonium salts, C₁-C₃₀ tetraalkylammonium salts; C₂-C₃₀ monoalkenylammonium salts, C₂-C₃₀ dialkenylammonium salts, C₂-C₃₀ trialkenylammonium salts, C₂-C₃₀ tetraalkenylammonium salts; C₂-C₃₀ monoalkynylammonium salts, C₂-C₃₀ dialkynylammonium salts, C₂-C₃₀ trialkynylammonium salts, C₂-C₃₀ tetraalkynylammonium salts; C₄-C₃₀ mono(cycloalkylalkylammonium) salts, C₄-C₃₀ di(cycloalkylalkylammonium) salts, C₄-C₃₀ tri(cycloalkylalkylammonium) salts, C₄-C₃₀ tetra(cycloalkylalkylammonium) salts, where cycloalkyl is C₃-C₁₀-cycloalkyl and alkyl is C₁-C₂₇-alkyl; C₅-C₃₀ mono(cycloalkylalkenylammonium) salts, C₅-C₃₀ di(cycloalkylalkenylammonium) salts, C₅-C₃₀ tri(cycloalkylalkenylammonium) salts, C₅-C₃₀ tetra(cycloalkylalkenylammonium) salts, where cycloalkyl is C₃-C₁₀-cycloalkyl and alkenyl is C₂-C₂₇-alkenyl; C₅-C₃₀ mono(cycloalkylalkynylammonium) salts, C₅-C₃₀ di(cycloalkylalkynylammonium) salts, C₅-C₃₀ tri(cycloalkylalkynylammonium) salts, C₅-C₃₀ tetra(cycloalkylalkynylammonium) salts, where cycloalkyl is C₃-C₁₀-cycloalkyl and alkynyl is C₂-C₂₇-alkynyl; C₇-C₃₀ mono(arylalkylammonium) salts, C₇-C₃₀ di(arylalkylammonium) salts, C₇-C₃₀ tri(arylalkylammonium) salts, C₇-C₃₀ tetra(arylalkylammonium) salts, where aryl is C₆-C₁₀-aryl and alkyl is C₁-C₂₄-alkyl; C₈-C₃₀ mono(arylalkenylammonium) salts, C₈-C₃₀ di(arylalkenylammonium) salts, C₈-C₃₀ tri(arylalkenylammonium) salts, C₈-C₃₀ tetra(arylalkenylammonium) salts, where aryl is C₆-C₁₀-aryl and alkenyl is C₂-C₂₄-alkenyl; C₈-C₃₀ mono(arylalkynylammonium) salts, C₈-C₃₀ di(arylalkynylammonium) salts, C₈-C₃₀ tri(arylalkynylammonium) salts, C₈-C₃₀ tetra(arylalkynylammonium) salts, where aryl is C₆-C₁₀-aryl and alkynyl is C₂-C₂₄-alkynyl, combinations of the ammonium salts listed above, and salts derived from heterocyclic bases, in particular heterocyclic nitrogen bases. These include salts derived from heterocyclic compounds comprising the following cycles: pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, pyrrolidine, imidazolidine, pyrazolidine, oxazolidine, isoxazolidine, thiazolidine, isothiazolidine, thiadiazolidine, sulpholane, imidazolidine, pyridine, pyridazine, pyrazine, pyrimidine, piperazine, piperidine, morpholine, tetrazole, triazole, triazolidine, tetrazolidine, azepine, homopiperazine and azetidine.

Particular preferred examples of compounds of EMBODIMENT 2 are the base addition salts and the following specific examples:
2-[(4,5α-Epoxy-3-hydroxy-14β-methoxy-17-methylmorphinan-6α-yl)amino]acetic acid Dihydrochloride
2-[(4,5α-Epoxy-3-hydroxy-14β-methoxy-17-methylmorphinan-6β-yl)amino]acetic acid Dihydrochloride (compound 70 and polymorphic forms thereof)
(2S)-2-[(4,5α-Epoxy-3-hydroxy-14β-methoxy-17-methylmorphinan-6α-yl)amino]propionic acid Dihydrochloride
(2S)-2-[(4,5α-Epoxy-3-hydroxy-14β-methoxy-17-methylmorphinan-6β-yl)amino]propionic acid Dihydrochloride
(2S)-2-[(4,5α-Epoxy-3-hydroxy-14β-methoxy-17-methylmorphinan-6α-yl)amino]-3-phenylpropionic acid Dihydrochloride
(2S)-2-[(4,5α-Epoxy-3-hydroxy-14β-methoxy-17-methylmorphinan-6β-yl)amino]-3-phenylpropionic acid Dihydrochloride
(2S)-2-[(4,5α-Epoxy-3-hydroxy-14β-methoxy-17-methylmorphinan-6α-yl)amino]-3-(4-hydroxyphenyl)propionic acid Dihydrochloride
(2S)-2-[(4,5α-Epoxy-3-hydroxy-14β-methoxy-17-methylmorphinan-6β-yl)amino]-3-(4-hydroxyphenyl)propionic acid Dihydrochloride

It has now been found that the compounds of the pertinent invention represent effective opioid receptor ligands of the type 6-aminomorphinan and exhibit a high therapeutic application potential as analgesics, as immunomodulators with immunostimulating or immunosuppressive effect, as cancer therapeutics, inflammation inhibitors, as anti-rheumatics, diuretics, anorectics, as an agent against diarrhoea, anaesthetics or as neuroprotective active substances.

The compounds quoted in the claims are therefore potentially applicable to the treatment of pain, functional intestinal diseases, such as abdominal pain, intestinal obstruction (ileus) or obstipation, for the treatment of mammals, in particular humans, for the treatment of Raynaud's disease, for the treatment of complaints caused by vasoconstriction, for the treatment of dysmenorrhoea, angina pectoris, myocardial infarct, emphysema, bronchial spasms, chronic obstructive bronchitis, rheumatic complaints, nephrosis, nephritis in conjunction with rheumatic diseases, for the treatment of tumours, phaeochromocytoma, Addison's disease, hepatic cirrhosis, chronic inflammation of the small and large intestines (e.g. irritable colon syndrome - colon irritabile, colitis ulcerosa, morbus Crohn), addiction withdrawal of, for example, opiates, cocaine or alcohol, or for the treatment of psychic diseases such as dysphoria or schizophrenia.

The compounds of this invention are suitable for application in the production of a medicament for the treatment of pain, including acute and chronic pain, on the locomotor system such as pain in the neck, back, hip, knee, shoulder or myofacial pain, treatment of complex regional pain syndromes, phantom pain, facial neuralgia, rheumatalgia, cancer pain, pain from burns, pain after accidents, pain due to chronic inflammation, visceralgia, headaches such as for example tension headaches, cervically related headache or migraine, pain after central lesions such as for example with paraplegia or thalamic lesions, neuralgic pain such as zoster neuralgia, postzoster neuralgia, ischaemic pain such as angina pectoris or peripheral occlusive arterial disease, postoperative pain, neuropathic pain such as pain with diabetic neuropathy, pain after virus infections or pain after nerve lesions.

The pharmaceutical compositions according to the invention, which contain a compound of this invention and / or a pharmaceutically acceptable salt of it as active ingredient together with a pharmaceutically acceptable carrier substance, are suitable for the treatment of the conditions quoted in the description.

The application according to the invention includes application as analgesic, immunomodulating, antitumour, antiproliferative, anti-inflammatory, antirheumatic, diuretic, anorectic, antidiarrhoeal, anaesthetic, neuroprotective active substance and as active substance for the prevention and treatment of intestinal obstruction (ileus).

Preferred applications take place for the production of a medicament for the treatment of pain, functional intestinal diseases, of the Raynaud's disease, for the treatment of complaints caused by vasoconstriction, angina pectoris, myocardial infarct, emphysema, bronchial spasms, chronic obstructive bronchitis, rheumatic complaints (including rheumatoid arthritis, arthrosis, osteoarthritis, spondylosis, lumbago, lupus erythematosus, spondyarthropathy), nephrosis, nephritis in conjunction with rheumatic diseases, for the treatment of tumours, cancer, phaeochromocytoma, Addison's disease, hepatic cirrhosis, chronic inflammation of the small and large intestines (e.g. irritable colon syndrome - colon irritabile, colitis ulcerosa, morbus Crohn), for the treatment of drug abuse, psychic diseases, erectile dysfunction and / or for the suppression of rejection of transplants after transplantation on mammals, particularly on humans.

Surprisingly it was also found that the compounds of this invention were not capable of overcoming the blood-brain barrier or only to a slight extent, and therefore a special significance could be attributed to them with regard to their application as peripherally effective therapeutics, for example as medicaments for the treatment of pain, rheumatic therapy, suppression of organ rejection after transplantations on mammals, particularly humans and also for the treatment of erectile disturbances. The limited access to the central nervous system is accompanied by a much reduced rate of side effects relating to central side effects such for example nausea, vomiting, sedation, dizziness, confusion, respiratory depression and mania.

In addition, it was surprisingly found that the compounds of this invention have a very long analgesically effective period. This enables a lower dosage and less frequent administration of the medicament, which results in a lower rate of side effects and toxicity as well as a higher readiness of patients to take the medicament.

When a compound exists in more than one crystallographic distinguishable form it is called polymorphic. Polymorphism often arises as a result of particular processing conditions used to synthesize the compound. In pharmaceutical applications, the polymorphic state (form) can have great influence on physical (e.g. solubility), chemical (e.g. stability) and biological (e.g. bioavailability) properties of a compound.

Compound 70 was obtained in different crystallographic forms. When synthesized as described in example 49a, compound 70 crystallized from 96% ethanol as monohydrate ethanolate in a highly crystalline modification (Form A) as shown by powder X-ray diffraction (Figure 1, upper curve). This X-ray powder diffraction pattern is concordant with the calculated pattern (Figure 1, lower curve) from the CIF file of the single crystal X-ray diffraction of compound 70 from example 49a (Figure 2).

Surprisingly, compound 70 was obtained in the same modification (Form A) when synthesized following an alternative pathway as described in example 49b shown by powder X-ray diffraction (Figure 3).

In example 50a, the thermal behaviour of compound 70 (Form A) is investigated. Thermogravimetry of compound 70 (Form A) shows a loss of mass above ca. 125°C (Figure 4, upper curve). Differential scanning calorimetry shows a phase transition at ca. 165°C (Figure 4, lower curve), above which compound 70 exists in an amorphous modification (Form B) as shown by powder X-ray diffraction (Figure 5). Surprisingly, this Form B can be converted into Form A by treatment with 96% ethanol followed by evaporation (example 50b, Figure 6).

Examples 50c - 50j show the crystallographic effects of treatment of compound 70 (Form A) with different solvents: Compound 70 was treated with water (example 50c), methanol (example 50d), n-propanol (example 50e) and isopropanol (example 50f), respectively, and then evaporated. Treatment with water resulted in the amorphous Form C (Figure 7). Form C does not contain ethanol anymore as shown by ¹H-NMR. Treatment of Form A with methanol gave the amorphous the Form D (Figure 8) of compound 70. In contrast, treatment with both n-propanol (Figure 9) and isopropanol (Figure 10) resulted in Form A of compound 70. Surprisingly, treatment of amorphous Form C (from treatment with water) gave once again the crystalline Form A of compound 70 (example 50g, Figure 11). The same result was obtained with amorphous Form D (from treatment with methanol, example 50h, Figure 12). Additional peaks in the powder X-ray diffraction pattern of compound 70 obtained as described in example 50e (treatment with n-propanol, Figure 9) derive from slight crystallographic inhomogenities. Those additional peaks surprisingly disappear after treatment of the inhomohenous material with 96% ethanol followed by evaporation (example 50i, Figure 13). Treatment of compound 70 obtained as described in example 50f (treatment with isopropanol) with 96% ethanol followed by evaporation gave once again Form A (example 50j, Figure 14).

The compounds of EMBODIMENT 2 may be prepared by methods known to the average skilled person, such as disclosed in WO 03/51888 and above in connection with EMBODIMENT 1.

An alternative to the reductive amination of the 6-keto compounds with amino acid tert-butyl esters (see EMBODIMENT 1) for the synthesis of the tert-butyl esters (precursors of compounds of EMBODIMENT 2), as defined in claim 31, is described here:

Azides (formula (II)) react with phosphorous compounds (formula (111)) in solvents as THF, diethyl ether, 1,4-dioxane, dimethoxyethane and DMF to form phosphorous imines of formula (IV) (Staudinger Reaction).

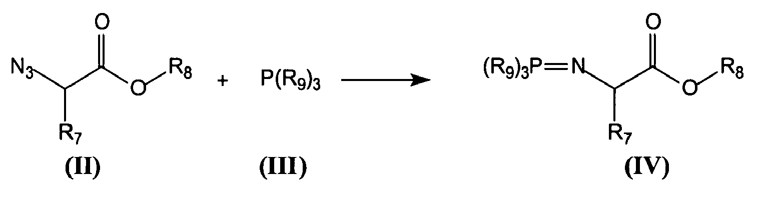

Reaction of phosphorous imines (formula (IV)) with 6-keto compounds of formula V (Aza-Wittig Reaction) forms imines (formula (VI)).

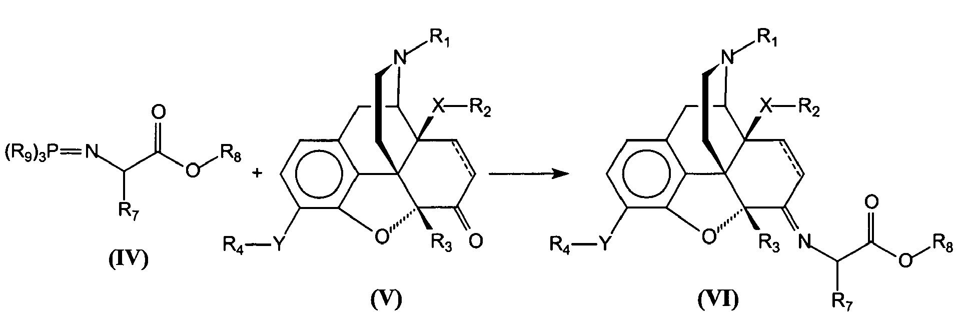

Imines of formula (VI) are reduced with hydrides as disclosed in WO 03/51888 and above in EMBODIMENT 1 to form the esters (formula (VII)) which are equal to the amino acid esters of formula (Ia).

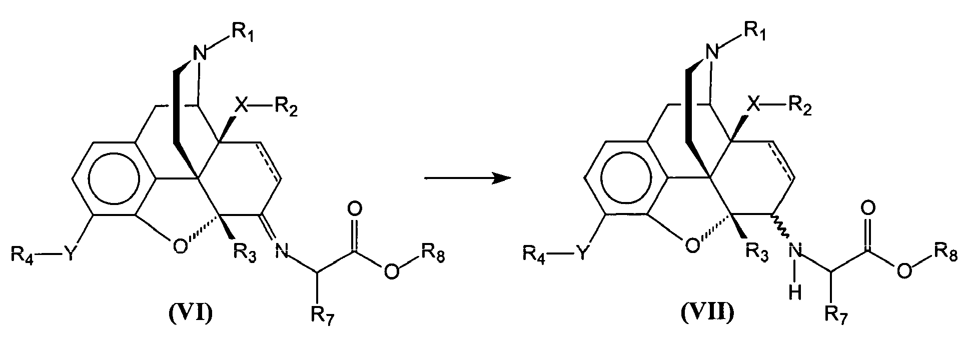

The following synthesis examples illustrate the invention as defined in EMBODIMENT 2:

### Example 48:

### 2-[(4,5α-Epoxy-3-hydroxy-14β-methoxy-17-methylmorphinan-6α-yl)amino]acetic acid Dihydrochloride (Compound 69)

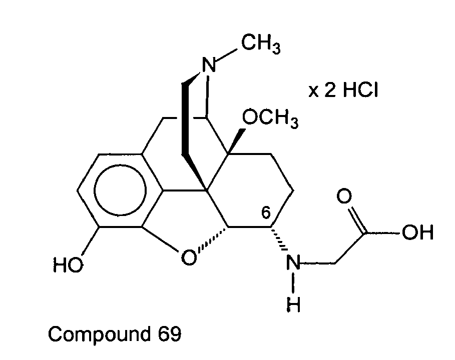

99% yield; ¹H-NMR (D₂O): 6.91 (d, J=8.2, 1 ar. H), 6.82 (d, J=8.2, 1 ar. H), 5.06 (d, J=3.0, H-C(5)), 3.92 (m, NHCH₂), 3.36 (s, MeO), 2.95 (s, MeN).

### Example 49a:

### 2-[(4,5α-Epoxy-3-hydroxy-14β-methoxy-17-methylmorphinan-6β-yl)amino]acetic acid Dihydrochloride Hydrate Ethanolate (Compound 70 × H2O × EtOH, Form A)

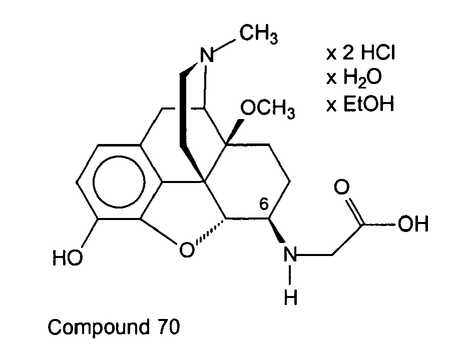

81% yield; ¹H-NMR (DMSO-d₆): 6.84 (d, J=8.2, 1 ar. H), 6.70 (d, J=8.2, 1 ar. H), 4.98 (d, J=7.2, H-C(5)), 4.05 (d, J=17.0, NHC*H*₂), 3.86 (d, J=17.0, NHC*H*₂), 3.44 (q, J=7.0, CH₃C*H*₂OH), 3.27 (s, MeO), 2.85 (s, MeN), 1.06 (t, J=7.0, C*H*₃CH₂OH). The powder X-ray diffraction pattern is shown in Figure 1. A single crystal was obtained by slow evaporation of a solution of compound 70 in 96% ethanol. The Ortep plot is shown in Figure 2.

### Example 49b:

### Alternative synthesis of 2-[(4,5α-Epoxy-3-hydroxy-14β-methoxy-17-methylmorphinan-6β-yl)amino]acetic acid Dihydrochloride Hydrate Ethanolate (Compound 70 × H2O × EtOH, Form A)

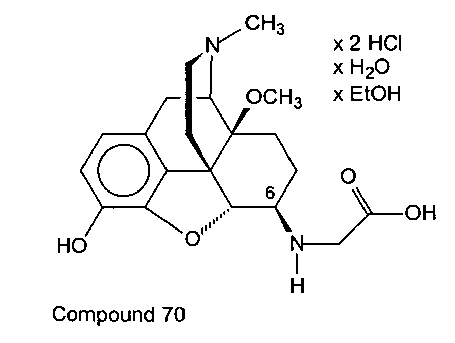

A mixture of 1 mmol 14-O-methyloxymorphone hydrochloride, 2 mmol glycine and 4 mL anhydrous methanol was stirred under inert conditions at room temperature. After addition of 1 mmol sodium cyanoborohydride, the mixture was stirred overnight at room temperature. After the end of the reaction, the mixture was filtered and the filtrate evaporated. The residue was precipitated from various mixtures of methanol, ethanol and propanol. The crude product was separated and purified by preparative HPLC (reversed phase chromatography; YMC Gel ODS AQ, 20 µm; 0.1 % formic acid/methanol). The obtained aqueous solution was evaporated to dryness, the residue treated with 1 M HCl and again evaporated to dryness. This procedure was repeated three times. The final residue was dissolved in ethanol and the product precipitated with tert-butyl methyl ether.

¹H-NMR (DMSO-d₆): 6.79 (d, J=8.0, 1 ar. H), 6.70 (d, J=8.0, 1 ar. H), 4.90 (d, J=7.2, H-C(5)), 4.09 (d, J=17.2, NHCH₂), 3.90 (d, J=17.2, NHCH₂), 3.44 (q, J=7.0, CH₃C*H*₂OH), 3.25 (s, MeO), 2.84 (d, J=3.8, MeN), 1.05 (t, J=7.0, C*H*₃CH₂OH). The powder X-ray diffraction pattern is shown in Figure 3.

### Example 50a:

Compound 70 Form A (80 mg material from example 49a) was heated to 170°C for 1 hour to obtain compound 70 Form B. The powder X-ray diffraction pattern is shown in Figure 5.

### Example 50b:

Compound 70 Form B (25 mg material from example 50a) was treated with 2 mL 96% ethanol and evaporated at room temperature to obtain compound 70 Form A. The powder X-ray diffraction pattern is shown in Figure 6.

### Example 50c:

Compound 70 Form A (50 mg material from example 49a) was treated with water and evaporated at room temperarture to obtain compound 70 Form C.

¹H-NMR (DMSO-d₆): 6.79 (d, J=8.0, 1 ar. H), 6.70 (d, J=8.0, 1 ar. H), 4.90 (d, J=7.0, H-C(5)), 4.06 (d, J=17.0, NHC*H₂*), 3.89 (d, J=17.0, NHC*H₂*)_{,} 3.25 (s, MeO), 2.84 (s, MeN). The powder X-ray diffraction pattern is shown in Figure 7.

### Example 50d:

Compound 70 Form A (80 mg material from example 49a) was treated with methanol and evaporated at room temperarture to obtain compound 70 Form D. The powder X-ray diffraction pattern is shown in Figure 8.

### Example 50e:

Compound 70 Form A (80 mg material from example 49a) was treated with n-propanol and evaporated at room temperarture to obtain compound 70 Form A. The powder X-ray diffraction pattern is shown in Figure 9.

### Example 50f:

Compound 70 Form A (80 mg material from example 49a) was treated with isopropanol and evaporated at room temperarture to obtain compound 70 Form A. The powder X-ray diffraction pattern is shown in Figure 10.

### Example 50g:

Compound 70 Form C (25 mg material from example 50c) was treated with ethanol and evaporated at room temperarture to obtain compound 70 Form A. The powder X-ray diffraction pattern is shown in Figure 11.

### Example 50h:

Compound 70 Form D (25 mg material from example 50d) was treated with ethanol and evaporated at room temperarture to obtain compound 70 Form A. The powder X-ray diffraction pattern is shown in Figure 12.

### Example 50i:

Compound 70 Form A (25 mg material from example 50e) was treated with ethanol and evaporated at room temperarture to obtain compound 70 Form A. The powder X-ray diffraction pattern is shown in Figure 13.

### Example 50j:

Compound 70 Form A (25 mg material from example 50e) was treated with ethanol and evaporated at room temperarture to obtain compound 70 Form A. The powder X-ray diffraction pattern is shown in Figure 14.

### Example 51:

### (2S)-2-[(4,5α-Epoxy-3-hydroxy-14β-methoxy-17-methylmorphinan-6α-yl)amino]propionic acid Dihydrochloride (Compound 71)

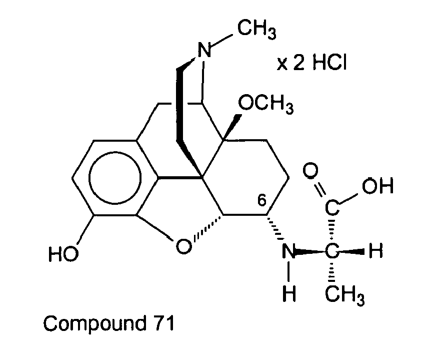

46% yield; ¹H-NMR (DMSO-d₆): 6.80 (d, J=8.1, 1 ar. H), 6.64 (d, J=8.1, 1 ar. H), 4.97 (d, J=3.2, H-C(5)), 3.31 (s, MeO), 2.87 (s, MeN), 1.52 (d, J=7.0, NHCHMe).

### Example 52:

### (2S)-2-[(4,5α-Epoxy-3-hydroxy-14β-methoxy-17-methylmorphinan-6β-yl)amino]propionic acid Dihydrochloride (Compound 72)

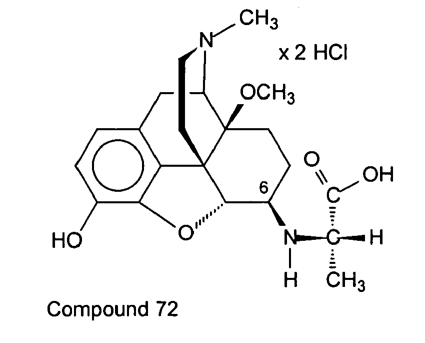

56% yield; ¹H-NMR (D₂O): 6.90 (d, J=8.2, 1 ar. H), 6.85 (d, J=8.2, 1 ar. H), 4.88 (d, J=7.2, H-C(5)), 3.33 (s, MeO), 2.93 (s, MeN), 1.56 (d, J=7.2, NHCHMe).

### Example 53:

### (2S)-2-[(4,5α-Epoxy-3-hydroxy-14β-methoxy-17-methylmorphinan-6α-yl)amino]-3-phenylpropionic acid Dihydrochloride (Compound 73)

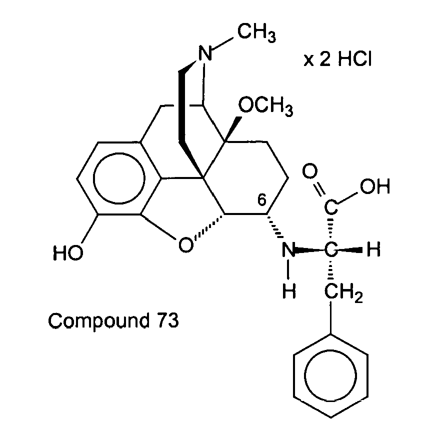

79% yield; ¹H-NMR (D₂O): 7.44-7.37 (m, 5 ar. H), 6.90 (d, J=8.4, 1 ar. H), 6.81 (d, J=8.4, 1 ar. H), 4.93 (dd, ³J=3.2, ⁴J=1.2, H-C(5)), 4.28 (t, J=7.0, NHCH), 3.29 (s, MeO), 2.93 (s, MeN).

### Example 54:

### (2S)-2-[(4,5α-Epoxy-3-hydroxy-14β-methoxy-17-methylmorphinan-6β-yl)amino]-3-phenylpropionic acid Dihydrochloride (Compound 74)

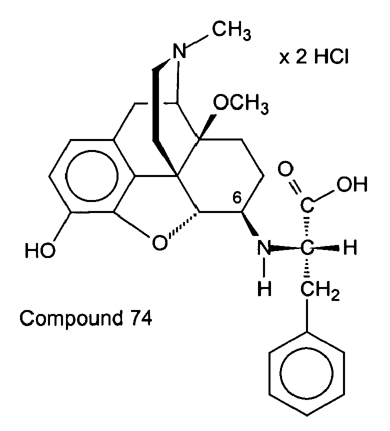

54% yield; ¹H-NMR (D₂O): 7.24 (s, 5 ar. H), 6.83 (d, J=8.1, 1 ar. H), 6.78 (d, J=8.1, 1 ar. H), 4.80 (d, J=7.5, H-C(5)), 4.37 (t, J=6.9, NHC*H*), 3.21 (s, MeO), 2.86 (s, MeN).

### Example 55:

### (2S)-2-[(4,5α-Epoxy-3-hydroxy-14β-methoxy-17-methylmorphinan-6α-yl)amino]-3-(4-hydroxyphenyl)propionic acid Dihydrochloride (Compound 75)

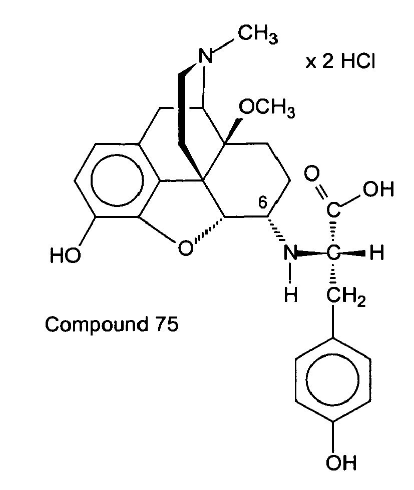

97% yield; ¹H-NMR (D₂O): 7.25 (d, J=8.4, 2 ar. H), 6.90 (d, J=8.4, 2 ar. H), 6.87 (d, J=8.5, 1 ar. H), 6.78 (d, J=8.5, 1 ar. H), 4.87 (d, J=2.4, H-C(5)), 4.21 (t, J=6.8, NHCH), 3.25 (s, MeO), 2.91 (s, MeN).

### Example 56:

### (2S)-2-[(4,5α-Epoxy-3-hydroxy-14β-methoxy-17-methylmorphinan-6β-yl)amino]-3-(4-hydroxyphenyl)propionic acid Dihydrochloride (Compound 76)

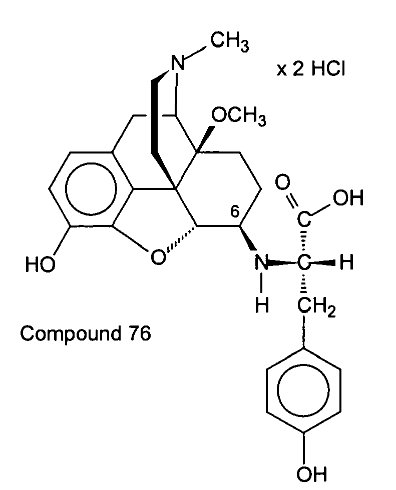

55% yield; ¹H-NMR (D₂O): 7.18 (d, J=8.4, 2 ar. H), 6.92 (d, J=8.5, 1 ar. H), 6.85 (d, J=8.5, 1 ar. H), 6.75 (d, J=8.4, 2 ar. H), 4.87 (d, J=7.8, H-C(5)), 4.36 (dd, J=5.4, J=5.8, NHCH), 3.28 (s, MeO), 2.90 (s, MeN).

### Example 57a:

### 2-[(4,5α-Epoxy-3-hydroxy-14β-methoxy-17-methylmorphinan-6α-yl)amino]acetic acid tert-butyl ester (Compound 77) and 2-[(4,5α-Epoxy-3-hydroxy-14β-methoxy-17-methylmorphinan-6β-yl)amino]acetic acid tert-butyl ester (Compound 78)

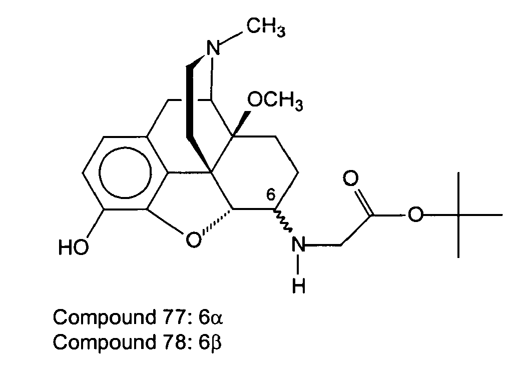

The reaction was carried out at room temperature under argon. 4.40 g Triphenylphosphine were disssolved in 25 mL anhydrous THF and slowly treated with a solution of 2.3 mL tert-butyl azidoacetate in 5 mL anhydrous THF whereby nitrogen was developed. The mixture was stirred for 1 hour and then treated with 3.01 g 14-O-methyloxymorphone hydrobromide and 1.5 mL triethylamine. After 23 hours the solvent was distilled off, the residue was dissolved in 30 mL anhydrous methanol and treated with 0.50 g NaCNBH₃. The mixture was stirred for 24 hours (the end of the reaction was monitored by TLC), then treated with 5 mL water and evaporated. The residue was treated with 200 mL water and 5 mL conc. NH₄OH solution and extracted with dichloromethane (1 × 100 mL, 3 × 50 mL). The combined organic phase was washed with brine (200 mL), dried over Na₂SO₄ and evaporated to give 7.88 g of an orange oil which was refluxed with 25 mL diethyl ether to form a white precipitate (triphenylphosphine oxide). The mixture was filtered, the filtrate was evaporated and the residue (4.42 g orange oil) separated and purified by high performance flash chromatography (silica gel; dichloromethane/methanol/conc. NH₄OH solution).

Compound 78: 1.09 g (33%); ¹H-NMR (CDCl₃): conforms to published data (WO 03/51888).

### Example 57b:

### 2-[(4,5α-Epoxy-3-hydroxy-14β-methoxy-17-methylmorphinan-6α-yl)amino]acetic acid tert-butyl ester (Compound 77) and 2-[(4,5α-Epoxy-3-hydroxy-14β-methoxy-17-methylmorphinan-6β-yl)amino]acetic acid tert-butyl ester (Compound 78)

The reaction was carried out under argon. 4.90 g Triphenylphosphine polymer bound (ca. 3 mmol/g) were treated with 100 mL anhydrous THF and allowed to swell for 16 hours. 4.2 mL tert-Butyl azidoacetate were dissolved in 20 mL anhydrous THF and added slowly to the mixture which was heated to 30°C whereby nitrogen was developed. The mixture was stirred for 4.5 hours, the resin filtered off and washed with anhydrous THF (20 mL) and anhydrous methanol (20 mL). The resin was transferred into a 3-necked round-bottomed flask, treated with 100 mL anhydrous THF, 1.50 g 14-O-methyloxymorphone hydrobromide and 0.6 mL triethylamine. The mixture was refluxed for ca. 2 hours and stored at room temperature overnight. The resin was filtered off, washed with 50 mL anhydrous methanol, and the filtrate was evaporated. The residue was dissolved in 35 mL anhydrous methanol and treated with 0.35 g NaCNBH₃. The mixture was stirred for 22 hours at room temperature (the end of the reaction was monitored by TLC), then treated with 5 mL water and evaporated. The residue was treated with 150 mL water and extracted with dichloromethane (1 × 100 mL, 3 × 50 mL). The combined organic phase was washed with brine (200 mL), dried over Na₂SO₄ and evaporated to give 1.35 g of an orange oil which was separated and purified by high performance flash chromatography (silica gel; dichloromethane/methanol/conc. NH₄OH solution).

Compound 78: 0.61 g (37%); ¹H-NMR (CDCl₃): conforms to published data (WO 03/51888).

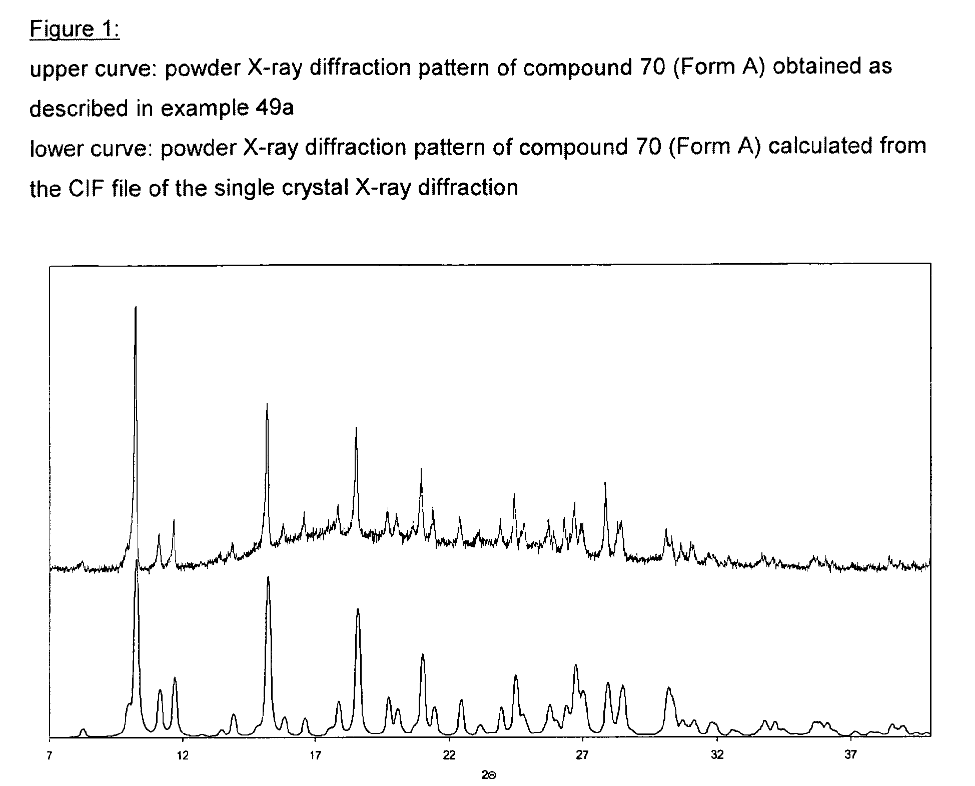

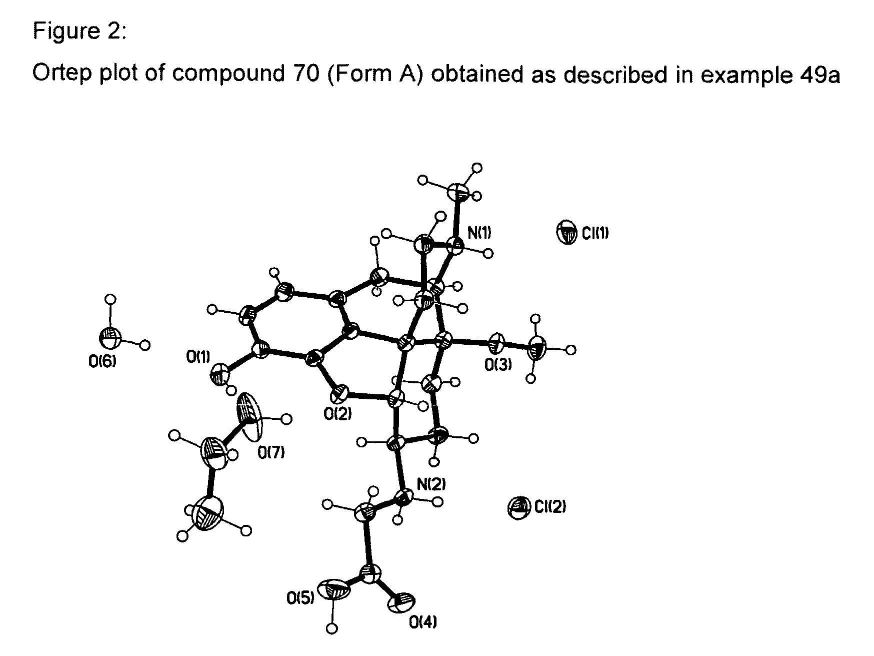

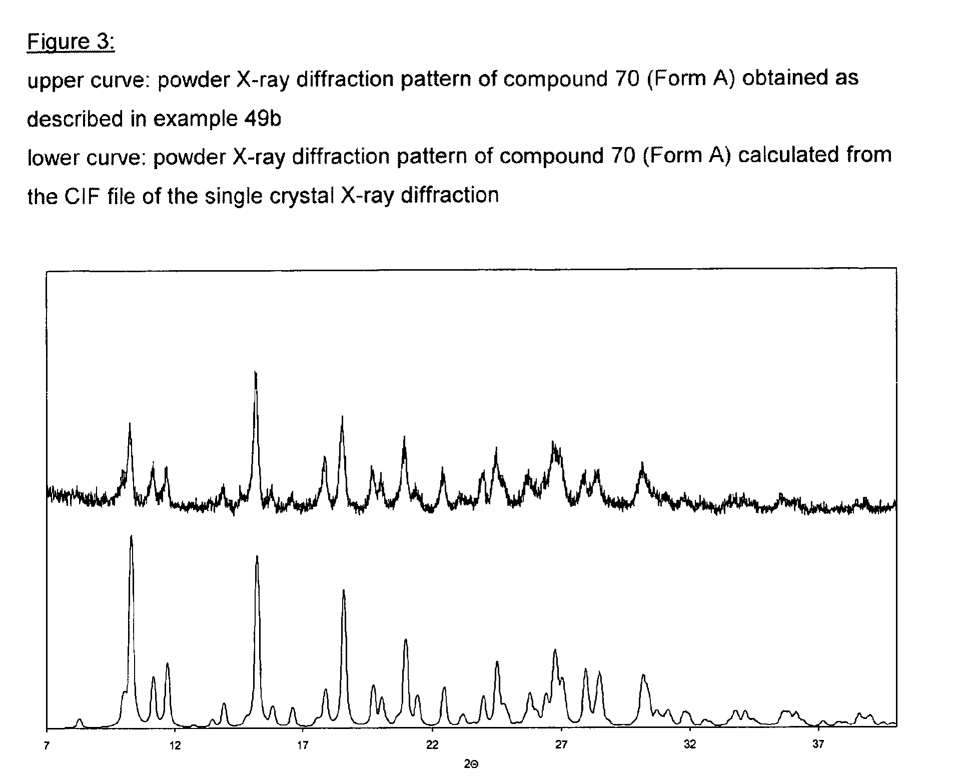

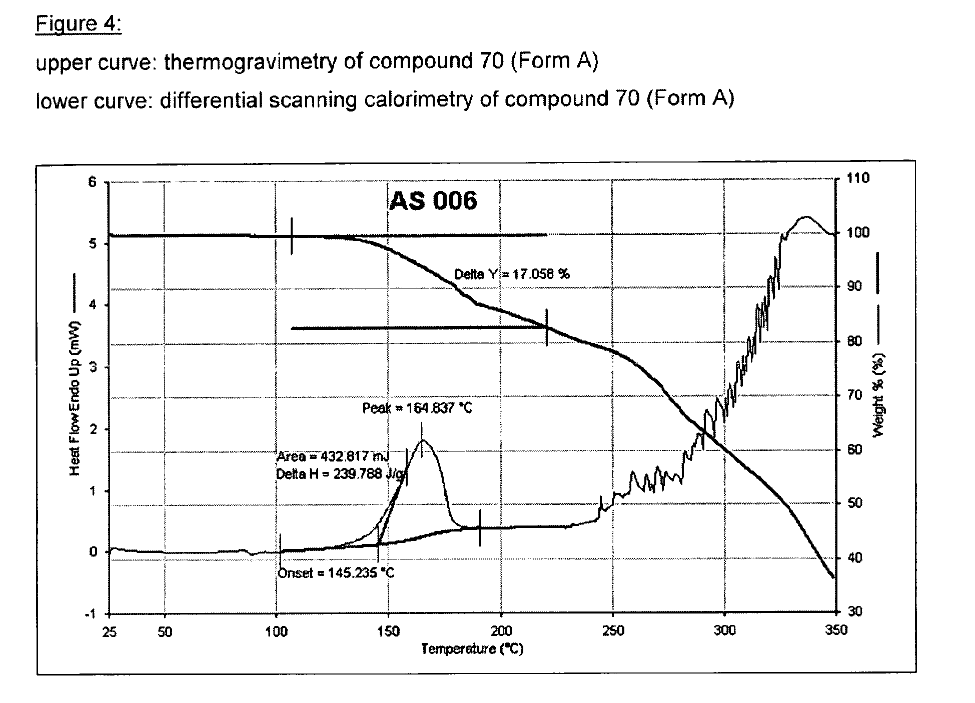

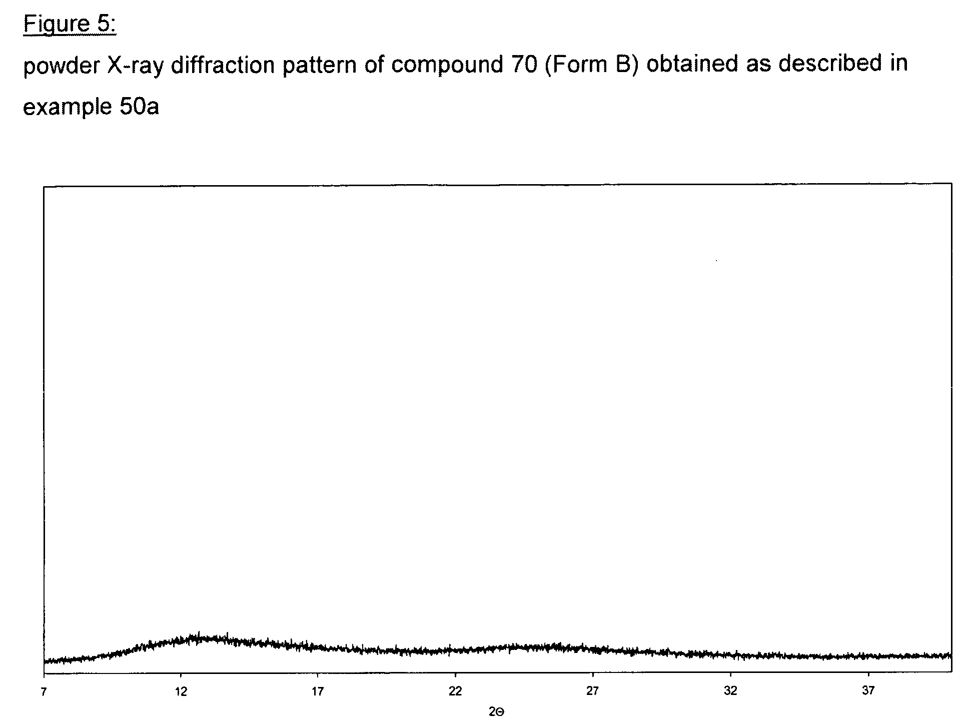

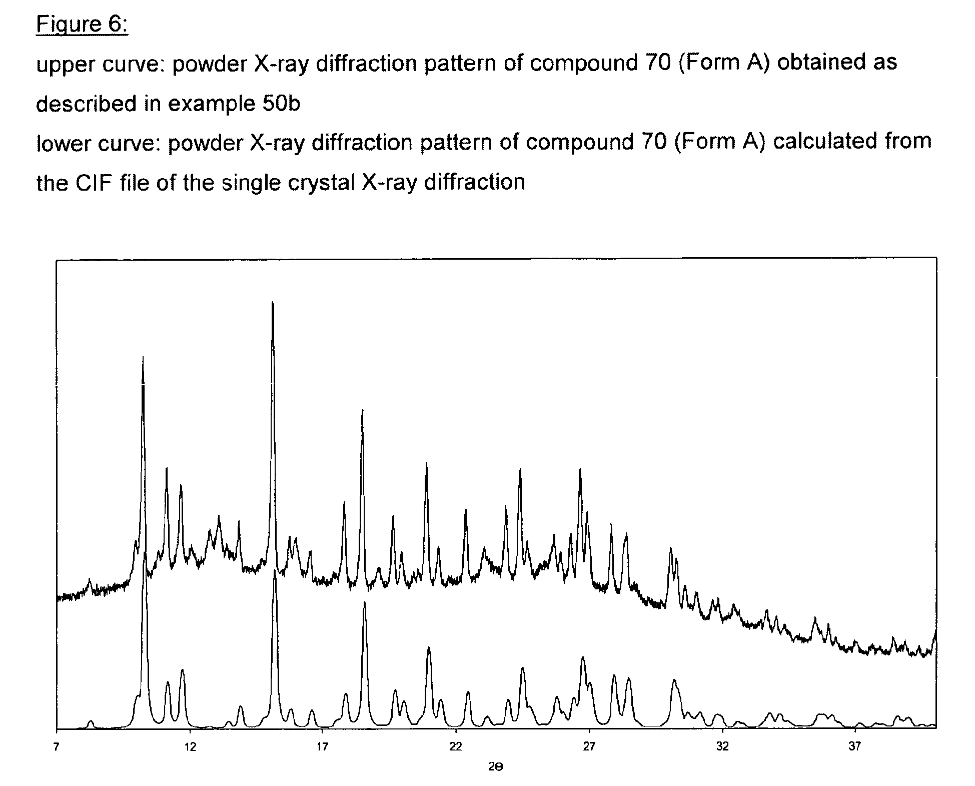

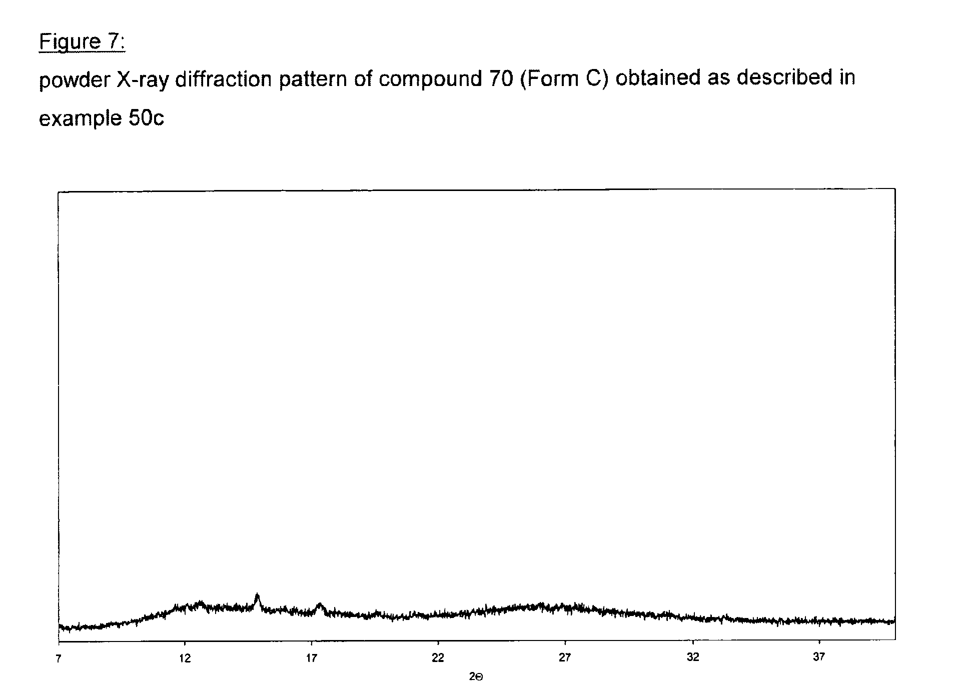

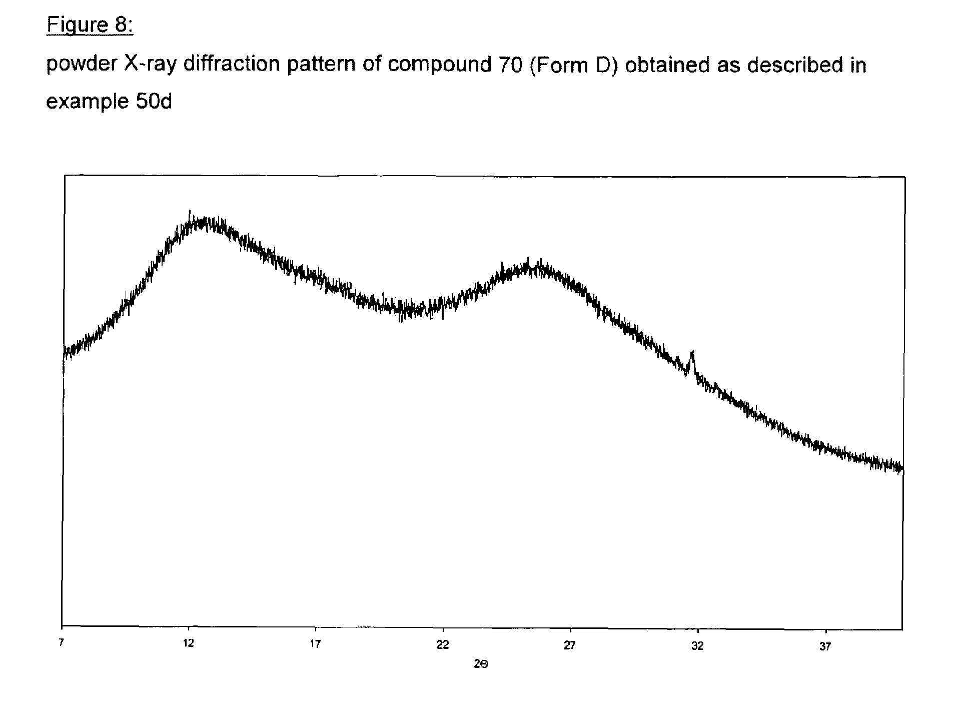

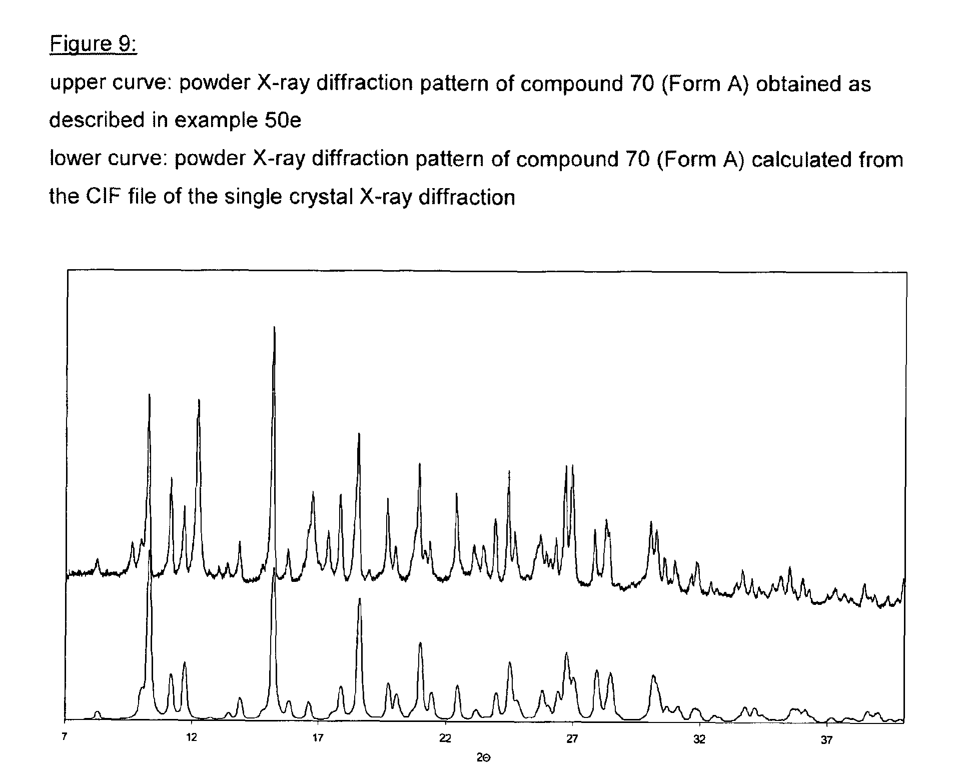

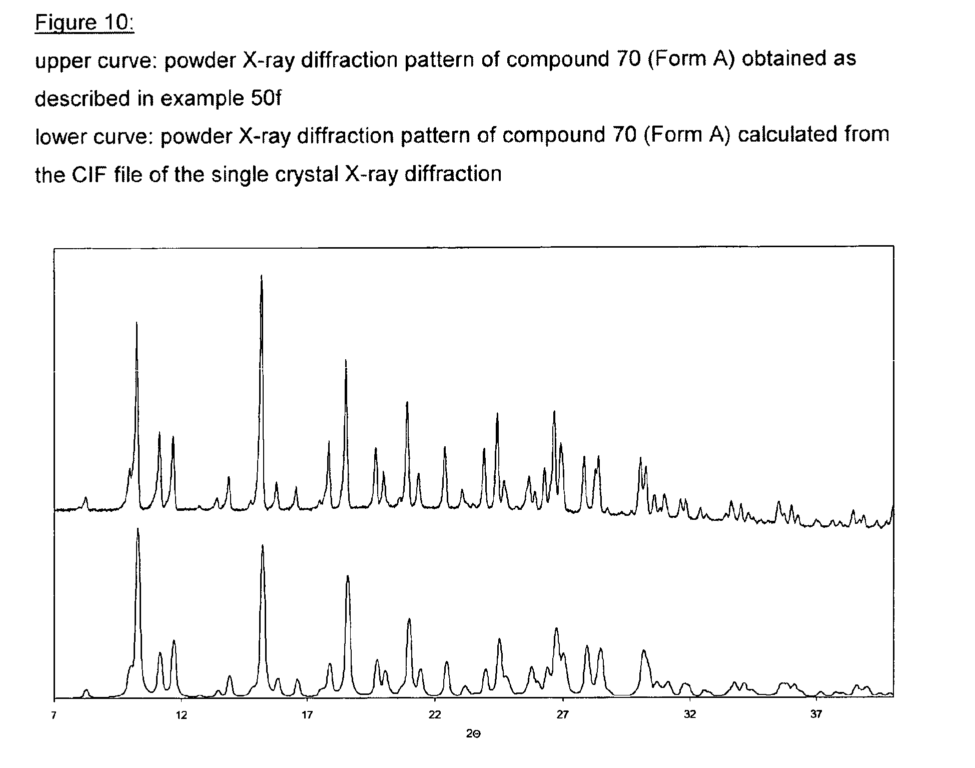

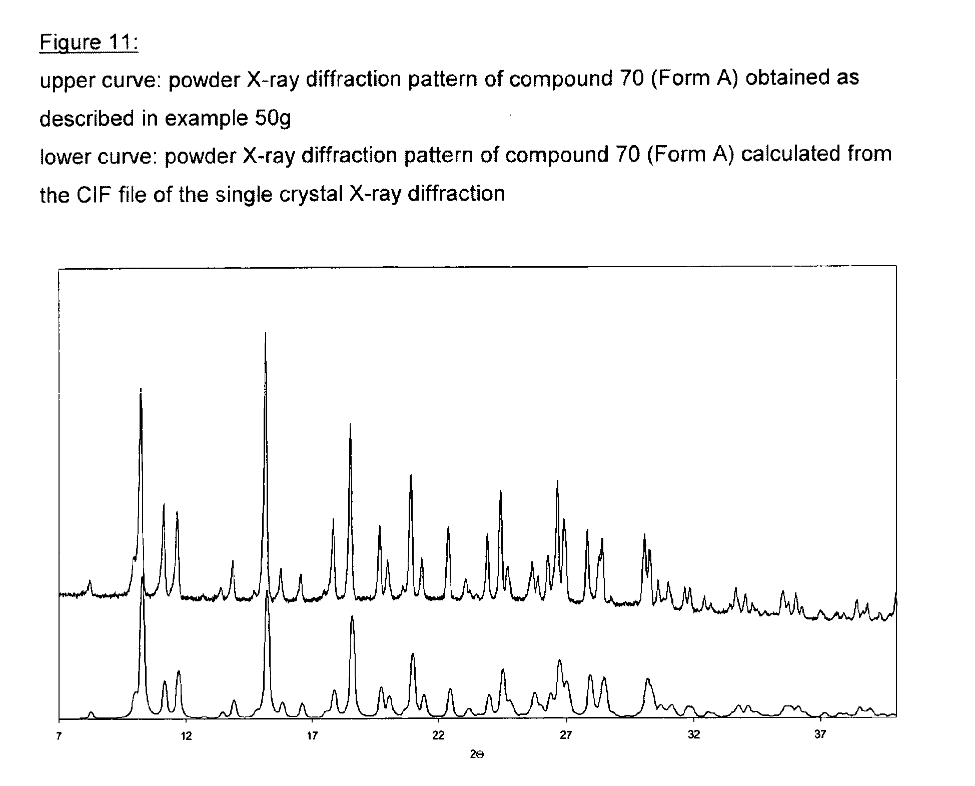

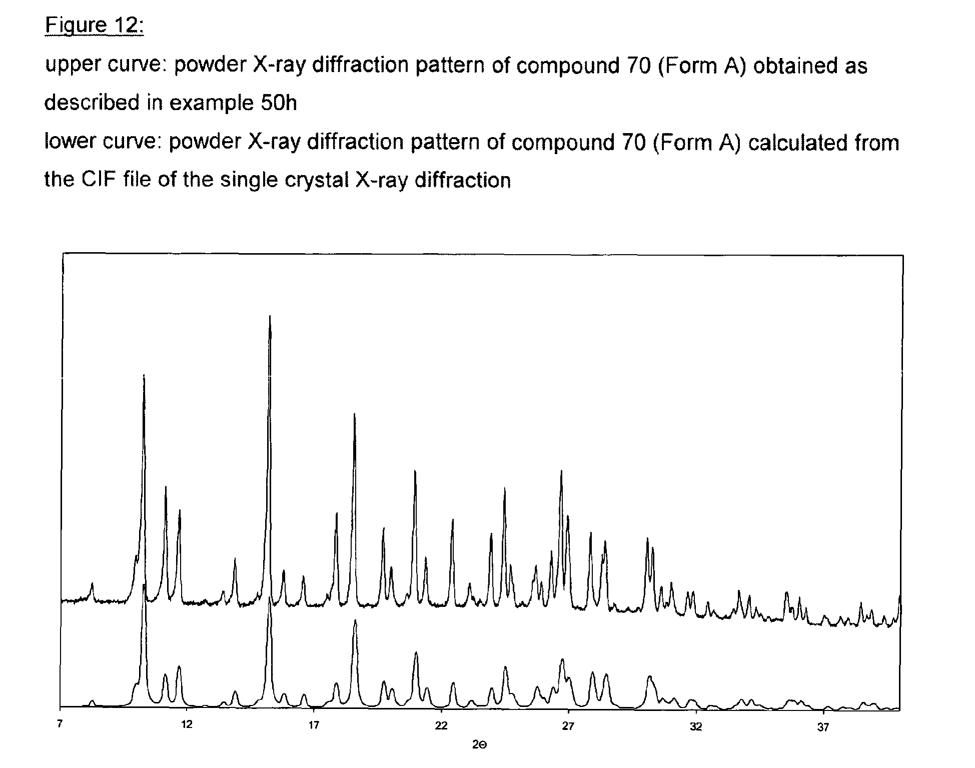

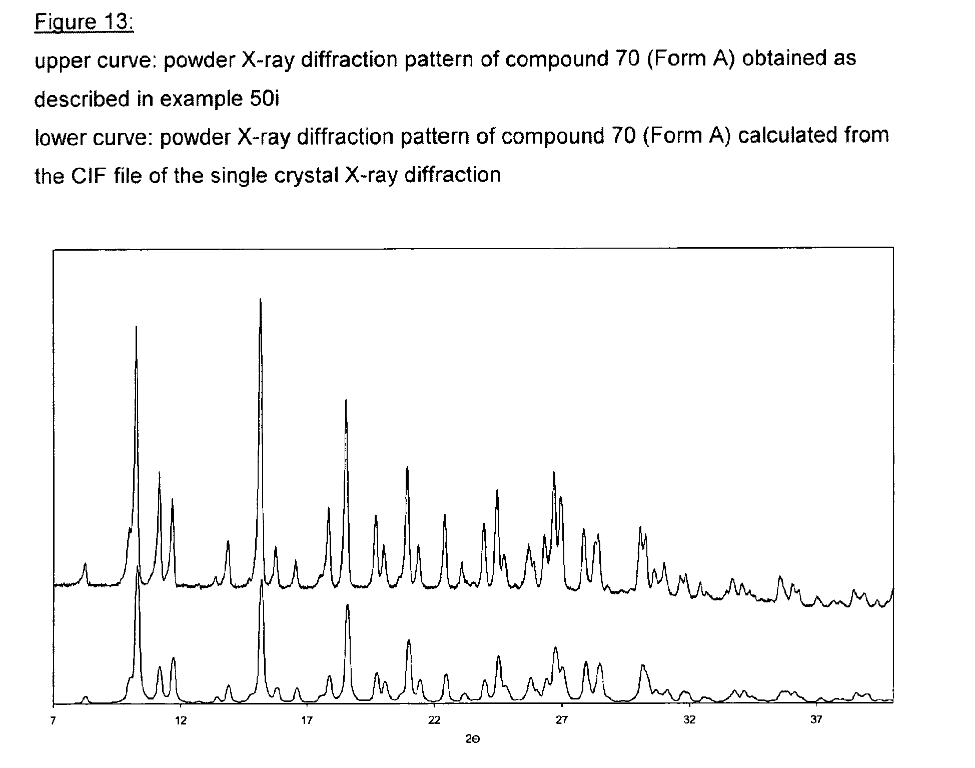

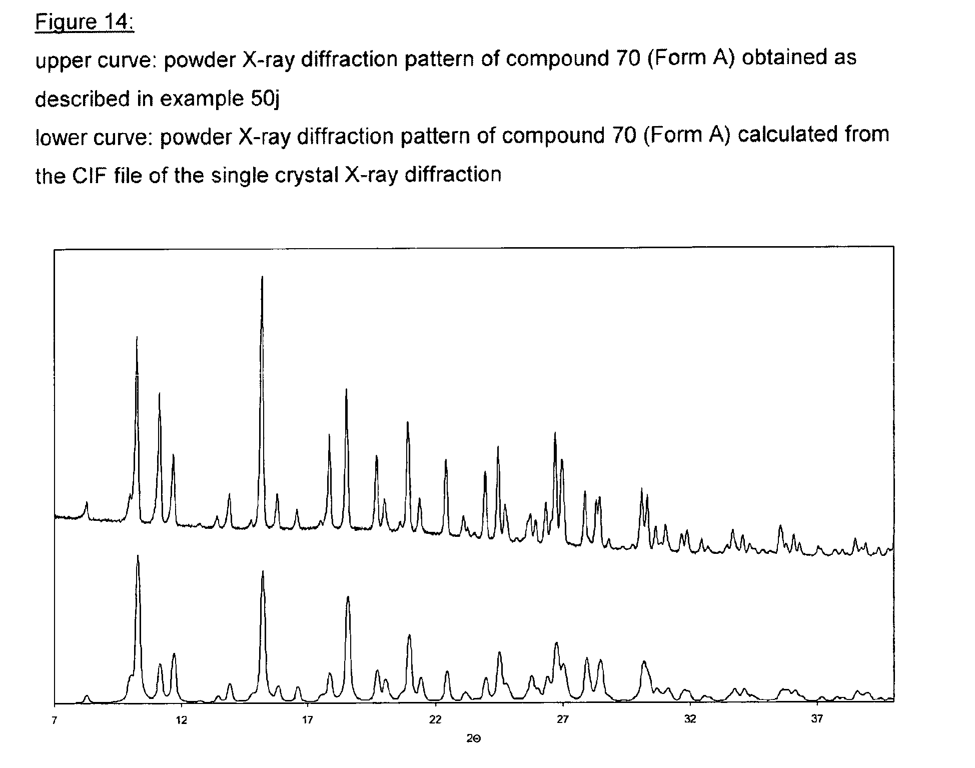

### EMBODIMENT 3

Compound of formula (VIII), 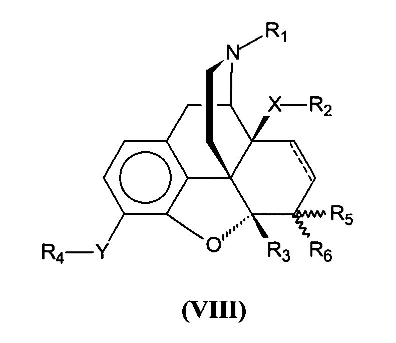 in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆C₁₀-aryl and alkinoyl preferably is C₃-C₆-alkinoyl; iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C₅-C₃₀, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl ; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-a!kyny!- and N,N'-dialkynylformamidinyl; C4-C30, preferably C₄-C₁₆-N-cycloalkylalkyl-and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably C₉-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1*H*-imidazol-2-yl, 1,4,5,6-tetrahydropyrimidin-2-yl, 4,5,6,7-tetrahydro-1H-[1,3]diazepin-2-yl;
and residues selected from the following group (VIIIa): (C₁-C₃₀-alkyl)CO₂B, werein alkyl is preferably C1-C12, more preferably C1-C6 alkyl; (C₂-C₃₀-alkenyl)CO₂B werein alkenyl is preferably C2-C12, more preferably C2-C6 alkenyl; (C₂-C₃₀-alkynyl)CO₂B werein alkynyl is preferably C2-C12, more preferably C2-C6 alkynyl; (C₄-C₃₀-cycloalkylalkyl)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CO₂B, where aryl is C₆-C₁₀-aryl and alkyl is C₁-C₂₄-alkyl, preferably C1-C12, more preferably C1-C6 alkyl; (C₈-C₃₀-arylalkenyl)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl)CO₂B, where aryl is C₆-C₁₀-aryl and alkynyl is C₂-C₂₄-alkynyl, preferably C2-C12, more preferably C2-C6 alkynyl;
[(C₁-C₃₀-alkyl)CO₂B]CO₂B, werein alkyl is preferably C1-C12, more preferably C1-C6 alkyl; [(C₂-C₃₀-alkenyl)CO₂B]CO₂B, werein alkenyl is preferably C2-C12, more preferably C2-C6 alkenyl; [(C₂-C₃₀-alkynyl)CO₂B]CO₂B, werein alkynyl is preferably C2-C12, more preferably C2-C6 alkynyl; [(C₄-C₃₀-cycloalkylalkyl)CO₂B]CO₂B, where cycloalkyl is C₃-C₁₀-cycloalkyl and alkyl is C₁-C₂₇-alkyl, preferably C1-C12, more preferably C1-C6 alkyl; [(C₅-C₃₀-cycloalkylalkenyl)CO₂B]CO₂B, where cycloalkyl is C₃-C₁₀-cycloalkyl and alkenyl is C₂-C₂₇-alkenyl preferably C2-C12, more preferably C2-C6 alkenyl; [(C₅-C₃₀-cycloalkylalkynyl)CO₂B]CO₂B, where cycloalkyl is C₃-C₁₀-cycloalkyl and alkynyl is C₂-C₂₇-alkynyl, preferably C2-C12, more preferably C2-C6 alkynyl; [(C₇-C₃₀-arylalkyl)CO₂B]CO₂B, where aryl is C₆-C₁₀-aryl and alkyl is C₁-C₂₄-alkyl, preferably C1-C12, more preferably C1-C6 alkyl; [(C₈-C₃₀-arylalkenyl)CO₂B]CO₂B, where aryl is C₆-C₁₀-aryl and alkenyl is C₂-C₂₄-alkenyl, preferably C2-C12, more preferably C2-C6 alkenyl; [(C₈-C₃₀-arylalkynyl)CO₂B]CO₂B, where aryl is C₆-C₁₀-aryl and alkynyl is C₂-C₂₄-alkynyl, preferably C2-C12, more preferably C2-C6 alkynyl; [(C₁-C₃₀-alkyl)CONH₂]CO₂B, werein alkyl is preferably C1-C12, more preferably C1-C6 alkyl; [(C₂-C₃₀-alkenyl)CONH₂]CO₂B, werein alkenyl is preferably C2-C12, more preferably C2-C6 alkenyl; [(C₂-C₃₀-alkynyl)CONH₂]CO₂B, werein alkynyl is preferably C2-C12, more preferably C2-C6 alkynyl; [(C₄-C₃₀-cycloalkylalkyl)CONH₂]CO₂B, where cycloalkyl is C₃-C₁₀-cycloalkyl and alkyl is C₁-C₂₇-alkyl, preferably C1-C12, more preferably C1-C6 alkyl; [(C₅-C₃₀-cycloalkylalkenyl)CONH₂]CO₂B, where cycloalkyl is C₃-C₁₀-cycloalkyl and alkenyl is C₂-C₂₇-alkenyl, preferably C2-C12, more preferably C2-C6 alkenyl; [(C₅-C₃₀-cycloalkylalkynyl)CONH₂]CO₂B, where cycloalkyl is C₃-C₁₀-cycloalkyl and alkynyl is C₂-C₂₇-alkynyl, preferably C2-C12, more preferably C2-C6 alkynyl; [(C₇-C₃₀-arylalkyl)CONH₂]CO₂B, where aryl is C₆-C₁₀-aryl and alkyl is C₁-C₂₄-alkyl, preferably C1-C12, more preferably C1-C6 alkyl; [(C₈-C₃₀-arylalkenyl)CONH₂]CO₂B, where aryl is C₆-C₁₀-aryl and alkenyl is C₂-C₂₄-alkenyl, preferably C2-C12, more preferably C2-C6 alkenyl; [(C₈-C₃₀-arylalkynyl)CONH₂]CO₂B, where aryl is C₆-C₁₀-aryl and alkynyl is C₂-C₂₄-alkynyl, preferably C2-C12, more preferably C2-C6 alkynyl; (C₁-C₃₀-alkyl-S-A")CO₂B, werein alkyl is preferably C1-C12, more preferably C1-C6 alkyl; (C₂-C₃₀-alkenyl-S-A")CO₂B, werein alkenyl is preferably C2-C12, more preferably C2-C6 alkenyl; (C₂-C₃₀-alkynyl-S-A")CO₂B, werein alkynyl is preferably C2-C12, more preferably C2-C6 alkynyl; (C₄-C₃₀-cycloalkylalkyl-S-A")CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl-S-A")CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl-S-A")CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl-S-A")CO₂B, where aryl is C₆-C₁₀-aryl and alkyl is C₁-C₂₄-alkyl, preferably C1-C12, more preferably C1-C6 alkyl; (C₈-C₃₀-arylalkenyl-S-A")CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl-S-A")CO₂B, where aryl is C₆-C₁₀-aryl and alkynyl is C₂-C₂₄-alkynyl, preferably C2-C12, more preferably C2-C6 alkynyl; wherein B is as defined above and A" is H; C₁-C₃₀-alkyl, preferably C1-C12, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cycloalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C₁-C₁₂, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl; n=1-30(CHDCONH)ₙCHDCO₂B, where D is H; C₁-C₃₀-alkyl, preferably C₁-C₁₂, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cycloalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C1-C12, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl; (C₁-C₃₀-alkyl)CO₂B, preferably C1-C12, more preferably C1-C6 alkyl; (C₂-C₃₀-alkenyl)CO₂B, preferably C2-C12, more preferably C2-C6 alkenyl; (C₂-C₃₀-alkynyl)CO₂B, preferably C2-C12, more preferably C2-C6 alkynyl; (C₄-C₃₀-cycloalkylalkyl)CO₂B, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CO₂B, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CO₂B, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CO₂B, where aryl is C₆-C₁₀-aryl and alkyl is C₁-C₂₄-alkyl, preferably C1-C12, more preferably C1-C6 alkyl; (C₈-C₃₀-arylalkenyl)CO₂B, where aryl is C₆-C₁₀-aryl and alkenyl is C₂-C₂₄-alkenyl, preferably C2-C12, more preferably C2-C6 alkenyl; (C₈-C₃₀-arylalkynyl)CO₂B, where aryl is C₆-C₁₀-aryl and alkynyl is C₂-C₂₄-alkynyl, preferably C2-C12, more preferably C2-C6 alkynyl;
(C₁-C₃₀-alkyl)CONH₂, preferably C1-C12, more preferably C1-C6 alkyl; (C₂-C₃₀-alkenyl)CONH₂ , preferably C2-C12, more preferably C2-C6 alkenyl; (C₂-C₃₀-alkynyl)CONH₂, preferably C2-C12, more preferably C2-C6 alkynyl; (C₄-C₃₀-cycloalkylalkyl)CONH₂, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)CONH₂, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)CONH₂, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)CONH₂, where aryl is C₆-C₁₀-aryl and alkyl is C₁-C₂₄-alkyl, preferably C1-C12, more preferably C1-C6 alkyl; (C₈-C₃₀-arylalkenyl)CONH₂, where aryl is C₆-C₁₀-aryl and alkenyl is C₂-C₂₄-alkenyl, preferably C2-C12, more preferably C2-C6 alkenyl; (C₈-C₃₀-arylalkynyl)CONH₂, where aryl is C₆-C₁₀-aryl and alkynyl is C₂-C₂₄-alkynyl, preferably C2-C12, more preferably C2-C6 alkynyl; where B is as defined above; (C₁-C₃₀-alkyl)SO₃A", preferably C1-C12, more preferably C1-C6 alkyl; (C₁-C₃₀-alkenyl)SO₃A", preferably C2-C12, more preferably C2-C6 alkenyl; (C₁-C₃₀-alkynyl)SO₃A", preferably C2-C12, more preferably C2-C6 alkynyl; (C₄-C₃₀-cycloalkylalkyl)SO₃A", where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)SO₃A", where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)SO₃A", where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)SO₃A", where aryl is C₆-C₁₀-aryl and alkyl is C₁-C₂₄-alkyl, preferably C1-C12, more preferably C1-C6 alkyl; (C₈-C₃₀-arylalkenyl)SO₃A", where aryl is C₆-C₁₀-aryl and alkenyl is C₂-C₂₄-alkenyl, preferably C2-C12, more preferably C2-C6 alkenyl; (C₈-C₃₀-arylalkynyl)SO₃A", where aryl is C₆-C₁₀-aryl and alkynyl is C₂-C₂₄-alkynyl, preferably C2-C12, more preferably C2-C6 alkynyl; (C₁-C₃₀-alkyl)PO(OA")₂, preferably C1-C12, more preferably C1-C6 alkyl; (C₂-C₃₀-alkenyl)PO(OA")₂ , preferably C2-C12, more preferably C2-C6 alkenyl; (C₂-C₃₀-alkynyl)PO(OA")₂ , preferably C2-C12, more preferably C2-C6 alkynyl; (C₄-C₃₀-cycloalkylalkyl)PO(OA")₂, where cycloalkyl is C₃-C₁₀-cycloalkyl and alkyl is C₁-C₂₇-alkyl, preferably C1-C12, more preferably C1-C6 alkyl; (C₅-C₃₀-cycloalkylalkenyl)PO(OA")₂, where cycloalkyl is C₃-C₁₀-cycloalkyl and alkenyl is C₂-C₂₇-alkenyl, preferably C2-C12, more preferably C2-C6 alkenyl; (C₅-C₃₀-cycloalkylalkynyl)PO(OA")₂, where cycloalkyl is C₃-C₁₀-cycloalkyl and alkynyl is C₂-C₂₇-alkynyl, preferably C2-C12, more preferably C2-C6 alkynyl; (C₇-C₃₀-arylalkyl)PO(OA")₂, where aryl is C₆-C₁₀-aryl and alkyl is C₁-C₂₄-alkyl, preferably C1-C12, more preferably C1-C6 alkyl; (C₈-C₃₀-arylalkenyl)PO(OA")₂, where aryl is C₆-C₁₀-aryl and alkenyl is C₂-C₂₄-alkenyl, preferably C2-C12, more preferably C2-C6 alkenyl; (C₈-C₃₀-arylalkynyl)PO(OA")₂, where aryl is C₆-C₁₀-aryl and alkynyl is C₂-C₂₄-alkynyl, preferably C2-C12, more preferably C2-C6 alkynyl; wherein A" is H; C₁-C₃₀-alkyl, preferably C1-C12, more preferably C1-C6 alkyl; C₂-C₃₀-alkenyl, preferably C2-C12, more preferably C2-C6 alkenyl; C₂-C₃₀-alkynyl, preferably C2-C12, more preferably C2-C6 alkynyl; C₄-C₃₀-cycloalkylalkyl, where cycloalkyl is C₃-C₁₀-cycloalkyl and alkyl is C₁-C₂₇-alkyl, preferably C1-C12, more preferably C1-C6 alkyl; C₅-C₃₀-cycloalkylalkenyl, where cycloalkyl is C₃-C₁₀-cycloalkyl and alkenyl is C₂-C₂₇-alkenyl, preferably C2-C12, more preferably C2-C6 alkenyl; C₅-C₃₀-cycloalkylalkynyl, where cycloalkyl is C₃-C₁₀-cycloalkyl and alkynyl is C₂-C₂₇-alkynyl, preferably C2-C12, more preferably C2-C6 alkynyl; C₇-C₃₀-arylalkyl, where aryl is C₆-C₁₀-aryl and alkyl is C₁-C₂₄-alkyl, preferably C1-C12, more preferably C1-C6 alkyl; C₈-C₃₀-arylalkenyl, where aryl is C₆-C₁₀-aryl and alkenyl is C₂-C₂₄-alkenyl, preferably C2-C12, more preferably C2-C6 alkenyl; C₈-C₃₀-arylalkynyl, where aryl is C₆-C₁₀-aryl and alkynyl is C₂-C₂₄-alkynyl, preferably C2-C12, more preferably C2-C6 alkynyl;
wherein at least one of R₅ and R₆ is selected from group (VIIIa);
and pharmaceutically acceptable acid addition salts as well as base addition salts and easily accessible derivatives (e.g. esters or amides of amino acid derivatives). Some compounds of EMBODIMENT 3 may exist in different stereochemical configuration and/or may show more than one crystalline structure, in particular the compounds possessing one or more chiral carbon atom. The present invention comprises all those specific embodiments, such as diastereomer, enantiomers, polymorphs etc, in any given or desired mixture or in isolated form.

The various terms as employed above do have the following meaning and preferred embodiments are as follows:

The dotted line between the carbon atoms 7 and 8 of the morphinan skeleton designates that these carbon atoms may be unsaturated (double bond between C7 and C8) or saturated (single bond between C7 and C8).

In this invention the terms alkyl, alkenyl and alkynyl include both branched and also unbranched alkyl, alkenyl and alkynyl groups as well as mono-, di- and trihydroxy-substituted b ranched a nd unbranched alkyl, alkenyl a nd alkynyl groups. These groups furthermore may be substituted once twice or three times with substituents selected independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents a re cyclic groups, including carbocycles and heterocycles which may be saturated unsaturated or aromatic. Preferred examples comprise from 3 to 8 ring atoms, selected from C, N, O, and S. Aryl can be unsubstituted or mono-, di- or tri-substituted, whereby the substituents can be chosen independently from hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino, C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio. Further suitable substituents are cyclic groups, including carbocycles and heterocycles which may be saturated unsaturated or aromatic. Preferred examples comprise from 3 to 8 ring atoms, selected from C, N, O, and S. The term aryl defines aromatic rings comprising preferably from 5 to 14 ring atoms and the term aryl comprises furthermore carbocyclic aryl groups as well as heterocyclic aryl groups, comprising preferably from 1 to 3 heteroatoms selected from N, O and S. The aryl goups as defined above may furthermore be fused ring systems such as naphthyl or anthracenyl or the corresponding heterocyclic groups comprising from 1 to 3 heteroatoms selected from N, O, and S. The definitions listed above for alkyl, alkenyl, alkynyl and aryl are valid for all substituents of this application.

The compounds of this invention contain pharmaceutically and pharmacologically acceptable salts of the compounds of formula (I). According to this invention both inorganic and also organic salts are suitable. Examples of suitable inorganic salts for this invention are hydrochlorides, hydrobromides, hydroiodides, sulphates, phosphates and tetrafluoroborates. Possible organic salts are, for example, acetates, tartrates, lactates, benzoates, stearates, pamoates, methane sulphonates, salicylates, fumarates, maleinates, succinates, aspartates, citrates, oxalates, trifluoroacetates and orotates.

Acid addition salts are preferred as conventional pharmaceutically acceptable addition salts, particularly preferred are the hydrochlorides, hydrobromides, tetrafluoroborates and trifluoroacetates. X and Y are preferably oxygen. Preferably R₁ is alkyl as defined above, in particular methyl or ethyl, whereby methyl is preferred, or cycloalkylalkyl, preferably cyclopropylmethyl. R₂ is preferably not H and also not a group which forms an ester unit with X. The other definitions for R₂ as defined in claim 18 are, in contrast, preferred, whereby especially alkyl as defined above is preferred, particularly preferred are methyl, ethyl and propyl, where necessary substituted, e.g. with a phenyl group, for example to produce a 3-phenylpropyl group (i.e., put differently, an arylalkyl group is also preferred for R₂, in particular 3-phenylpropyl). R₁ and R₂ are especially preferably both simultaneously alkyl, in particular either both simultaneously methyl or methyl (R₁) and ethyl (R₂). A further preferred combination of R₁ and R₂ is cycloalkylalkyl, in particular cyclopropylmethyl for R₁ and arylalkyl, preferably phenylpropyl for R₂. R₃ and R₄ are in each case preferably hydrogen or alkyl, whereby methyl is especially preferred as an alkyl group. R₄ is in addition preferred as C(N-Boc)(NH-Boc). R₅ and R₆ are preferably chosen such that one is H and the other is different to H, whereby this radical, different to H, is preferably not halogenated.

In a specially preferred representation X and Y are oxygen. Then preferably, R₁ is methyl and cyclopropylmethyl and R₂ is alkyl and arylalkyl, in particular methyl and 3-phenylpropyl, and R₃, R₄ and R₆ are hydrogen.

Preferred compounds of the present invention are further the base addition salts, comprising metal salts, such as lithium salts, sodium salts, potassium salts, beryllium salts, magnesium salts, calcium salts, strontium salts, aluminum salts and zinc salts; ammonium salts, such as C₁-C₃₀ monoalkylammonium salts, C₁-C₃₀ dialkylammonium salts, C₁-C₃₀ trialkylammonium salts, C₁-C₃₀ tetraalkylammonium salts; C₂-C₃₀ monoalkenylammonium salts, C₂-C₃₀ dialkenylammonium salts, C₂-C₃₀ trialkenylammonium salts, C₂-C₃₀ tetraalkenylammonium salts; C₂-C₃₀ monoalkynylammonium salts, C₂-C₃₀ dialkynylammonium salts, C₂-C₃₀ trialkynylammonium salts, C₂-C₃₀ tetraalkynylammonium salts; C₄-C₃₀ mono(cycloalkylalkylammonium) salts, C₄-C₃₀ di(cycloalkylalkylammonium) salts, C₄-C₃₀ tri(cycloalkylalkylammonium) salts, C₄-C₃₀ tetra(cycloalkylalkylammonium) salts, where cycloalkyl is C₃-C₁₀-cycloalkyl and alkyl is C₁-C₂₇-alkyl; C₅-C₃₀ mono(cycloalkylalkenylammonium) salts, C₅-C₃₀ di(cycloalkylalkenylammonium) salts, C₅-C₃₀ tri(cycloalkylalkenylammonium) salts, C₅-C₃₀ tetra(cycloalkylalkenylammonium) salts, where cycloalkyl is C₃-C₁₀-cycloalkyl and alkenyl is C₂-C₂₇-alkenyl; C₅-C₃₀ mono(cycloalkylalkynylammonium) salts, C₅-C₃₀ di(cycloalkylalkynylammonium) salts, C₅-C₃₀ tri(cycloalkylalkynylammonium) salts, C₅-C₃₀ tetra(cycloalkylalkynylammonium) salts, where cycloalkyl is C₃-C₁₀-cycloalkyl and alkynyl is C₂-C₂₇-alkynyl; C₇-C₃₀ mono(arylalkylammonium) salts, C₇-C₃₀ di(arylalkylammonium) salts, C₇-C₃₀ tri(arylalkylammonium) salts, C₇-C₃₀ tetra(arylalkylammonium) salts, where aryl is C₆-C₁₀-aryl and alkyl is C₁-C₂₄-alkyl; C₈-C₃₀ mono(arylalkenylammonium) salts, C₈-C₃₀ di(arylalkenylammonium) salts, C₈-C₃₀ tri(arylalkenylammonium) salts, C₈-C₃₀ tetra(arylalkenylammonium) salts, where aryl is C₆-C₁₀-aryl and alkenyl is C₂-C₂₄-alkenyl; C₈-C₃₀ mono(arylalkynylammonium) salts, C₈-C₃₀ di(arylalkynylammonium) salts, C₈-C₃₀ tri(arylalkynylammonium) salts, C₈-C₃₀ tetra(arylalkynylammonium) salts, where aryl is C₆-C₁₀-aryl and alkynyl is C₂-C₂₄-alkynyl, combinations of the ammonium salts listed above, and salts derived from heterocyclic bases, in particular heterocyclic nitrogen bases. These include salts derived from heterocyclic compounds comprising the following cycles: pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, pyrrolidine, imidazolidine, pyrazolidine, oxazolidine, isoxazolidine, thiazolidine, isothiazolidine, thiadiazolidine, sulpholane, imidazolidine, pyridine, pyridazine, pyrazine, pyrimidine, piperazine, piperidine, morpholine, tetrazole, triazole, triazolidine, tetrazolidine, azepine, homopiperazine and azetidine.

The compounds according to EMBODIMENT 3 may be synthesized according to procedures usua in the art ans as derivable from the experimental part of WO 03/51888, under due consideration of the synthetic methods disclosed by Parra et al., in Eur.J.Org.Chem. 2003, 1386-1388.

It has now been found that the compounds of the pertinent invention represent effective opioid receptor ligands of the type 6-aminomorphinan and exhibit a high therapeutic application potential as analgesics, as immunomodulators with immunostimulating or immunosuppressive effect, as cancer therapeutics, inflammation inhibitors, as anti-rheumatics, diuretics, anorectics, as an agent against diarrhoea, anaesthetics or as neuroprotective active substances.

The compounds quoted in the claims are therefore potentially applicable to the treatment of pain, functional intestinal diseases, such as abdominal pain, intestinal obstruction (ileus) or obstipation, for the treatment of mammals, in particular humans, for the treatment of Raynaud's disease, for the treatment of complaints caused by vasoconstriction, for the treatment of dysmenorrhoea, angina pectoris, myocardial infarct, emphysema, bronchial spasms, chronic obstructive bronchitis, rheumatic complaints, nephrosis, nephritis in conjunction with rheumatic diseases, for the treatment of tumours, phaeochromocytoma, Addison's disease, hepatic cirrhosis, chronic inflammation of the small and large intestines (e.g. irritable colon syndrome - colon irritabile, colitis ulcerosa, morbus Crohn), addiction withdrawal of, for example, opiates, cocaine or alcohol, or for the treatment of psychic diseases such as dysphoria or schizophrenia.

The compounds of this invention are suitable for application in the production of a medicament for the treatment of pain, including acute and chronic pain, on the locomotor system such as pain in the neck, back, hip, knee, shoulder or myofacial pain, treatment of complex regional pain syndromes, phantom pain, facial neuralgia, rheumatalgia, cancer pain, pain from burns, pain after accidents, pain due to chronic inflammation, visceralgia, headaches such as for example tension headaches, cervically related headache or migraine, pain after central lesions such as for example with paraplegia or thalamic lesions, neuralgic pain such as zoster neuralgia, postzoster neuralgia, ischaemic pain such as angina pectoris or peripheral occlusive arterial disease, postoperative pain, neuropathic pain such as pain with diabetic neuropathy, pain after virus infections or pain after nerve lesions.

The pharmaceutical compositions according to the invention, which contain a compound of this invention and / or a pharmaceutically acceptable salt of it as active ingredient together with a pharmaceutically acceptable carrier substance, are suitable for the treatment of the conditions quoted in the description.

The application according to the invention includes application as analgesic, immunomodulating, antitumour, antiproliferative, anti-inflammatory, antirheumatic, diuretic, anorectic, antidiarrhoeal, anaesthetic, neuroprotective active substance and as active substance for the prevention and treatment of intestinal obstruction (ileus).

Preferred applications take place for the production of a medicament for the treatment of pain, functional intestinal diseases, of the Raynaud's disease, for the treatment of complaints caused by vasoconstriction, angina pectoris, myocardial infarct, emphysema, bronchial spasms, chronic obstructive bronchitis, rheumatic complaints (including rheumatoid arthritis, arthrosis, osteoarthritis, spondylosis, lumbago, lupus erythematosus, spondyarthropathy), nephrosis, nephritis in conjunction with rheumatic diseases, for the treatment of tumours, cancer, phaeochromocytoma, Addison's disease, hepatic cirrhosis, chronic inflammation of the small and large intestines (e.g. irritable colon syndrome - colon irritabile, colitis ulcerosa, morbus Crohn), for the treatment of drug abuse, psychic diseases, erectile dysfunction and / or for the suppression of rejection of transplants after transplantation on mammals, particularly on humans.

Surprisingly it was also found that the compounds of this invention were not capable of overcoming the blood-brain barrier or only to a slight extent, and therefore a special significance could be attributed to them with regard to their application as peripherally effective therapeutics, for example as medicaments for the treatment of pain, rheumatic therapy, suppression of organ rejection after transplantations on mammals, particularly humans and also for the treatment of erectile disturbances. The limited access to the central nervous system is accompanied by a much reduced rate of side effects relating to central side effects such for example nausea, vomiting, sedation, dizziness, confusion, respiratory depression and mania.

In addition, it was surprisingly found that the compounds of this invention have a very iong analgesically effective period. This enables a lower dosage and less frequent administration of the medicament, which results in a lower rate of side effects and toxicity as well as a higher readiness of patients to take the medicament.

## Claims

1. Compounds of formula (I), 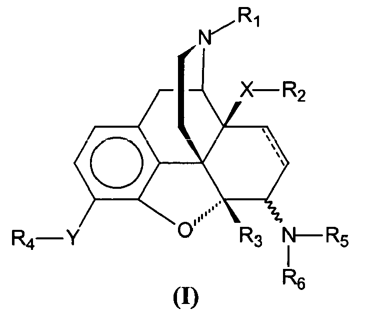 in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N-oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cydoalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl; iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably C₉-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1*H*-imidazol-2-yl, 1,4,5,6-tetrahydropyrimidin-2-yl, 4,5,6,7-tetrahydro-1 H-[1,3]diazepin-2-yl;
and a group selected from an acid group or a derivative thereof bearing residues and moieties forming, together with the nitrogen atom to which they are bound, a residue corresponding to an amino acid, an amino acid derivative and/or a dimer or oligomer thereof and/or a peptide comprising up to 30 amino acid units, wherein at least one of R₅ and R₆ is selected from such a group;
X is oxygen, sulphur or methylene or the group (X-R₂) is H and
Y is oxygen or the group (Y-R₄) is H;
and pharmaceutically acceptable acid addition salts as well as base addition salts and easily accessible derivatives (e.g. esters or amides of amino acid derivatives), as well as polymorphic forms thereof.

2. Compounds according to claim 1, wherein the at least one group selected from an acid group or a derivative thereof bearing residue and moieties forming, together with the nitrogen atom to which they are bound, a residue corresponding to an amino acid, an amino acid derivative and/or a dimer or oligomer thereof and/or a peptide comprising up to 30 amino acid units for R₅ and R₆, which can be the same or different, is selected from
(C₁-C₃₀-alkyl)CO₂B; (C₂-C₃₀-alkenyl)CO₂B; (C₂-C₃₀-alkynyl)CO₂B; (C₄-C₃₀-cycloalkylalkyl)CO₂B, where cycloalkyl is C₃-C₁₀-cycloalkyl and alkyl is C₁-C₂₇-alkyl; (C₅-C₃₀-cycloalkylalkenyl)CO₂B, where cycloalkyl is C₃-C₁₀-cycloalkyl and alkenyl is C₂-C₂₇-alkenyl; (C₅-C₃₀-cycloalkylalkynyl)CO₂B, where cycloalkyl is C₃-C₁₀-cycloalkyl and alkynyl is C₂-C₂₇-alkynyl; (C₇-C₃₀-arylalkyl)CO₂B, where aryl is C₆-C₁₀-aryl and alkyl is C₁-C₂₄-alkyl; (C₈-C₃₀-arylalkenyl)CO₂B, where aryl is C₆-C₁₀-aryl and alkenyl is C₂-C₂₄-alkenyl; (C₈-C₃₀-arylalkynyl)CO₂B, where aryl is C₆-C₁₀-aryl and alkynyl is C₂-C₂₄-alkynyl;
(cyclic C₃-C₁₀-alkyl)CO₂B; (cyclic C₃-C₁₀-alkenyl)CO₂B; (cyclic C₃-C₁₀-alkynyl)CO₂B; (bicyclic C₆-C₂₀-alkyl)CO₂B; (bicyclic C₆-C₂₀-alkenyl)CO₂B; (bicyclic C₆-C₂₀-alkynyl)CO₂B; (cyclic C₃-C₁₀-alkyl fused with C₆-C₁₄ aromatic ring system)CO₂B; (cyclic C₃-C₁₀-alkenyl fused with C₆-C₁₄ aromatic ring system)CO₂B; (cyclic C₃-C₁₄-alkynyl fused with C₆-C₁₀ aromatic ring system)CO₂B;
[(C₁-C₃₀-alkyl)CO₂B]CO₂B; [(C₂-C₃₀-alkenyl)CO₂B]CO₂B; [(C₂-C₃₀-alkynyl)CO₂B]CO₂B; [(C₄-C₃₀-cycloalkylalkyl)CO₂B]CO₂B, where cycloalkyl is C₃-C₁₀-cycloalkyl and alkyl is C₁-C₂₇-alkyl; [(C₅-C₃₀-cycloalkylalkenyl)CO₂B]CO₂B, where cycloalkyl is C₃-C₁₀-cycloalkyl and alkenyl is C₂-C₂₇-alkenyl; [(C₅-C₃₀-cycloalkylalkynyl)CO₂B]CO₂B, where cycloalkyl is C₃-C₁₀-cycloalkyl and alkynyl is C₂-C₂₇-alkynyl; [(C₇-C₃₀-arylalkyl)CO₂B]CO₂B, where aryl is C₆-C₁₀-aryl and alkyl is C₁-C₂₄-alkyl; [(C₈-C₃₀-arylalkenyl)CO₂B]CO₂B, where aryl is C₆-C₁₀-aryl and alkenyl is C₂-C₂₄-alkenyl; [(C₈-C₃₀-arylalkynyl)CO₂B]CO₂B, where aryl is C₆-C₁₀-aryl and alkynyl is C₂-C₂₄-alkynyl;
[(C₁-C₃₀-alkyl)CONH₂]CO₂B; [(C₂-C₃₀-alkenyl)CONH₂]CO₂B; [(C₂-C₃₀-alkynyl)CONH_{2]}CO₂B; [(C₄-C₃₀-cycloalkylalkyl)CONH₂]CO₂B, where cycloalkyl is C₃-C₁₀-cycloalkyl and alkyl is C₁-C₂₇-alkyl; [(C₅-C₃₀-cycloalkylalkenyl)CONH₂]CO₂B, where cycloalkyl is C₃-C₁₀-cycloalkyl and alkenyl is C₂-C₂₇-alkenyl; [(C₅-C₃₀-cycloalkylalkynyl)CONH_{2]}CO₂B, where cycloalkyl is C₃-C₁₀-cycloalkyl and alkynyl is C₂-C₂₇-alkynyl; [(C₇-C₃₀-arylalkyl)CONH₂]CO₂B, where aryl is C₆-C₁₀-aryl and alkyl is C₁-C₂₄-alkyl; [(C₈-C₃₀-arylalkenyl)CONH₂]CO₂B, where aryl is C₆-C₁₀-aryl and alkenyl is C₂-C₂₄-alkenyl; [(C₈-C₃₀-arylalkynyl)CONH₂]CO₂B, where aryl is C₆-C₁₀-aryl and alkynyl is C₂-C₂₄-alkynyl;
(C₁-C₃₀-alkyl-S-A)CO₂B; (C₂-C₃₀-alkenyl-S-a)CO₂B; (C₂-C₃₀-alkynyl-S-A)CO₂B; (C₄-C₃₀-cycloalkylalkyl-S-A)CO₂B, where cycloalkyl is C₃-C₁₀-cycloalkyl and alkyl is C₁-C₂₇-alkyl; (C₅-C₃₀-cycloalkylalkenyl-S-A)CO₂B, where cycloalkyl is C₃-C₁₀-cycloalkyl and alkenyl is C₂-C₂₇-alkenyl; (C₅-C₃₀-cycloalkylalkynyl-S-A)CO₂B, where cycloalkyl is C₃-C₁₀-cycloalkyl and alkynyl is C₂-C₂₇-alkynyl; (C₇-C₃₀-arylalkyl-S-A)CO₂B, where aryl is C₆-C₁₀-aryl and alkyl is C₁-C₂₄-alkyl; (C₈-C₃₀-arylalkenyl-S-A)CO₂B, where aryl is C₆-C₁₀-aryl and alkenyl is C₂-C₂₄-alkenyl; (C₈-C₃₀-arylalkynyl-S-A)CO₂B, where aryl is C₆-C₁₀-aryl and alkynyl is C₂-C₂₄-alkynyl; A is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃₀-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl; or A is selected from (C₁-C₃₀-alkyl-S-a)CO₂B; (C₂-C₃₀-alkenyl-S-a)CO₂B; (C₂-C₃₀-alkynyl-S-a)CO₂B; (C₄-C₃₀-cycloalkylalkyl-S-a)CO₂B, where cycloalkyl is C₃-C₁₀-cycloalkyl and alkyl is C₁-C₂₇-alkyl; (C₅-C₃₀-cycloalkylalkenyl-S-a)CO₂B, where cycloalkyl is C₃-C₁₀-cycloalkyl and alkenyl is C₂-C₂₇-alkenyl; (C₅-C₃₀-cycloalkylalkynyl-S-a)CO₂B, where cycloalkyl is C₃-C₁₀-cycloalkyl and alkynyl is C₂-C₂₇-alkynyl; (C₇-C₃₀-arylalkyl-S-a)CO₂B, where aryl is C₆-C₁₀-aryl and alkyl is C₁-C₂₄-alkyl; (C₈-C₃₀-arylalkenyl-S-a)CO₂B, where aryl is C₆-C₁₀-aryl and alkenyl is C₂-C₂₄-alkenyl; (C₈-C₃₀-arylalkynyl-S-a)CO₂B, where aryl is C₆-C₁₀-aryl and alkynyl is C₂-C₂₄-alkynyl; wherein a designates the connecting bond;
(CHDCONH)ₙCHDCO₂B, where n is from 1 to 30, where D is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃₀-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl;
(C₁-C₃₀-alkyl)CO₂B; (C₂-C₃₀-alkenyl)CO₂B; (C₂-C₃₀-alkynyl)CO₂B; (C₄-C₃₀-cycloalkylalkyl)CO₂B, where cycloalkyl is C₃-C₁₀-cycloalkyl and alkyl is C₁-C₂₇-alkyl; (C₅-C₃₀-cycloalkylalkenyl)CO₂B, where cycloalkyl is C₃-C₁₀-cycloalkyl and alkenyl is C₂-C₂₇-alkenyl; (C₅-C₃₀-cycloalkylalkynyl)CO₂B, where cycloalkyl is C₃-C₁₀-cycloalkyl and alkynyl is C₂-C₂₇-alkynyl; (C₇-C₃₀-arylalkyl)CO₂B, where aryl is C₆-C₁₀-aryl and alkyl is C₁-C₂₄-alkyl; (C₈-C₃₀-arylalkenyl)CO₂B, where aryl is C₆-C₁₀-aryl and alkenyl is C₂-C₂₄-alkenyl; (C₈-C₃₀-arylalkynyl)CO₂B, where aryl is C₆-C₁₀-aryl and alkynyl is C₂-C₂₄-alkynyl;
(C₁-C₃₀-alkyl)CONH₂; (C₂-C₃₀-alkenyl)CONH₂; (C₂-C₃₀-alkynyl)CONH₂; (C₄-C₃₀-cycloalkylalkyl)CONH₂, where cycloalkyl is C₃-C₁₀-cycloalkyl and alkyl is C₁-C₂₇-alkyl; (C₅-C₃₀-cycloalkylalkenyl)CONH₂, where cycloalkyl is C₃-C₁₀-cycloalkyl and alkenyl is C₂-C₂₇-alkenyl; (C₅-C₃₀-cycloalkylalkynyl)CONH₂, where cycloalkyl is C₃-C₁₀-cycloalkyl and alkynyl is C₂-C₂₇-alkynyl; (C₇-C₃₀-arylalkyl)CONH₂, where aryl is C₆-C₁₀-aryl and alkyl is C₁-C₂₄-alkyl; (C₈-C₃₀-arylalkenyl)CONH₂, where aryl is C₆-C₁₀-aryl and alkenyl is C₂-C₂₄-alkenyl; (C₈-C₃₀-arylalkynyl)CONH₂, where aryl is C₆-C₁₀-aryl and alkynyl is C₂-C₂₄-alkynyl;
wherein B is as defined in claim 1;
(C₁-C₃₀-alkyl)SO₃A#; (C₂-C₃₀-alkenyl)SO₃A#; (C₂-C₃₀-alkynyl)SO₃A#; (C₄-C₃₀-cycloalkylalkyl)SO₃A#, where cycloalkyl is C₃-C₁₀-cycloalkyl and alkyl is C₁-C₂₇-alkyl; (C₅-C₃₀-cycloalkylalkenyl)SO₃A#, where cycloalkyl is C₃-C₁₀-cycloalkyl and alkenyl is C₂-C₂₇-alkenyl; (C₅-C₃₀-cycloalkylalkynyl)SO₃A#, where cycloalkyl is C₃-C₁₀-cycloalkyl and alkynyl is C₂-C₂₇-alkynyl; (C₇-C₃₀-arylalkyl)SO₃A#, where aryl is C₆-C₁₀-aryl and alkyl is C₁-C₂₄-alkyl; (C₈-C₃₀-arylalkenyl)SO₃A#, where aryl is C₆-C₁₀-aryl and alkenyl is C₂-C₂₄-alkenyl; (C₈-C₃₀-arylalkynyl)SO₃A#, where aryl is C₆-C₁₀-aryl and alkynyl is C₂-C₂₄-alkynyl; (C₁-C₃₀-alkyl)PO(OA#)₂; (C₂-C₃₀-alkenyl)PO(OA#)₂; (C₂-C₃₀-alkynyl)PO(OA#)₂; (C₄-C₃₀-cycloalkylalkyl)PO(OA#)₂, where cycloalkyl is C₃-C₁₀-cycloalkyl and alkyl is C₁-C₂₇-alkyl; (C₅-C₃₀-cycloalkylalkenyl)PO(OA#)₂, where cycloalkyl is C₃-C₁₀-cycloalkyl and alkenyl is C₂-C₂₇-alkenyl; (C₅-C₃₀-cycloalkylalkynyl)PO(OA#)₂, where cycloalkyl is C₃-C₁₀-cycloalkyl and alkynyl is C₂-C₂₇-alkynyl; (C₇-C₃₀-arylalkyl)PO(OA#)₂, where aryl is C₆-C₁₀-aryl and alkyl is C₁-C₂₄-alkyl; (C₈-C₃₀-arylalkenyl)PO(OA#)₂, where aryl is C₆-C₁₀-aryl and alkenyl is C₂-C₂₄-alkenyl; (C₈-C₃₀-arylalkynyl)PO(OA#)₂, where aryl is C₆-C₁₀-aryl and alkynyl is C₂-C₂₄-alkynyl; A# is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃ₒ-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl.

3. Compound according to claim 1 or 2, wherein one of R₅ and R₆ is hydrogen and the other one is selected among an acid group or a derivative thereof bearing residue and moieties forming, together with the nitrogen atom to which they are bound, a residue corresponding to an amino acid, an amino acid derivative and/or a dimer or oligomer thereof and/or a peptide comprising up to 30 amino acid units.

4. Compounds of formula (Ia), 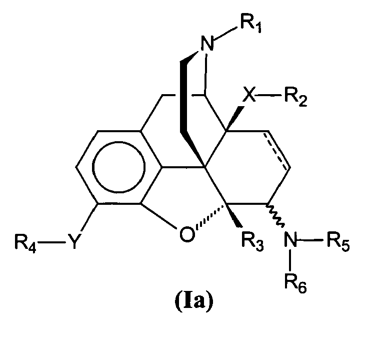 in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cydoalkyl and alkyl preferably is C₁-C₆-alkyl; C5-C30, preferably C₅-C₁₆-cycloalkylalkenyl, where cycloalkyl preferably is C₃-C₁₀-cydoalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl; iminomethyl, formamidinyl, C1-C30, preferably C₁-C₁₂, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl-and N,N'-dicycloalkylalkylformamidinyl, where cycloalkyl preferably is C₃-C₁₀-cydoalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cydoalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cydoalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably C₉-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1*H*-imidazol-2-yl, 1,4,5,6-tetrahydropyrimidin-2-yl, 4,5,6,7-tetrahydro-1 H-[1,3]diazepin-2-yl;
X is oxygen, sulphur or methylene or the group (X-R₂) is H and
Y is oxygen or the group (Y-R₄) is H;
and pharmaceutically acceptable acid addition salts as well as base addition salts and easily accessible derivatives (e.g. esters or amides of amino acid derivatives), as well as polymorphic forms thereof.

5. Compounds of claim 4 in which R₁ is C₁-C₆-alkyl; R₂ is C₁-C₆-alkyl or C₇-C₁₆-arylalkyl, where aryl is C₆-C₁₀-aryl and alkyl is C₁-C₆-alkyl; R₃, R₄ and R₆ are hydrogen; R₅ is CH(A)CO₂B where A is hydrogen; hydroxyl; C₁-C₆-alkyl; C₇-C₁₆-arylalkyl, where aryl is C₆-C₁₀-aryl and alkyl is C₁-C₆-alkyl; amino; or guanidino; B is hydrogen or C₁-C₆-alkyl; R₅ can furthermore be formamidinyl; C₂-C₇-(alkyloxycarbonyl)formamidinyl; C₃-C₈-(alkenyloxycarbonyl)formamidinyl; C₃-C₈-(alkynyloxycarbonyl)formamidinyl; C₈-C₁₇-(arylalkyloxycarbonyl)formamidinyl, where aryl is C₆-C₁₀-aryl and alkyloxy is C₁-C₆-alkyloxy; C₉-C₁₇-(arylalkenyloxycarbonyl)formamidinyl, where aryl is C₆-C₁₀-aryl and alkenyloxy is C₂-C₆-alkenyloxy; C₉-C₁₇-(arylalkynyloxycarbonyl)formamidinyl, where aryl is C₆-C₁₀-aryl and alkynyloxy is C₂-C₆-alkynyloxy.

6. Compounds according to one of the claims 1, 2, 3, 4 or 5, wherein X is oxygen.

7. Compounds according to one of claims 1 to 6, wherein the compound is present as base addition salt.

8. Compounds according to claim 7, wherein the base addition salt is selected among lithium salts, sodium salts, potassium salts, beryllium salts, magnesium salts, calcium salts, strontium salts, aluminum salts and zinc salts, C₁-C₃₀ monoalkylammonium salts, C₁-C₃₀ dialkylammonium salts, C₁-C₃₀ trialkylammonium salts, C₁-C₃₀ tetraalkylammonium salts; C₂-C₃₀ monoalkenylammonium salts, C₂-C₃₀ dialkenylammonium salts, C₂-C₃₀ trialkenylammonium salts, C₂-C₃₀ tetraalkenylammonium salts; C₂-C₃₀ monoalkynylammonium salts, C₂-C₃₀ dialkynylammonium salts, C₂-C₃₀ trialkynylammonium salts, C₂-C₃₀ tetraalkynylammonium salts; C₄-C₃₀ mono(cycloalkylalkylammonium) salts, C₄-C₃₀ di(cycloalkylalkylammonium) salts, C₄-C₃₀ tri(cycloalkylalkylammonium) salts, C₄-C₃₀ tetra(cycloalkylalkylammonium) salts, where cycloalkyl is C₃-C₁₀-cycloalkyl and alkyl is C₁-C₂₇-alkyl; C₅-C₃₀ mono(cycloalkylalkenylammonium) salts, C₅-C₃₀ di(cycloalkylalkenylammonium) salts, C₅-C₃₀ tri(cycloalkylalkenylammonium) salts, C₅-C₃₀ tetra(cycloalkylalkenylammonium) salts, where cycloalkyl is C₃-C₁₀-cycloalkyl and alkenyl is C₂-C₂₇-alkenyl; C₅-C₃₀ mono(cycloalkylalkynylammonium) salts, C₅-C₃₀ di(cycloalkylalkynylammonium) salts, C₅-C₃₀ tri(cycloalkylalkynylammonium) salts, C₅-C₃₀ tetra(cycloalkylalkynylammonium) salts, where cycloalkyl is C₃-C₁₀-cycloalkyl and alkynyl is C₂-C₂₇-alkynyl; C₇-C₃₀ mono(arylalkylammonium) salts, C₇-C₃₀ di(arylalkylammonium) salts, C₇-C₃₀ tri(arylalkylammonium) salts, C₇-C₃₀ tetra(arylalkylammonium) salts, where aryl is C₆-C₁₀-aryl and alkyl is C₁-C₂₄-alkyl; C₈-C₃₀ mono(arylalkenylammonium) salts, C₈-C₃₀ di(arylalkenylammonium) salts, C₈-C₃₀ tri(arylalkenylammonium) salts, C₈-C₃₀ tetra(arylalkenylammonium) salts, where aryl is C₆-C₁₀-aryl and alkenyl is C₂-C₂₄-alkenyl; C₈-C₃₀ mono(arylalkynylammonium) salts, C₈-C₃₀ di(arylalkynylammonium) salts, C₈-C₃₀ tri(arylalkynylammonium) salts, C₈-C₃₀ tetra(arylalkynylammonium) salts, where aryl is C₆-C₁₀-aryl and alkynyl is C₂-C₂₄-alkynyl, combinations of the ammonium salts listed above, and salts derived from heterocyclic nitrogen bases.

9. Compounds of claim 4, selected from:
(4,5α-epoxy-3-hydroxy-14β-methoxy-17-methylmorphinan-6α-ylamino)-acetic acid-tert.-butylester, (4,5α-epoxy-3-hydroxy-14β-methoxy-17-methylmorphinan-6β-ylamino)-acetic acid-tert.-butylester, (4,5α-epoxy-3-hydroxy-14β-methoxy-17-methylmorphinan-6α-ylamino)-acetic acid, (4,5α-epoxy-3-hydroxy-14β-methoxy-17-methylmorphinan-6β-ylamino)-acetic acid, (2S)-2-(4,5α-epoxy-3-hydroxy-14β-methoxy-17-methylmorphinan-6α-ylamino)-propionic acid-tert.-butylester, (2S)-2-(4,5α-epoxy-3-hydroxy-14β-methoxy-17-methylmorphinan-6β-ylamino)-propionic acid-tert.-butylester, (2S)-2-(4,5α-epoxy-3-hydroxy-14β-methoxy-17-methylmorphinan-6α-ylamino)-propionic acid, (2S)-2-(4,5α-epoxy-3-hydroxy-14β-methoxy-17-methylmorphinan-6β-ylamino)-propionic acid, (2S)-2-(4,5α-epoxy-3-hydroxy-14β-methoxy-17-methylmorphinan-6α-ylamino)-3'-phenylpropionic acid-tert.-butylester, (2S)-2-(4,5α-epoxy-3-hydroxy-14β-methoxy-17-methylmorphinan-6β-ylamino)-3'-phenylpropionic acid-tert.-butylester, (2S)-2-(4,5α-epoxy-3-hydroxy-14β-methoxy-17-methylmorphinan-6α-ylamino)-3'-phenylpropionic acid, (2S)-2-(4,5α-epoxy-3-hydroxy-14β-methoxy-17-methylmorphinan-6β-ylamino)-3'-phenylpropionic acid, 6α-amino-4,5α-epoxy-14β-methoxy-17-methylmorphinan-3-ol, 6β-dibenzylamino-4,5α-epoxy-14β-methoxy-17-methylmorphinan-3-ol, 6β-amino-4,5α-epoxy-14β-methoxy-17-methylmorphinan-3-ol, 4,5α-epoxy-6β-[N,N'-bis-(tert.-butoxycarbonyl)guanidinyl]-14β-methoxy-17-methylmorphinan-3-ol, 4,5α-epoxy-6β-guanidinyl-14β-methoxy-17-methylmorphinan-3-ol, 4,5α-epoxy-6α-[N,N'-bis-(tert.-butoxycarbonyl)guanidinyl]-14β-methoxy-17-methylmorphinan-3-ol, 4,5α-epoxy-6α-guanidinyl-14β-methoxy-17-methylmorphinan-3-ol, 1,3-bis-(tert.-butoxycarbonyl)-2-{4,5α-epoxy-6β-[N,N'-bis-(tert.-butoxycarbonyl)guanidinyl]-14β-methoxy-17-methylmorphinan-3-yl}-isourea, 1,3-bis-(tert.-butoxycarbonyl)-2-{4,5α-epoxy-6α-[N,N'-bis-(tert.-butoxycarbonyl)guanidinyl]-14β-methoxy-17-methylmorphinan-3-yl}-isourea, (4,5α-epoxy-3-hydroxy-14β-methoxy-17-methylmorphinan-6α-ylamino)-acetic acid-ethylester dihydrochloride, (4,5α-epoxy-3-hydroxy-14β-methoxy-17-methylmorphinan-6β-ylamino)-acetic acid-ethylester dihydrochloride, (4,5α-epoxy-3-hydroxy-14β-ethoxy-17-methylmorphinan-6α-ylamino)-acetic acid-tert.-butylester, (4,5α-epoxy-3-hydroxy-14β-ethoxy-17-methylmorphinan-6β-ylamino)-acetic acid-tert.-butylester, (4,5α-epoxy-3-hydroxy-14β-ethoxy-17-methylmorphinan-6α-ylamino)-acetic acid bis(tetrafluoroborate), (4,5α-epoxy-3-hydroxy-14β-ethoxy-17-methylmorphinan-6β-ylamino)-acetic acid bis(tetrafluoroborate), (2S)-2-(17-cyclopropylmethyl-4,5α-epoxy-3,14β-dihydroxymorphinan-6β-ylamino)-3-phenylpropionic acid-tert.-butylester, (2S)-2-(17-cyclopropylmethyl-4,5α-epoxy-3,14β-dihydroxymorphinan-6β-ylamino)-3-phenylpropionic acid bis(tetrafluoroborate), {17-cyclopropylmethyl-4,5α-epoxy-3-hydroxy-14β-[(3-phenylpropyl)oxy]-morphinan-6α-ylamino)-acetic acid-tert.-butylester, {17-cyclopropylmethyl-4,5a-epoxy-3-hydroxy-1 4p-[(3-phenylpropyl)oxy]-morphinan-6a-ylamino}-acetic acid-tert.-butylester, (2S)-2-(17-cyclopropylmethyl-4,5a-epoxy-3-hydroxy-14β-[(3-phenylpropyl)oxy]-morphinan-6α-ylamino)-3-phenylpropionic acid-tert.-butylester, {17-cyclopropylmethyl-4,5α-epoxy-3-hydroxy-14β-[(3-phenylpropyl)oxy]-morphinan-6β-ylamino}-acetic acid dihydrochloride; or any pharmaceutically acceptable salt or an easily accessible derivative of them.

10. Compounds of claim 4 selected from
2-[(4,5α-Epoxy-3-hydroxy-14β-methoxy-17-methylmorphinan-6α-yl)amino]acetic acid dihydrochloride
2-[(4,5α-Epoxy-3-hydroxy-14β-methoxy-17-methylmorphinan-6β-yl)amino]acetic acid dihydrochloride
2-[(4,5α-Epoxy-3-hydroxy-14β-methoxy-17-methylmorphinan-6β-yl)amino]acetic acid dihydrochloride monohydrate ethanolate
(2S)-2-[(4,5α-Epoxy-3-hydroxy-14β-methoxy-17-methylmorphinan-6α-yl)amino]propionoic acid dihydrochloride
(2S)-2-[(4,5α-Epoxy-3-hydroxy-14β-methoxy-17-methylmorphinan-6β-yl)amino]propionoic acid dihydrochloride
(2S)-2-[(4,5α-Epoxy-3-hydroxy-14β-methoxy-17-methylmorphinan-6α-yl)amino]-3-phenylpropionoic acid dihydrochloride
(2S)-2-[(4,5α-Epoxy-3-hydroxy-14β-methoxy-17-methylmorphinan-6β-yl)amino]-3-phenylpropionoic acid dihydrochloride

11. Composition, comprising a compound of any of claims 1 to 10 and / or a pharmaceutically acceptable acid addition salt or base addition salt of it, together with a pharmaceutically acceptable carrier substance.

12. Compounds according to any of Claims 1 to 10 as medicament.

13. Use of a compound of Claims 1 to 10 for the manufacture of a medicament for the treatment of pain.

14. Use of a compound of Claims 1 to 10 for the manufacture of a medicament for the treatment of intestinal diseases, in particular chronic inflammation of the small and large intestines (irritable colon syndrome - colon irritabile, colitis ulcerosa, morbus Crohn), diarrhoea or obstipation.

15. Use of a compound of Claims 1 to 10 for the manufacture of a medicament for the treatment of rheumatic diseases, including rheumatoid arthritis, osteoarthritis, arthrosis, spondylosis, lumbago, lupus erythematosus, spondylarthropathy.

16. Use of a compound of Claims 1 to 10 for the manufacture of a medicament for the treatment of tumours and cancer as well as for the treatment of obesity and overweight, and also for the suppression of rejection of transplants after transplantations and for the prevention and treatment of intestinal obstruction (ileus).

17. Use of a compound of Claims 1 to 10 for the manufacture of a medicament for the withdrawal from drug addiction, for example, to opiates, cocaine or alcohol and for the treatment of psychic diseases.

18. Compounds of formula (VIII), 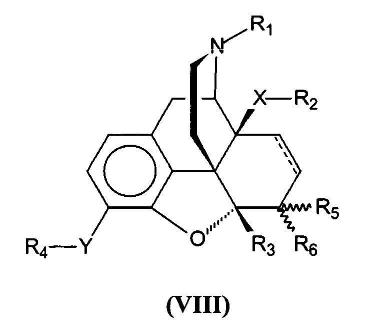 in which the substituents have the following meaning:
R₁ is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl;
the nitrogen joined with R₁ can also be quarternised by two substituents R₁, which can be the same or different and which are defined as previously shown, and whereby the second, quarternised substituent can additionally have the meaning hydroxyl, oxyl (N-oxide) as well as alkoxyl;
R₂, subject to the following definition of X, is hydrogen; C1-C30, preferably C1-C12, more preferably C₁-C₆-alkyl; C1-C30, preferably C1-C12, more preferably C₁-C₆-monohydroxyalkyl; C2-C30, preferably C2-C12, more preferably C₂-C₆-dihydroxyalkyl; C3-C30, preferably C3-C12, more preferably C₃-C₆-trihydroxyalkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl;
R₃ is hydrogen; C1-C30, preferably C1-C12, more preferably C₁-C₆-alkyl; C2-C30, preferably C2-C12, more preferably C₂-C₆-alkenyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; C2-C30, preferably C2-C12, more preferably C₂-C₆-alkanoyl; C3-C30, preferably C3-C12, more preferably C₃-C₆-alkenoyl; C3-C30, preferably C3-C12, more preferably C₃-C₆-alkinoyl; C7-C30, preferably C₇-C₁₆-arylalkanoyl, where aryl preferably is C₆-C₁₀-aryl and alkanoyl preferably is C₁-C₆-alkanoyl; C9-C30, preferably C₉-C₁₆-arylalkenoyl, where aryl preferably is C₆-C₁₀-aryl and alkenoyl preferably is C₃-C₆-alkenoyl; C9-C30, preferably C₉-C₁₆-arylalkinoyl, where aryl preferably is C₆-C₁₀-aryl and alkinoyl preferably is C₃-C₆-alkinoyl; iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl- and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl;
R₅ and R₆, which can be the same or different, are selected from hydrogen; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cydoalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; furthermore, CH(A')CO₂B, where A' is hydrogen; hydroxyl; C1-C30, preferably C1-C12, more preferably C₁-C₆-alkyl; C2-C30, preferably C2-C12, more preferably C₂-C₆-alkenyl; C2-C30, preferably C2-C12, more preferably C₂-C₆-alkynyl; C4-C30, preferably C₄-C₁₆-cycloalkylalkyl, where cycloalkyl preferably is C₃-C₁₀-cycloalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cycloalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; amino; C1-C30, preferably C1-C12, more preferably C₁-C₆-alkylamino; guanidino; C1-C30, preferably C1-C12, more preferably C₁-C₆-alkyl-CO₂B; and where B is hydrogen; C₁-C₃₀-, preferably C1-C12, more preferably C₁-C₆-alkyl; C₂-C₃₀-, preferably C2-C12, more preferably C₂-C₆-alkenyl; C₂-C₃₀-, preferably C2-C12, more preferably C₂-C₆-alkynyl; C4-C30, preferably C₄-C₁₆-cycloalkylalkyl, where cycloalkyl preferably is C₃-C₁₀-cydoalkyl and alkyl preferably is C₁-C₆-alkyl; C5-C30, preferably C₅-C₁₆-cycloalkylalkenyl, where cycloalkyl preferably is C₃-C₁₀-cycloalkyl and alkenyl preferably is C₂-C₆-alkenyl; C5-C30, preferably C₅-C₁₆-cycloalkylalkynyl, where cycloalkyl preferably is C₃-C₁₀-cydoalkyl and alkynyl preferably is C₂-C₆-alkynyl; C7-C30, preferably C₇-C₁₆-arylalkyl, where aryl preferably is C₆-C₁₀-aryl and alkyl preferably is C₁-C₆-alkyl; C8-C30, preferably C₈-C₁₆-arylalkenyl, where aryl preferably is C₆-C₁₀-aryl and alkenyl preferably is C₂-C₆-alkenyl; C8-C30, preferably C₈-C₁₆-arylalkynyl, where aryl preferably is C₆-C₁₀-aryl and alkynyl preferably is C₂-C₆-alkynyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl, furthermore CH(A)SO₃B, whereby A and B are defined as above; also iminomethyl, formamidinyl, C1-C30, preferably C1-C12, more preferably C₁-C₆-N-alkyl- and N,N'-dialkylformamidinyl; C2-C30, preferably C2-C12, more preferably C₂-C₆-N-alkenyl- and N,N'-dialkenylformamidinyl; C2-C30, preferably C2-C12, more preferably C₂-C₆-N-alkynyl- and N,N'-dialkynylformamidinyl; C4-C30, preferably C₄-C₁₆-N-cycloalkylalkyl-and N,N'-dicycloalkylalkylformamidinyl, where cycloalkyl preferably is C₃-C₁₀-cycloalkyl and alkyl preferably is C₁-C₆-alkyl; C5-C30, preferably C₅-C₁₆-N-cylcoalkylalkenyl- and N,N'-dicycloalkylalkenylformamidinyl, where cycloalkyl preferably is C₃-C₁₀-cycloalkyl and alkenyl preferably is C₂-C₆-alkenyl; C5-C30, preferably C₅-C₁₆-N-cycloalkylalkynyl- and N,N'-dicycloalkylalkynylformamidinyl, where cycloalkyl preferably is C₃-C₁₀-cycloalkyl and alkynyl preferably is C₂-C₆-alkynyl; C7-C30, preferably C₇-C₁₆-N-arylalkyl- and N,N'-diarylalkylformamidinyl, where aryl preferably is C₆-C₁₀-aryl and alkyl preferably is C₁-C₆-alkyl; C8-C30, preferably C₈-C₁₆-N-arylalkenyl- and N,N'-diarylalkenylformamidinyl, where aryl preferably is C₆-C₁₀-aryl and alkenyl preferably is C₂-C₆-alkenyl; C8-C30, preferably C₈-C₁₆-N-arylalkynyl- and N,N'-diarylalkynylformamidinyl, where aryl preferably is C₆-C₁₀-aryl and alkynyl preferably is C₂-C₆-alkynyl; C2-C30, preferably C2-C12, more preferably C₂-C₇-N-alkyloxycarbonyl- and N,N'-bis(alkyloxycarbonyl)formamidinyl; C3-C30, preferably C3-C12, more preferably C₃-C₈-N-alkenyloxycarbonyl- and N,N'-bis(alkenyloxycarbonyl)formamidinyl; C3-C30, preferably C3-C12, more preferably C₃-C₈-N-alkynyloxycarbonyl- and N,N'-bis(alkynyloxycarbonyl)formamidinyl; C8-C30, preferably C₈-C₁₇-N-arylalkyloxycarbonyl-and N,N'-bis(arylalkyloxycarbonyl)formamidinyl, where aryl preferably is C₆-C₁₀-aryl and alkyloxy preferably is C₁-C₆-alkyloxy; C9-C30, preferably C₉-C₁₇-N-arylalkenyloxycarbonyl- and N,N'-bis(arylalkenyloxycarbonyl)formamidinyl, where aryl preferably is C₆-C₁₀-aryl and alkenyloxy preferably is C₂-C₆-alkenyloxy; C9-C30, preferably C₉-C₁₇-N-arylalkynyloxycarbonyl- and N,N'-bis(arylalkynyloxycarbonyl)formamidinyl, where aryl preferably is C₆-C₁₀-aryl and alkynyloxy preferably is C₂-C₆-alkynyloxy; C1-C30, preferably C1-C12, more preferably C₁-C₆-N-alkanoyl- and N,N'-dialkanoylformamidinyl; C3-C30, preferably C3-C12, more preferably C₃-C₆-N-alkenoyl- and N,N'-dialkenoylformamidinyl; C3-C30, preferably C3-C12, more preferably C₃-C₆-N-alkinoyl- and N,N'-dialkinoylformamidinyl; C7-C30, preferably C₇-C₁₆-N-arylalkanoyl- and N,N'-diarylalkanoylformamidinyl, where aryl preferably is C₆-C₁₀-aryl and alkanoyl preferably is C₁-C₆-alkyl; C9-C30, preferably Cg-C₁₆-N-arylalkenoyl- and N,N'-diarylalkenoylformamidinyl, where aryl preferably is C₆-C₁₀-aryl and alkenoyl preferably is C₃-C₆-alkenoyl; C9-C30, preferably C₉-C₁₆-N-arylalkinoyl-and N,N'-diarylalkinoylformamidinyl, where aryl preferably is C₆-C₁₀-aryl and alkinoyl preferably is C₃-C₆-alkinoyl; 4,5-dihydro-1*H*-imidazol-2-yl, 1,4,5,6-tetrahydropyrimidin-2-yl, 4,5,6,7-tetrahydro-1 H-[1,3]diazepin-2-yl;
and residues selected from the following group (Vllla): (C₁-C₃₀-alkyl)CO₂B; (C₂-C₃₀-alkenyl)CO₂B; (C₂-C₃₀-alkynyl)CO₂B; (C₄-C₃₀-cycloalkylalkyl)CO₂B, where cycloalkyl is C₃-C₁₀-cycloalkyl and alkyl is C₁-C₂₇-alkyl; (C₅-C₃₀-cycloalkylalkenyl)CO₂B, where cycloalkyl is C₃-C₁₀-cycloalkyl and alkenyl is C₂-C₂₇-alkenyl; (C₅-C₃₀-cycloalkylalkynyl)CO₂B, where cycloalkyl is C₃-C₁₀-cycloalkyl and alkynyl is C₂-C₂₇-alkynyl; (C₇-C₃₀-arylalkyl)CO₂B, where aryl is C₆-C₁₀-aryl and alkyl is C₁-C₂₄-alkyl; (C₈-C₃₀-arylalkenyl)CO₂B, where aryl is C₆-C₁₀-aryl and alkenyl is C₂-C₂₄-alkenyl; (C₈-C₃₀-arylalkynyl)CO₂B, where aryl is C₆-C₁₀-aryl and alkynyl is C₂-C₂₄-alkynyl; [(C₁-C₃₀-alkyl)CO₂B]CO₂B; [(C₂-C₃₀-alkenyl)CO₂B]CO₂B; [(C₂-C₃₀-alkynyl)CO₂B]CO₂B; [(C₄-C₃₀-cycloalkylalkyl)CO₂B]CO₂B, where cycloalkyl is C₃-C₁₀-cycloalkyl and alkyl is C₁-C₂₇-alkyl; [(C₅-C₃₀-cycloalkylalkenyl)CO₂B]CO₂B, where cycloalkyl is C₃-C₁₀-cycloalkyl and alkenyl is C₂-C₂₇-alkenyl; [(C₅-C₃₀-cycloalkylalkynyl)CO₂B]CO₂B, where cycloalkyl is C₃-C₁₀-cycloalkyl and alkynyl is C₂-C₂₇-alkynyl; [(C₇-C₃₀-arylalkyl)CO₂B]CO₂B, where aryl is C₆-C₁₀-aryl and alkyl is C₁-C₂₄-alkyl; [(C₈-C₃₀-arylalkenyl)CO₂B]CO₂B, where aryl is C₆-C₁₀-aryl and alkenyl is C₂-C₂₄-alkenyl; [(C₈-C₃₀-arylalkynyl)CO₂B]CO₂B, where aryl is C₆-C₁₀-aryl and alkynyl is C₂-C₂₄-alkynyl; [(C₁-C₃₀-alkyl)CONH₂]CO₂B; [(C₂-C₃₀-alkenyl)CONH₂]CO₂B; [(C₂-C₃₀-alkynyl)CONH₂]CO₂B; [(C₄-C₃₀-cycloalkylalkyl)CONH₂]CO₂B, where cycloalkyl is C₃-C₁₀-cycloalkyl and alkyl is C₁-C₂₇-alkyl; [(C₅-C₃₀-cycloalkylalkenyl)CONH₂]CO₂B, where cycloalkyl is C₃-C₁₀-cycloalkyl and alkenyl is C₂-C₂₇-alkenyl; [(C₅-C₃₀-cycloalkylalkynyl)CONH₂]CO₂B, where cycloalkyl is C₃-C₁₀-cycloalkyl and alkynyl is C₂-C₂₇-alkynyl; [(C₇-C₃₀-arylalkyl)CONH₂]CO₂B, where aryl is C₆-C₁₀-aryl and alkyl is C₁-C₂₄-alkyl; [(C₈-C₃₀-arylalkenyl)CONH₂]CO₂B, where aryl is C₆-C₁₀-aryl and alkenyl is C₂-C₂₄-alkenyl; [(C₈-C₃₀-arylalkynyl)CONH₂]CO₂B, where aryl is C₆-C₁₀-aryl and alkynyl is C₂-C₂₄-alkynyl; (C₁-C₃₀-alkyl-S-A")CO₂B; (C₂-C₃₀-alkenyl-S-A")CO₂B; (C₂-C₃₀-alkynyl-S-A")CO₂B; (C₄-C₃₀-cycloalkylalkyl-S-A")CO₂B, where cycloalkyl is C₃-C₁₀-cycloalkyl and alkyl is C₁-C₂₇-alkyl; (C₅-C₃₀-cycloalkylalkenyl-S-A")CO₂B, where cycloalkyl is C₃-C₁₀-cycloalkyl and alkenyl is C₂-C₂₇-alkenyl; (C₅-C₃₀-cycloalkylalkynyl-S-A")CO₂B, where cycloalkyl is C₃-C₁₀-cycloalkyl and alkynyl is C₂-C₂₇-alkynyl; (C₇-C₃₀-arylalkyl-S-A")CO₂B, where aryl is C₆-C₁₀-aryl and alkyl is C₁-C₂₄-alkyl; (C₈-C₃₀-arylalkenyl-S-A")CO₂B, where aryl is C₆-C₁₀-aryl and alkenyl is C₂-C₂₄-alkenyl; (C₈-C₃₀-arylalkynyl-S-A")CO₂B, where aryl is C₆-C₁₀-aryl and alkynyl is C₂-C₂₄-alkynyl; wherein B is as defined above and A" is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃₀-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl; n=1-30(CHDCONH)ₙCHDCO₂B, where D is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃₀-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl; (C₁-C₃₀-alkyl)CO₂B; (C₂-C₃₀-alkenyl)CO₂B; (C₂-C₃₀-alkynyl)CO₂B; (C₄-C₃₀-cycloalkylalkyl)CO₂B, where cycloalkyl is C₃-C₁₀-cycloalkyl and alkyl is C₁-C₂₇-alkyl; (C₅-C₃₀-cycloalkylalkenyl)CO₂B, where cycloalkyl is C₃-C₁₀-cycloalkyl and alkenyl is C₂-C₂₇-alkenyl; (C₅-C₃₀-cycloalkylalkynyl)CO₂B, where cycloalkyl is C₃-C₁₀-cycloalkyl and alkynyl is C₂-C₂₇-alkynyl; (C₇-C₃₀-arylalkyl)CO₂B, where aryl is C₆-C₁₀-aryl and alkyl is C₁-C₂₄-alkyl; (C₈-C₃₀-arylalkenyl)CO₂B, where aryl is C₆-C₁₀-aryl and alkenyl is C₂-C₂₄-alkenyl; (C₈-C₃₀-arylalkynyl)CO₂B, where aryl is C₆-C₁₀-aryl and alkynyl is C₂-C₂₄-alkynyl;
(C₁-C₃₀-alkyl)CONH₂; (C₂-C₃₀-alkenyl)CONH₂; (C₂-C₃₀-alkynyl)CONH₂; (C₄-C₃₀-cycloalkylalkyl)CONH₂, where cycloalkyl is C₃-C₁₀-cycloalkyl and alkyl is C₁-C₂₇-alkyl; (C₅-C₃₀-cycloalkylalkenyl)CONH₂, where cycloalkyl is C₃-C₁₀-cycloalkyl and alkenyl is C₂-C₂₇-alkenyl; (C₅-C₃₀-cycloalkylalkynyl)CONH₂, where cycloalkyl is C₃-C₁₀-cycloalkyl and alkynyl is C₂-C₂₇-alkynyl; (C₇-C₃₀-arylalkyl)CONH₂, where aryl is C₆-C₁₀-aryl and alkyl is C₁-C₂₄-alkyl; (C₈-C₃₀-arylalkenyl)CONH₂, where aryl is C₆-C₁₀-aryl and alkenyl is C₂-C₂₄-alkenyl; (C₈-C₃₀-arylalkynyl)CONH₂, where aryl is C₆-C₁₀-aryl and alkynyl is C₂-C₂₄-alkynyl; where B is as defined above;
(C₁-C₃₀-alkyl)SO₃A"; (C₂-C₃₀-alkenyl)SO₃A"; (C₂-C₃₀-alkynyl)SO₃A"; (C₄-C₃₀-cycloalkylalkyl)SO₃A", where cycloalkyl is C₃-C₁₀-cycloalkyl and alkyl is C₁-C₂₇-alkyl; (C₅-C₃₀-cycloalkylalkenyl)SO₃A", where cycloalkyl is C₃-C₁₀-cycloalkyl and alkenyl is C₂-C₂₇-alkenyl; (C₅-C₃₀-cycloalkylalkynyl)SO₃A", where cycloalkyl is C₃-C₁₀-cycloalkyl and alkynyl is C₂-C₂₇-alkynyl; (C₇-C₃₀-arylalkyl)SO₃A", where aryl is C₆-C₁₀-aryl and alkyl is C₁-C₂₄-alkyl; (C₈-C₃₀-arylalkenyl)SO₃A", where aryl is C₆-C₁₀-aryl and alkenyl is C₂-C₂₄-alkenyl; (C₈-C₃₀-arylalkynyl)SO₃A", where aryl is C₆-C₁₀-aryl and alkynyl is C₂-C₂₄-alkynyl; (C₁-C₃₀-alkyl)PO(OA")₂; (C₂-C₃₀-alkenyl)PO(OA")₂; (C₂-C₃₀-alkynyl)PO(OA")₂; (C₄-C₃₀-cycloalkylalkyl)PO(OA")₂, where cycloalkyl is C₃-C₁₀-cycloalkyl and alkyl is C₁-C₂₇-alkyl; (C₅-C₃₀-cycloalkylalkenyl)PO(OA")₂, where cycloalkyl is C₃-C₁₀-cycloalkyl and alkenyl is C₂-C₂₇-alkenyl; (C₅-C₃₀-cycloalkylalkynyl)PO(OA")₂, where cycloalkyl is C₃-C₁₀-cycloalkyl and alkynyl is C₂-C₂₇-alkynyl; (C₇-C₃₀-arylalkyl)PO(OA")2, where aryl is C₆-C₁₀-aryl and alkyl is C₁-C₂₄-alkyl; (C₈-C₃₀-arylalkenyl)PO(OA")₂, where aryl is C₆-C₁₀-aryl and alkenyl is C₂-C₂₄-alkenyl; (C₈-C₃₀-arylalkynyl)PO(OA")₂, where aryl is C₆-C₁₀-aryl and alkynyl is C₂-C₂₄-alkynyl; wherein A" is H; C₁-C₃₀-alkyl; C₂-C₃₀-alkenyl; C₂-C₃₀-alkynyl; C₄-C₃₀-cycloalkylalkyl, where cycloalkyl is C₃-C₁₀-cycloalkyl and alkyl is C₁-C₂₇-alkyl; C₅-C₃₀-cycloalkylalkenyl, where cycloalkyl is C₃-C₁₀-cycloalkyl and alkenyl is C₂-C₂₇-alkenyl; C₅-C₃₀-cycloalkylalkynyl, where cycloalkyl is C₃-C₁₀-cycloalkyl and alkynyl is C₂-C₂₇-alkynyl; C₇-C₃₀-arylalkyl, where aryl is C₆-C₁₀-aryl and alkyl is C₁-C₂₄-alkyl; C₈-C₃₀-arylalkenyl, where aryl is C₆-C₁₀-aryl and alkenyl is C₂-C₂₄-alkenyl; C₈-C₃₀-arylalkynyl, where aryl is C₆-C₁₀-aryl and alkynyl is C₂-C₂₄-alkynyl;
wherein at least one of R₅ and R₆ is selected from group (VIIIa);
and pharmaceutically acceptable acid addition salts as well as base addition salts and easily accessible derivatives (e.g. esters or amides of amino acid derivatives), as well as polymorphic forms thereof.

19. Compounds according to claim 18, wherein one of R₅ and R₆ is selected from group (VIIIa) and the other one represents H or OH.

20. Compounds according to one of the claims 18 or 19, wherein X is oxygen.

21. Compounds according to one of claims 18 to 20, wherein the compound is present as base addition salt.

22. Compounds according to claim 21, wherein the base addition salt is selected among lithium salts, sodium salts, potassium salts, beryllium salts, magnesium salts, calcium salts, strontium salts, aluminum salts and zinc salts, C₁-C₃₀ monoalkylammonium salts, C₁-C₃₀ dialkylammonium salts, C₁-C₃₀ trialkylammonium salts, C₁-C₃₀ tetraalkylammonium salts; C₂-C₃₀ monoalkenylammonium salts, C₂-C₃₀ dialkenylammonium salts, C₂-C₃₀ trialkenylammonium salts, C₂-C₃₀ tetraalkenylammonium salts; C₂-C₃₀ monoalkynylammonium salts, C₂-C₃₀ dialkynylammonium salts, C₂-C₃₀ trialkynylammonium salts, C₂-C₃₀ tetraalkynylammonium salts; C₄-C₃₀ mono(cycloalkylalkylammonium) salts, C₄-C₃₀ di(cycloalkylalkylammonium) salts, C₄-C₃₀ tri(cycloalkylalkylammonium) salts, C₄-C₃₀ tetra(cycloalkylalkylammonium) salts, where cycloalkyl is C₃-C₁₀-cycloalkyl and alkyl is C₁-C₂₇-alkyl; C₅-C₃₀ mono(cycloalkylalkenylammonium) salts, C₅-C₃₀ di(cycloalkylalkenylammonium) salts, C₅-C₃₀ tri(cycloalkylalkenylammonium) salts, C₅-C₃₀ tetra(cycloalkylalkenylammonium) salts, where cycloalkyl is C₃-C₁₀-cycloalkyl and alkenyl is C₂-C₂₇-alkenyl; C₅-C₃₀ mono(cycloalkylalkynylammonium) salts, C₅-C₃₀ di(cycloalkylalkynylammonium) salts, C₅-C₃₀ tri(cycloalkylalkynylammonium) salts, C₅-C₃₀ tetra(cycloalkylalkynylammonium) salts, where cycloalkyl is C₃-C₁₀-cycloalkyl and alkynyl is C₂-C₂₇-alkynyl; C₇-C₃₀ mono(arylalkylammonium) salts, C₇-C₃₀ di(arylalkylammonium) salts, C₇-C₃₀ tri(arylalkylammonium) salts, C₇-C₃₀ tetra(arylalkylammonium) salts, where aryl is C₆-C₁₀-aryl and alkyl is C₁-C₂₄-alkyl; C₈-C₃₀ mono(arylalkenylammonium) salts, C₈-C₃₀ di(arylalkenylammonium) salts, C₈-C₃₀ tri(arylalkenylammonium) salts, C₈-C₃₀ tetra(arylalkenylammonium) salts, where aryl is C₆-C₁₀-aryl and alkenyl is C₂-C₂₄-alkenyl; C₈-C₃₀ mono(arylalkynylammonium) salts, C₈-C₃₀ di(arylalkynylammonium) salts, C₈-C₃₀ tri(arylalkynylammonium) salts, C₈-C₃₀ tetra(arylalkynylammonium) salts, where aryl is C₆-C₁₀-aryl and alkynyl is C₂-C₂₄-alkynyl, combinations of the ammonium salts listed above, and salts derived from heterocyclic nitrogen bases.

23. Compounds according to any of Claims 18 to 22 as medicament.

24. Use of a compound of Claims 18 to 22 for the manufacture of a medicament for the treatment of pain.

25. Use of a compound of claims 18 to 22 for the manufacture of a medicament for the treatment of intestinal diseases, in particular chronic inflammation of the small and large intestines (irritable colon syndrome - colon irritabile, colitis ulcerosa, morbus Crohn), diarrhoea or obstipation.

26. Use of a compound of Claims 18 to 22 for the manufacture of a medicament for the treatment of rheumatic diseases, including rheumatoid arthritis, osteoarthritis, arthrosis, spondylosis, lumbago, lupus erythematosus, spondylarthropathy.

27. Use of a compound of Claims 18 to 22 for the manufacture of a medicament for the treatment of tumours and cancer as well as for the treatment of obesity and overweight, and also for the suppression of rejection of transplants after transplantations and for the prevention and treatment of intestinal obstruction (ileus).

28. Use of a compound of Claims 18 to 22 for the manufacture of a medicament for the withdrawal from drug addiction, for example, to opiates, cocaine or alcohol and for the treatment of psychic diseases.

29. Compounds of claim 1, 4, or 18 wherein the polymorphic form is a crystalline polymorphic form.

30. Compounds of claim 1, 4, or 18 wherein the polymorphic form is an amorphous polymorphic form.

31. Method for synthesizing a compound according to claim 4, comprising the following reaction sequence: 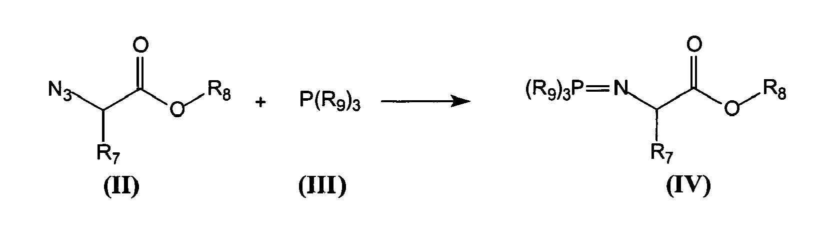 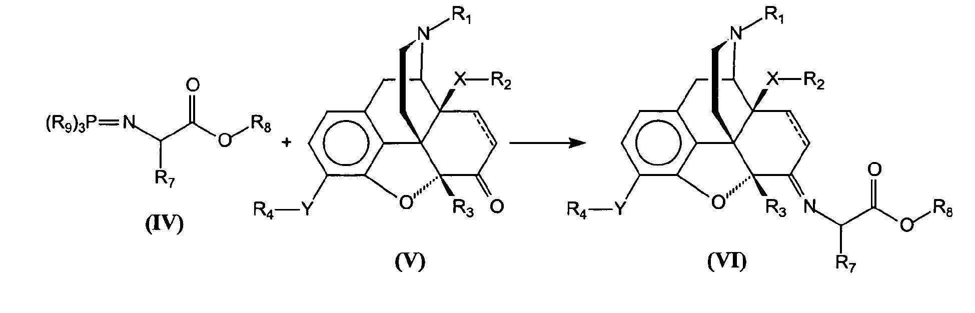 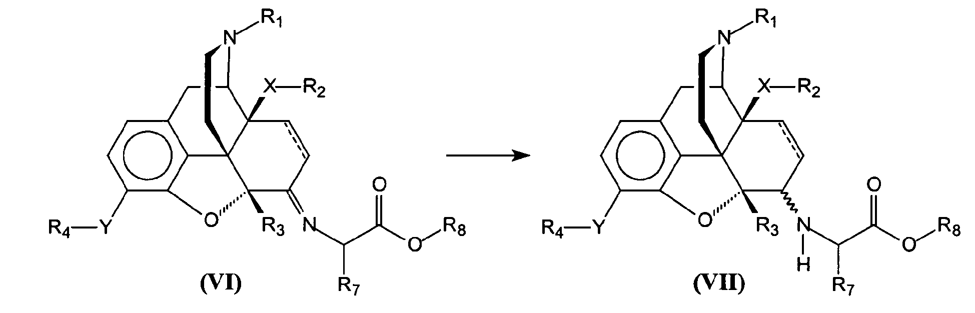 wherein R₁ to R₄ are as defined in claim 4 and R₇ and R₈ are selected so that compound (VII) is in accordance with the definitions for R₅ and R₆ as given in claim 4 and R₉ is selected from any suitably organic moiety not detrimental for the reaction.

32. Compounds of claim 1, selected from:
(2S)-2-[(4,5α-Epoxy-3,14β-dihydroxy-17-methylmorphinan-6α-yl)amino]propionic acid tert-butyl ester
(2S)-2-[(4,5α-Epoxy-3,14β-dihydroxy-17-methylmorphinan-6β-yl)amino]propionic acid tert-butyl ester
(2S)-2-[(4,5α-Epoxy-3,14β-dihydroxy-17-methylmorphinan-6α-yl)amino]propionic acid
(2S)-2-[(4,5α-Epoxy-3,14β-dihydroxy-17-methy)morphinan-6β-yl)amino]propionic acid
(2S)-2-[(4,5α-Epoxy-3,14β-dihydroxy-17-methylmorphinan-6β-yl)amino]-3-phenylpropionic acid tert-butyl ester
(2S)-2-[(4,5α-Epoxy-3,14β-dihydroxy-17-methytmorphinan-6β-yl)amino]-3-phenylpropionic acid tert-butyl ester
(2S)-2-[(4,5α-Epoxy-3,14β-dihydroxy-17-methylmorphinan-6α-yl)amino]-3-phenylpropionic acid
(2S)-2-[(4,5α-Epoxy-3,14β-dihydroxy-17-methylmorphinan-6β-yl)amino]-3-phenylpropionic acid
3-[(4,5α-Epoxy-3-hydroxy-14β-methoxy-17-methylmorphinan-6α-yl)amino]propionic acid tert-butyl ester
3-[(4,5α-Epoxy-3-hydroxy-14β-methoxy-17-methylmorphinan-6β-yl)amino]propionic acid tert-butyl ester
3-[(4,5α-Epoxy-3-hydroxy-14β-methoxy-17-methylmorphinan-6α-yl)amino]propionic acid
3-[(4,5α-Epoxy-3-hydroxy-14β-methoxy-17-methylmorphinan-6β-yl)amino]propionic acid
4-[(4,5α-Epoxy-3-hydroxy-14β-methoxy-17-methylmorphinan-6α-yl)amino]butyric acid tert-butyl ester
4-[(4,5α-Epoxy-3-hydroxy-14β-methoxy-17-methylmorphinan-6β-yl)amino]butyric acid tert-butyl ester
4-[(4,5α-Epoxy-3-hydroxy-14β-methoxy-17-methylmorphinan-6α-yl)amino]butyric acid
4-[(4,5α-Epoxy-3-hydroxy-14β-methoxy-17-methylmorphinan-6β-yl)amino]butyric acid
(2S)-2-[(4,5α-Epoxy-3-hydroxy-14β-methoxy-17-methylmorphinan-6α-yl)amino]-3-methylbutyric acid tert-butyl ester
(2S)-2-[(4,5α-Epoxy-3-hydroxy-14β-methoxy-17-methylmorphinan-6β-yl)amino]-3-methylbutyric acid tert-butyl ester
(2S)-2-[(4,5α-Epoxy-3-hydroxy-14β-methoxy-17-methylmorphinan-6α-yl)amino]-3-methylbutyric acid
(2S)-2-[(4,5α-Epoxy-3-hydroxy-14β-methoxy-17-methylmorphinan-6β-yl)amino]-3-methylbutyric acid
(2R)-2-[(4,5α-Epoxy-3-hydroxy-14β-methoxy-17-methylmorphinan-6α-yl)amino]-3-methylbutyric acid tert-butyl ester
(2R)-2-[(4,5α-Epoxy-3-hydroxy-14β-methoxy-17-methylmorphinan-6β-yl)amino]-3-methylbutyric acid tert-butyl ester
(2R)-2-[(4,5α-Epoxy-3-hydroxy-14β-methoxy-17-methylmorphinan-6α-yl)amino]-3-methylbutyric acid
(2R)-2-[(4,5α-Epoxy-3-hydroxy-14β-methoxy-17-methylmorphinan-6β-yl)amino]-3-methylbutyric acid
(2S)-2-[(4,5α-Epoxy-3-hydroxy-14β-methoxy-17-methylmorphinan-6α-yl)amino]butanedioic acid di-tert-butyl ester
(2S)-2-[(4,5α-Epoxy-3-hydroxy-14β-methoxy-17-methylmorphinan-6β-yl)amino]butanedioic acid di-tert-butyl ester
(2S)-2-[(4,5α-Epoxy-3-hydroxy-14β-methoxy-17-methylmorphinan-6α-yl)amino]butanedioic acid
(2S)-2-[(4,5a-Epoxy-3-hydroxy-,4p-methoxy-1 7-methylmorphinan-6p-yl)amino]butanedioic acid
(2S)-2-[(4,5α-Epoxy-3-hydroxy-14β-methoxy-17-methylmorphinan-6α-yl)amino]pentanedioic acid di-tert-butyl ester
(2S)-2-[(4,5α-Epoxy-3-hydroxy-14β-methoxy-17-methylmorphinan-6β-yl)amino]pentanedioic acid di-tert-butyl ester
(2S)-2-[(4,5α-Epoxy-3-hydroxy-14β-methoxy-17-methylmorphinan-6α-yl)amino]pentanedioic acid
(2S)-2-[(4,5α-Epoxy-3-hydroxy-14β-methoxy-17-methylmorphinan-6β-yl)amino]pentanedioic acid
(2S)-3-Carbamoyl-2-[(4,5α-epoxy-3-hydroxy-14β-methoxy-17-methylmorphinan-6α-yl)amino]propionic acid tert-butyl ester
(2S)-3-Carbamoyl-2-[(4,5α-epoxy-3-hydroxy-14β-methoxy-17-methylmorphinan-6β-yl)amino]propionic acid tert-butyl ester
(2S)-3-Carbamoyl-2-[(4,5α-epoxy-3-hydroxy-14β-methoxy-17-methylmorphinan-6α-yl)amino]propionic acid
(2S)-3-Carbamoyl-2-[(4,5α-epoxy-3-hydroxy-14β-methoxy-17-methylmorphinan-6β-yl)amino]propionic acid
(2S)-4-Carbamoyl-2-[(4,5α-epoxy-3-hydroxy-14β-methoxy-17-methylmorphinan-6α-yl)amino]butyric acid tert-butyl ester
(2S)-4-Carbamoyl-2-[(4,5α-epoxy-3-hydroxy-14β-methoxy-17-methylmorphinan-6β-yl)amino]butyric acid tert-butyl ester
(2S)-4-Carbamoyl-2-[(4,5α-epoxy-3-hydroxy-14β-methoxy-17-methylmorphinan-6α-yl)amino]butyric acid
(2S)-4-Carbamoy)-2-[(4,5α-epoxy-3-hydroxy-14β-methoxy-17-methylmorphinan-6β-yl)amino]butyric acid
(2S)-2-[(4,5α-Epoxy-3-hydroxy-14β-methoxy-17-methylmorphinan-6α-yl)amino]-4-methylthiobutyric acid tert-butyl ester
(2S)-2-[(4,5α-Epoxy-3-hydroxy-14β-methoxy-17-methylmorphinan-6β-yl)amino]-4-methylthiobutyric acid tert-butyl ester
(2S)-2-[(4,5α-Epoxy-3-hydroxy-14β-methoxy-17-methylmorphinan-6α-yl)amino]-4-methylthiobutyric acid
(2S)-2-[(4,5α-Epoxy-3-hydroxy-14β-methoxy-17-methylmorphinan-6β-yl)amino]-4-methylthiobutyric acid
(2S)-2-[(4,5α-Epoxy-3-hydroxy-14β-methoxy-17-methylmorphinan-6α-yl)amino]-3-(1 H-indol-3-yl)propionic acid tert-butyl ester
(2S)-2-[(4,5α-Epoxy-3-hydroxy-14β-methoxy-17-methylmorphinan-6β-yl)amino]-3-(1 H-indol-3-yl)propionic acid tert-butyl ester
(2S)-2-[(4,5α-Epoxy-3-hydroxy-14β-methoxy-17-methylmorphinan-6α-yl)amino]-3-(1 H-indol-3-yl)propionic acid
(2S)-2-[(4,5α-Epoxy-3-hydroxy-14β-methoxy-17-methylmorphinan-6β-yl)amino]-3-(1 H-indol-3-yl)propionic acid
(2S)-1-[(4,5α-Epoxy-3-hydroxy-14β-methoxy-17-methylmorphinan-6α-yl)]pyrrolidine-2-carboxylic acid tert-butyl ester
(2S)-1-[(4,5α-Epoxy-3-hydroxy-14p-methoxy-17-methy)morphinan-6β-yl)]pyrrolidine-2-carboxylic acid tert-butyl ester
2-[(4,5α-Epoxy-3-hydroxy-14β-methoxy-17-methylmorphinan-6α-yl)amino]ethanesulfonic acid
2-[(4,5α-Epoxy-3-hydroxy-14β-methoxy-17-methylmorphinan-6α-yl)amino]ethanesulfonic acid
2-{2-[(4,5α-epoxy-3-hydroxy-14β-methoxy-17-methylmorphinan-6α-yl)amino]acetylamino}acetic acid benzyl ester
2-{2-[(4,5α-epoxy-3-hydroxy-14β-methoxy-17-methylmorphinan-6β-yl)amino]acetylaminolacetic acid benzyl ester
2-{2-[(4,5α-epoxy-3-hydroxy-14β-methoxy-17-methylmorphinan-6α-yl)amino]acetylaminolacetic acid
2-{2-[(4,5α-epoxy-3-hydroxy-14β-methoxy-17-methylmorphinan-6β-yl)amino]acetylamino}acetic acid
(2S)-2-{[(4,5α-Epoxy-3-hydroxy-14β-methoxy-17-methylmorphinan-6α-yl)amino]-(2S)-3-methylbutyrylamino}-3-(4-hydroxyphenyl)propionic acid benzyl ester
(2S)-2-([(4,5α-Epoxy-3-hydroxy-14β-methoxy-17-methylmorphinan-6β-yl)amino]-(2S)-3-methylbutyrylamino}-3-(4-hydroxyphenyl)propionic acid benzyl ester
(2S)-2-{[(4,5α-Epoxy-3-hydroxy-14β-methoxy-17-methylmorphinan-6α-yl)amino]-(2S)-3-methylbutyrylamino}-3-(4-hydroxyphenyl)propionic acid
(2S)-2-{[(4,5α-Epoxy-3-hydroxy-14β-methoxy-17-methylmorphinan-6β-yl)amino]-(2S)-3-methylbutyrylamino}-3-(4-hydroxyphenyl)propionic acid
3-[(17-Cyclopropylmethyl-4,5α-epoxy-3-hydroxy-14β-methoxymorphinan-6α-yl)amino]propionic acid tert-butyl ester
3-[(17-Cyclopropylmethyl-4,5α-epoxy-3-hydroxy-14β-methoxymorphinan-6β-yl)amino]propionic acid tert-butyl ester
3-[(17-Cyclopropylmethyl-4,5α-epoxy-3-hydroxy-14β-methoxymorphinan-6α-yl)amino]propionic acid
3-[(17-Cyclopropylmethyl-4,5α-epoxy-3-hydroxy-14β-methoxymorphinan-6β-yl)amino]propionic acid
4-[(17-Cyclopropylmethyl-4,5α-epoxy-3-hydroxy-14β-methoxymorphinan-6α-yl)amino]butyric acid tert-butyl ester
3-[(17-Cyclopropylmethyl-4,5α-epoxy-3-hydroxy-14β-methoxymorphinan-6β-yl)amino]butyric acid tert-butyl ester
3-[(17-Cyclopropylmethyl-4,5α-epoxy-3-hydroxy-14β-methoxymorphinan-6α-yl)amino]butyric acid
3-[(17-Cyclopropylmethyl-4,5α-epoxy-3-hydroxy-14β-methoxymorphinan-6β-yl)amino]butyric acid
(2S)-2-[(17-Cyclopropylmethyl-4,5α-epoxy-3-hydroxy-14β-methoxymorphinan-6α-yl)amino]-3-methylbutyric acid tert-butyl ester
(2S)-2-[(17-Cyclopropylmethyl-4,5α-epoxy-3-hydroxy-14β-methoxymorphinan-6β-yl)amino]-3-methylbutyric acid tert-butyl ester
(2S)-2-[(17-Cyclopropylmethyl-4,5α-epoxy-3-hydroxy-14β-methoxymorphinan-6α-yl)amino]-3-methylbutyric acid
(2S)-2-[(17-Cyclopropylmethyl-4,5α-eEpoxy-3-hydroxy-14β-methoxymorphinan-6β-yl)amino]-3-methylbutyric acid
(2R)-2-[(17-Cyclopropylmethyl-4,5α-epoxy-3-hydroxy-14β-methoxymorphinan-6α-yl)amino]-3-methylbutyric acid tert-butyl ester
(2R)-2-[(17-Cyclopropylmethyl-4,5α-epoxy-3-hydroxy-14β-methoxymorphinan-6β-yl)amino]-3-methylbutyric acid tert-butyl ester
(2R)-2-[(17-Cyclopropylmethyl-4,5α-epoxy-3-hydroxy-14β-methoxymorphinan-6α-yl)amino]-3-methylbutyric acid
(2R)-2-[(17-Cyclopropylmethyl-4,5α-eEpoxy-3-hydroxy-14β-methoxymorphinan-6β-yl)amino]-3-methylbutyric acid
(2S)-2-[(17-Cyclopropylmethyl-4,5α-epoxy-3-hydroxy-14β-methoxymorphinan-6α-yl)amino]butanedioic acid di-tert-butyl ester
(2S)-2-[(17-Cyclopropylmethy)-4,5α-epoxy-3-hydroxy-14β-methoxymorphinan-6β-yl)amino]butanedioic acid di-tert-butyl ester
(2S)-2-[(17-Cyclopropylmethyl-4,5α-epoxy-3-hydroxy-14β-methoxymorphinan-6α-yl)amino]butanedioic acid
(2S)-2-[(17-Cyclopropylmethyl-4,5α-epoxy-3-hydroxy-14β-methoxymorphinan-6β-yl)amino]butanedioic acid
